# EUROPEAN PATENT APPLICATION

(11) **EP 3 957 995 A1**
(43) Date of publication of application: **23.02.2022**
(21) Application number: 21177920.2
(22) Date of filing: 18.11.2019
(51) Int. Cl.: G01N 33/543, G01N 33/68

(54) **METHODS AND REAGENTS FOR MULTIPLEX BINDING EXPERIMENTS**

(30) Priority: 19.11.2018 EP 18306517
(62) Divisional of application: 19806184.8
(71) Applicant: Bioaster, 69007 Lyon (FR)
(72) Inventor: LECINE, Patrick, 69290 Craponne (FR); VEDRINE, Christophe, 78420 Carrieres-sur-Seine (FR); LUGARI, Adrien, 83170 Rougiers (FR)
(74) Representative: Cabinet Laurent & Charras

(57) **Abstract**

The present invention concerns a support for a multiplex binding experiment functionalized with at least two different polypeptides having high affinity to their cognate binding partners provided in a reaction mixture, and their use in e.g. immunoassays.

## Description

### FIELD OF THE INVENTION

The invention relates to methods and reagents for multiplex binding experiments. It makes use of different polypeptide couples having high affinity, able to display molecules of interest in an appropriate manner on the surface of a support.

### BACKGROUND OF THE INVENTION

An immunoassay is generally defined as a biochemical test that measures the presence or concentration of a macromolecule or a small molecule in solution through the use of a binder, especially an antibody and/or an antigen. The molecule detected by the immunoassay is often referred to as an analyte and is in many cases a protein, although it may be other kinds of molecules, of different size and nature as long as the proper antibodies that have the adequate properties for the assay are developed. The basic components of an immunoassay generally include an analyte, an antibody and a detectable label, which can be an enzyme (e.g. horseradish peroxidase or HRP, alkaline phosphatase or AP, glucose oxidase, luciferase or Luc), a radioactive isotope (in radioimmunoassays or RIA), a DNA reporter (in e.g. real-time immunoquantitative polymerase chain reaction or iqPCR), a fluorogenic reporter (e.g. phycoerythrin), or an electrochemiluminescent tag.

Multiplex assays have been developed for the simultaneous measurement of multiple analytes in a single sample. Multiplex testing is becoming indispensable in contemporary clinical diagnosis. With the increasing numbers of (bio)markers discovered, there is often a need to detect several (bio)markers simultaneously to generate meaningful or conclusive information. This is important for reliable disease detection and monitoring, as a single (bio)marker may be indicative of more than one disease, with a statistically inadequate predictive value. In addition, false positives and negatives may appear quite frequently with single (bio)marker detection, but could be minimized by detecting a (bio)marker panel. Therefore multiplex testing ensures precise diagnostics and mitigates patient risk. Another benefit of multiplex testing is the reduction of the required quantity of samples, as well as the reduction of diagnostic time and costs in comparison to performing multiple single tests.

Many multiplex testing methods have been developed for diagnostics. The commonly used ones include multiplex real-time polymerase chain reaction (PCR), microarrays, next-generation sequencing, enzyme immunoassay (EIA) / enzyme-linked immunoabsorbant assay (ELISA), multiplex lateral flow biosensors (LF), vertical flow assays (VFA) or Luminex^{®} multiplex assay.

A well-established format for such multiplex assays makes use of flow-based technology and ligand (e.g. antibody) - coated beads. Luminex^{™} systems are based on xMAP^{™} (multi-analyte profiling) technology combined with single and multiplex bead-based immunoassays. The beads used in xMAP^{™} immunoassays are dyed with different concentrations of fluorophores to generate bead sets that can be easily discriminated. Individual bead sets are coated with a capture antibody qualified for one specific analyte. The captured analyte from a sample is detected using an analyte-specific biotinylated antibody that binds to the appropriate epitope of the immobilized analyte, plus streptavidin-conjugated R-phycoerythrin (S-RPE). For detection of the immunoassay sandwich complex, Luminex^{™} instruments use either light-emitting diodes (LEDs) for excitation of each fluorescent bead combined with a CCD camera for bead and analyte detection, or a flow-based detection system using a red and green laser. High-speed digital signal processors are used to interrogate the data. As each antibody-coated bead is individually identifiable for a specific analyte, multiple beads can be combined to simultaneously measure the levels of up to 500 targets for nucleic acid and typically no more than 50 targets for proteins due to biological interference in a single sample.

EIA is usually performed in a 96-well plate: when based on the competitive enzyme immunoassay principle, the microplate in the kit is coated with a target specific antibody, which is competitively bound by a mixture of the endogenous peptide in the biological sample and biotinylated peptide, provided in the kit and producing a colorimetric signal through its interaction with HRP-streptavidin. Based thereon, the MSD^{®} (Meso Scale Discovery) technology offers a multi-array technology combining electrochemiluminescence (ECL) detection and patterned arrays: Up to 10 working electrodes (enabling multiplexing up to 10 analytes) are spotted in a well, to support a capture antibody able to bind an analyte, further detected via a detection antibody, e.g. SULFO-TAG^{™} labeled.

Document WO2014/164594 discloses methods for conducting solid-phase multiplex binding assays. In practice, distinct oligonucleotide sequences are located on distinct areas of a multi-assay plate, whereas each capture antibody is labeled with each individual oligonucleotide sequence complement with conventional coupling protocols. After incubation with the plate, the capture antibodies are immobilized to the multi-well plate to form a plurality of binding reagent complexes. A solution including a plurality of analytes is then added, as well as a set of labeled detection antibodies. Alternatively, the individual oligonucleotide sequence complements are bound to streptavidin or avidin, whereas the antibodies are biotinylated so as to prepare a set of individual biotinylated capture antibody/oligonucleotide-SA mixtures.

Concerning lateral flow immunochromatographic assays, document WO03/062824 discloses a lateral flow method and strip capable of quantifying a plurality of analytes at the same time. It illustrates the preparation of monoclonal antibodies specifically reacting with each of the proteins AFP, CEA, CRP and PSA. Said capture antibodies were dispensed sequentially in test lines at intervals of 2 mm. Other monoclonal antibodies having epitopes different from the immobilized antibodies were reacted with fluorescent material to be used as detectors when impregnated on a glass fiber pad (antibody/fluorescent conjugate pad). To get a greater sensitivity and reproducibility, it is proposed to immobilize avidin on the support and couple the capture antibodies with biotin. However, the development of multiplex lateral flow biosensors is still compromised by sensitivity and specificity problems, partly due to cross-reaction(s) occurring among a mixture of analytes (Li et Macdonald, Biosensors and Bioelectronics 83(2016) 177-92).

In view of this, there exists a persistent need for developing further multiplex binding assays, efficient, simple, cheap, polyvalent and easy to use.

### BRIEF SUMMARY OF THE INVENTION

Having conducted extensive experiments and tests, the inventors have identified new linkers, in the form of polypeptide couples with low dissociation constants, to be used on a support to present molecules of interest, especially analyte-capture entities.

This is of particular interest for multiplex binding experiments: whereas the dissociation constant of an antigen/antibody couple is generally in the nanomolar range, the polypeptide couples used in the frame of the present invention have a dissociation constant at least in the range of the picomolar range, or even in the femtomolar range, leading to an increased specificity and/or sensitivity.

### Definitions

The articles "a" and "an" are used herein to refer to one or to more than one (*i.e.,* to at least one) of the grammatical object of the article. By way of example, "an element" means one element or more than one element.

"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

The terms "comprising", "comprises" and "comprised of' as used herein are synonymous with "including", "includes" or "containing", "contains", and are inclusive or open-ended and do not exclude additional, non-recited members, elements or method steps.

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homo- or hetero-multimers, variants of polypeptides, modified polypeptides, derivatives, analogs, fusion proteins, among others. The polypeptides include natural peptides, recombinant peptides, synthetic peptides, or a combination thereof.

"Homologous" or "identical" refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous or identical at that position. The percent of homology/identity between two sequences is a function of the number of matching or homologous positions shared by the two sequences divided by the number of positions compared X 100. For example, if 6 of 10 of the positions in two sequences are matched or homologous then the two sequences are 60% homologous/identical. Generally, a comparison is made when two sequences are aligned to give maximum homology/identity.

The term "isolated" with reference to a particular component (such as for instance, a protein, polypeptide, peptide or fragment thereof) generally denotes that such component exists in separation from -for example, has been separated from or prepared in separation from- one or more other components of its natural environment. For instance, an isolated human or animal protein, polypeptide, peptide or fragment exists in separation from a human or animal body where it occurs naturally.

The term "isolated" as used herein may preferably also encompass the qualifier "purified". As used herein, the term "purified" with reference to protein(s), polypeptide(s), peptide(s) and/or fragment(s) thereof does not require absolute purity. Instead, it denotes that such protein(s), polypeptide(s), peptide(s) and/or fragment(s) is (are) in a discrete environment in which their abundance (conveniently expressed in terms of mass or weight or concentration) relative to other proteins is greater than in a biological sample. A discrete environment denotes a single medium, such as for example a single solution, gel, precipitate, lyophilisate, *etc.* Purified peptides, polypeptides or fragments may be obtained by known methods including, for example, laboratory or recombinant synthesis, chromatography, preparative electrophoresis, centrifugation, precipitation, affinity purification, *etc.* Purified protein(s), polypeptide(s), peptide(s) and/or fragment(s) may preferably constitute by weight 10%, more preferably 50%, such as 60%, yet more preferably 70%, such as 80%, and still more preferably 90%, such as 95%, 96%, 97%, 98%, 99% or even 100%, of the protein content of the discrete environment. Protein content may be determined, *e.g.,* by the Lowry method (Lowry et al. 1951. J Biol Chem 193: 265), optionally as described by Hartree 1972 (Anal Biochem 48: 422-427). Also, purity of peptides or polypeptides may be determined by SDS-PAGE under reducing or nonreducing conditions using Coomassie blue or, preferably, silver stain.

The term "marker" or "biomarker" is widespread in the art and may broadly denote a biological molecule and/or a detectable portion thereof whose qualitative and/or quantitative evaluation in a subject is informative (e.g., predictive, diagnostic and/or prognostic) with respect to one or more aspects of the subject's phenotype and/or genotype, such as, for example, with respect to the status of the subject as to a given disease or condition.

The terms "assessing risk of' or "risk assessment", "detecting" or "detection", "screening", "diagnosing" or "diagnosis", "prognosing" or "prognosis", "predicting" or "prediction", and "monitoring" are commonplace and well-understood in medical and clinical practice.

By means of further explanation and without limitation, "assessing risk of' or "risk assessment" generally refer to an advance declaration, indication or foretelling of a disease or condition in a subject not (yet) having said disease or condition. For example, a risk assessment of a disease or condition in a subject may indicate a probability, chance or risk that the subject will develop said disease or condition, for example within a certain time period or by a certain age. Said probability, chance or risk may be indicated *inter alia* as an absolute value, range or statistics, or may be indicated relative to a suitable control subject or subject population (such as, e.g., relative to a general, normal or healthy subject or subject population). Hence, the probability, chance or risk that a subject will develop a disease or condition may be advantageously indicated as increased or decreased, or as fold-increased or fold-decreased relative to a suitable control subject or subject population. As used herein, the term "risk assessment of a disease" in a subject may also particularly mean that the subject is at risk of having said disease *(e.g.*, the risk is significantly increased vis-à-vis a control subject or subject population).

The terms "diagnosing" or "diagnosis" generally refer to the process or act of recognising, deciding on or concluding on a disease or condition in a subject on the basis of symptoms and signs and/or from results of various diagnostic procedures (such as, for example, from knowing the presence, absence and/or quantity of one or more biomarkers characteristic of the diagnosed disease or condition). As used herein, "diagnosis of a disease" in a subject may particularly mean that the subject has said disease, hence, is diagnosed as having said disease. A subject may be diagnosed as taught herein as not having said disease despite displaying one or more conventional symptoms or signs reminiscent thereof.

In the frame of the present invention, the terms "detecting" or "detection" mean in general find the presence of the disease and/or of the agent responsible therefor, and encompass both risk assessment and diagnosis, i.e. refer to the process of measuring the level of a biomarker in subjects having or not having symptoms of the disease, potentially in any subject. The term "screening" is rather used in relation to the detection in subjects having no symptoms of the disease.

The terms "prognosing" or "prognosis" generally refer to an anticipation on the progression of a disease or condition and the prospect *(e.g.,* the probability, duration, and/or extent) of recovery. A good prognosis of a disease may generally encompass anticipation of a satisfactory partial or complete recovery from said disease, preferably within an acceptable time period. A good prognosis of said disease may more commonly encompass anticipation of not further worsening or aggravating of the conditions, preferably within a given time period. A poor prognosis of a disease may generally encompass anticipation of a substandard recovery and/or unsatisfactorily slow recovery, or to substantially no recovery or even further worsening of said disease.

The terms "predicting" or "prediction" generally refer to an anticipation on the efficacy/efficiency of a medical or surgical treatment on the progression of a disease or condition and the prospect *(e.g.,* the probability, duration, and/or extent) of recovery. A good prediction may generally encompass anticipation of a satisfactory partial or complete recovery from said disease in response to the treatment, preferably within an acceptable time period. A good prediction may more commonly encompass anticipation of not further worsening or aggravating of the conditions in response to the treatment, preferably within a given time period. A poor prediction may generally encompass anticipation of a substandard recovery and/or unsatisfactorily slow recovery, or to substantially no recovery or even further worsening of said disease in response to the treatment.

The terms "monitor" or "monitoring" generally refer to observe and check the progress of a disease or condition (e.g. the presence of a pathogen) in a subject over a period of time, e.g. to evaluate the response to treatment, or to identify relapse of the disease.

A molecule or analyte, or a group of two or more molecules or analytes, is "measured" in a sample when the presence or absence and/or quantity of said molecule(s) or analyte(s) is detected or determined in the sample, preferably substantially to the exclusion of other molecules and analytes.

The terms "quantity", "amount" and "level" are synonymous and generally well-understood in the art. The terms as used herein may particularly refer to an absolute quantification of a molecule or an analyte in a sample, or to a relative quantification of a molecule or analyte in a sample, *i.e*., relative to another value such as relative to a reference value as taught herein, or to a range of values indicating a base-line expression of the biomarker. These values or ranges can be obtained from a single patient or from a group of patients.

An absolute quantity of a molecule or analyte in a sample may be advantageously expressed as weight or as molar amount, or more commonly as a concentration, e.g., weight per volume or mole per volume.

A relative quantity of a molecule or analyte in a sample may be advantageously expressed as an increase or decrease or as a fold-increase or fold-decrease relative to said another value, such as relative to a reference value as taught herein. Performing a relative comparison between first and second parameters (e.g., first and second quantities) may but need not require to first determine the absolute values of said first and second parameters. For example, a measurement method can produce quantifiable readouts (such as, *e.g.,* signal intensities) for said first and second parameters, wherein said readouts are a function of the value of said parameters, and wherein said readouts can be directly compared to produce a relative value for the first parameter vs. the second parameter, without the actual need to first convert the readouts to absolute values of the respective parameters.

The terms "patient," "subject," "individual," and the like are used interchangeably herein, and refer to any animal, or cells thereof whether *in vitro* or *in situ,* amenable to the methods described herein. As used herein they typically denote humans, but may also encompass reference to non-human animals, preferably warm-blooded animals, more preferably mammals, such as, *e.g*., non-human primates, rodents, canines, felines, equines, ovines, porcines, and the like.

A "disease" is a state of health of an animal wherein the animal cannot maintain homeostasis, and wherein if the disease is not ameliorated then the animal's health continues to deteriorate. In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health. A disease or disorder is "alleviated" if the severity of a symptom of the disease or disorder, the frequency with which such a symptom is experienced by a patient, or both, is reduced. A disease or disorder is "cured" if the severity of a symptom of the disease or disorder, the frequency with which such a symptom is experienced by a patient, or both, is eliminated.

As used herein, "treating a disease or disorder" means reducing the frequency or severity of at least one sign or symptom of a disease or disorder experienced by a subject. Disease and disorder are used interchangeably herein in the context of treatment.

### DETAILED DESCRIPTION OF THE INVENTION

According to a first aspect, the present invention concerns a support for a multiplex binding experiment functionalized with at least two different polypeptides having high affinity to their cognate binding partner.

According to a specific aspect, the at least two different polypeptides having high affinity to their cognate binding partner are provided as reagents for a multiplex binding experiment, and are further bonded to the support.

In the sense of the invention, multiplex binding experiment allows simultaneously binding of multiple kinds of molecules in a single run/cycle of the experiment, and not only one kind of molecules at a time.

In the frame of the invention, a support is defined as a physical entity able to carry at least two polypeptides. As known by the skilled person, the nature and the form of said support can vary, depending on the technique used to perform the multiplex binding experiment.

In relation to EIA, a support can be a well of a plate, advantageously of a microplate. A microplate, also called microtiter plate, microwell plate or multiwell, is a flat plate with multiple wells used as small test tubes, e.g. in ELISA assays.

A microplate typically has 6, 12, 24, 48, 96, 384 or 1536 sample wells arranged in a 2:3 rectangular matrix. Each well of a microplate typically holds somewhere between tens of nanoliters to several milliliters of liquid. Wells can be either circular or square. Microplates are manufactured in a variety of materials. The most common is polystyrene, possibly colored white by the addition of titanium dioxide for optical absorbance or luminescence detection or black by the addition of carbon for fluorescent biological assays. Polypropylene, polycarbonate, cyclo-olefins or solid pieces of glass and quartz can also be used.

In relation to lateral and vertical flow assays, the support is a membrane or a strip. This technology is based on a series of capillary beds, such as pieces of porous paper, microstructured polymer, or sintered polymer. According to a specific embodiment, the support is made of cellulose or a derivative thereof, advantageously nitrocellulose.

In relation to the Luminex^{™} technology, the support is made of microcarriers or microbeads.

By "microcarrier", it is herein referred to any type of particle microscopic in size, typically with the largest dimension being from 100 nm to 300 µm, preferably from 1 µm to 200 µm. The microcarrier may be of any shape but has preferably a spherical shape (microbeads) or the form of a wafer, e.g. a disk-like shape.

The microcarriers may be made from or comprise any material routinely used in high-throughput screening technology and diagnostics, e.g. polystyrene or silica.

According to specific embodiments, the support is polymeric, wherein the polymers are advantageously selected from the group consisting of carbohydrate-based polymers, polyaliphatic alcohols, poly(vinyl) polymers, polyacrylic acids, polyorganic acids, polyamino acids, co-polymers, block copolymers, tertpolymers, polyethers, naturally occurring polymers, polyimids, surfactants, polyesters, branched polymers, cyclo-polymers, polyaldehydes and mixtures thereof, brominated polystyrene, polyacrylic acid, polyacrylonitrile, polyamide, polyacrylamide, polyacrolein, polybutadiene, polycaprolactone, polyester, polyethylene, polyethylene terephthalate, polydimethylsiloxane, polyisoprene, polyurethane, polyvinylacetate, polyvinylchloride polyvinylpyridine, polyvinylbenzylchloride, polyvinyltoluene, polyvinylidene chloride, polydivinylbenzene, polymethylmethacrylate, polylactide, polyglycolide, poly(lactide-co-glycolide), polyanhydride, polyorthoester, polyphosphazene, polyphosophaze, poly-(styrene-co-vinylbenzyl chloride-co-acrylic acid) (85:10:5 molar ratio), poly(styrene-co-acrylic acid) (99:1 molar ratio), poly(styrene-co-methacrylic acid) (90:10 molar ratio), poly(styrene-co-acrylic acid-co-m&p-divinylbenzene) (89:10:1 molar ratio), poly-(styrene-co-2-carboxyethyl acrylate) (90:10 molar ratio), poly(methyl methacrylate-co-acrylic acid) (70:30 molar ratio) and poly(styrene-co-butyl acrylate-co-methacrylic acid) (45:45:10 weight ratio) synthetic polymers polystyrene, polyacrylamide, polyacrylate, latex, and any combinations or modifications thereof.

According to some embodiments, the support may comprise plastic, cellulose, dextran, dextran cross linked with epichlorohydrin, agarose, acrylamide, glass, polystyrene, polyethylene glycol, Teflon, or nylon.

According to the invention, the support is functionalized with at least two different polypeptides. By "functionalized", it is herein referred to a non-covalent (e.g. electrostatic or ionic) or covalent (e.g. chemical) bonding between the support and the polypeptides. This encompasses adsorption, chemical bonding, especially through thiol, carboxyl or amine functions, and bonding by Ultra-Violet (UV) irradiation.

According to a specific embodiment, the polypeptide(s) can comprise or be fused to an entity having high affinity for the support, e.g. a cellulose-binding domain. As known in the art, the resulting complex (polypeptide + entity) can be obtained recombinantly, by chemical synthesis or through chemical conjugation methods. Advantageously, the entity is a peptide or a protein fused to the polypeptide(s).

According to another embodiment, the support according to the invention is functionalized on 2 distinct sites thereof, with the at least two different polypeptides. By "on 2 distinct sites thereof', it is herein referred to the fact that each of the polypeptides is individualized on the support, i.e. physically distinguishable. In other words, said sites constitute distinct binding areas on the support.

In that specific case and when the support is a set of microcarriers, each different polypeptide can be immobilized on microcarriers differentially labeled. As an example, the microcarriers may further be encoded, to facilitate their identification. Preferably, a microcarrier to be used in the frame of the invention is encoded in such a way that its function, i.e. the polypeptide functionalized thereon, can be determined by reading the code, preferably using optical means.

Concerning wells or membranes, the different polypeptides can be immobilized on the support in the form of distinct dots or lines, with a sufficient distance between each other.

Moreover, the support according to the invention comprises at least two different polypeptides having high affinity to their cognate binding partner, with preferably at least one polypeptide, more preferably both, having a dissociation constant (Kd) for its/their binding partner inferior or equal to 10⁻¹⁰ M, advantageously inferior or equal to 10⁻¹¹ M, more advantageously inferior or equal to 10⁻¹² M.

As known in the art, the binding affinity is the strength of the binding interaction between a single biomolecule, in the present case a polypeptide, to its ligand/binding partner. Binding affinity is typically measured and reported by the equilibrium dissociation constant (Kd), which is used to evaluate and rank order strengths of bimolecular interactions. The smaller the Kd value, the greater the binding affinity of the ligand for its target. The larger the Kd value, the weaker the target molecule and ligand are attracted to and bind to each other.

Binding affinity is influenced by non-covalent intermolecular interactions such as hydrogen bonding, electrostatic interactions, hydrophobic and Van der Waals forces between the two molecules. In addition, binding affinity between a ligand and its target molecule may be affected by the presence of other molecules.

The measurement of a dissociation constant (Kd) as determined by kinetics is routine for the skilled person. There are many techniques available for measuring binding affinity and dissociation constants, such as ELISAs, gel-shift assays, pull-down assays, equilibrium dialysis, analytical ultracentrifugation, Surface Plasma Resonance (SPR), spectroscopic assays and Isothermal Titration Calorimetry (ITC).

The Kd value can correspond to the one disclosed in the literature or determined in standard conditions, i.e. using the polypeptide and its binding partner in the optimal conditions for their binding, especially concerning buffer, pH, temperature. Preferably and in the frame of the invention, it corresponds to the value determined in the absence of destabilizing reagents and in the presence of all the cofactors to be used in the binding assay.

According to preferred embodiments of the invention, the support comprises at least one polypeptide having a dissociation constant (Kd) for its binding partner inferior or equal to 10⁻¹² M, advantageously inferior or equal to 10⁻¹³ M, more advantageously inferior or equal to 10⁻¹⁴ M, or even inferior or equal to 10⁻¹⁵ M. According to a preferred embodiment, the at least one or two polypeptides, or even all the polypeptides, have a dissociation constant (Kd) for their respective cognate binding partner inferior or equal to 10⁻¹⁰ M, 10⁻¹¹ M or 10⁻¹² M, advantageously inferior or equal to 10⁻¹³ M, more advantageously inferior or equal to 10⁻¹⁴ M, or even inferior or equal to 10⁻¹⁵ M.

On another hand, since the support comprises at least two different polypeptides having high affinity to their cognate binding partner, it is preferred that each polypeptide has high affinity specifically for its cognate partner. In other words, the dissociation constant (Kd) of a given polypeptide for the binding partners of the other polypeptides (non-cognate) present on the support is advantageously higher, i.e. greater than 10⁻¹² M, more advantageously greater than 10⁻¹¹ M, 10⁻¹⁰ M, 10⁻⁹ M, or even 10⁻⁸ M, 10⁻⁷ M, 10⁻⁶ M.

According to one further embodiment, it is preferred that the ratio between the Kd value of a given polypeptide for its cognate binding partner and the Kd value of said polypeptide for its non-cognate binding partner(s), measured in the same experimental conditions, is inferior or equal to 10⁻², advantageously inferior or equal to 10⁻³, more advantageously inferior or equal to 10⁻⁴, or even inferior or equal to 10⁻⁵. As an example, in case the Kd value between a first polypeptide and its cognate partner is equal to 10⁻¹⁴ M, the second or further polypeptide(s) will be chosen so that the Kd value between the first polypeptide and its non-cognate partner(s), i.e. the partner(s) of the second or further polypeptide(s), is equal to 10⁻¹² M, advantageously 10⁻¹¹ M, more advantageously 10⁻¹⁰ M, still more advantageously 10⁻⁹ M.

As used therein, the polypeptides belong to a binding pair or couple, i.e. they are the first member of a binding pair or couple. Advantageously, they are a member of a high affinity complex, possibly of a protein-protein complex.

According to a specific embodiment, at least one polypeptide is a member of a toxin-antitoxin couple or system, advantageously polypeptidic toxin-antitoxin couple or system.

According to one embodiment, the invention concerns a support for a multiplex binding experiment functionalized with at least two different polypeptides having high affinity to their cognate binding partner. According to a preferred embodiment, each of said two polypeptides is a bacteriocin or its cognate Immunity protein (Im). In the present application, "Immunity protein" or "Immunity polypeptide" have the same meaning and are used interchangeably.

According to a first embodiment, the support is functionalized with bacteriocins, fragments or mutants thereof.

Bacteriocins (or protein antibiotics) are a large and diverse family of multidomain polypeptidic toxins. According to a preferred embodiment, bacteriocins to be used in the frame of the invention are nuclease bacteriocins (NB), i.e. toxins that target nucleic acids (DNA or RNA in case of ribonucleases) in the cytoplasm of bacteria, also called endonucleases.

As shown in the examples below or in previous work (Sharp et al., PLOS Computational Biology, 2017, https://doi.org/10.1371/iournal.pcbi.1005652), bacteriocins have been identified in a large number of bacterial species, exclusively in γ-proteobacteria, and are particularly abundant in *Enterobacteriacae* and *Pseudomonodaceae* families.

According to a specific embodiment, the polypeptide used in the context of the invention is a fragment or a mutant of a bacteriocin, advantageously a fragment or a mutant able to bind the cognate Immunity polypeptide with high affinity, more advantageously with a dissociation constant (Kd) as defined above. It has been shown that the region responsible for this binding, also called Immunity Protein Exosite, is located in the C-terminal domain of the bacteriocin.

As an example and in relation to the *Escherichia coli* Colicin E2 (noted ColE2), Joshi et al. (J Mol Biol., 2015, 427(17), 2852-66) have reported that the region responsible for this binding corresponds to residues 520 to 553 of ColE2. This sequence further corresponds to residues 325 to 358 of SEQ ID NO: 1.

Therefore and according to a preferred embodiment, the fragment or mutant of the bacteriocin contains the Immunity Protein Exosite. As shown by Joshi et al. (J Mol Biol., 2015, 427(17), 2852-66), the relevant domain can be identified in any bacteriocin by sequence alignment. More generally, a fragment or a mutant of a bactericin to be used in the frame of the invention contains the C-terminal part thereof.

As known in the art, the nuclease active site (or cytotoxic domain) of bacteriocins is also located in their C-terminal part. As shown in figure S1 of Sharp et al. (PLOS Computational Biology, 2017, https://doi.org/10.1371/journal.pcbi.1005652), multiple sequence alignments of cytotoxic domains have identified conserved motifs identifiable in the cytotoxic domain of all types of nucleases, i.e. HNH-type DNAses, non HNH-type DNAses, rRNAses and tRNAses.

According to a preferred embodiment, the fragment or mutant of the bacteriocin corresponds to or contains the cytotoxic domain of the bacteriocin.

According to a specific embodiment and in relation to HNH-type DNAses, the bacteriocin or a fragment thereof contains a 30-residue motif (also referred to as the ββα-Me motif) of sequence HH-XXXXXXXXXXXXXX-N-XXXXXXXX-H-XXX-H (SEQ ID NO: 28).

According to a further embodiment and in relation to HNH-type DNAses, the polypeptides used in the frame of the present invention are mutated so as to be deprived of cytotoxic activity. As reported by Walker et al. (Nucleic Acid Research, 2002, 30(14), 3225-34) and in relation to *E. coli* Colicin E9 (noted ColE9), the enzymatic domain thereof can be inactivated by e.g. changing a Histidine to Alanine in the N-part of SEQ ID NO: 28 to prevent the DNase activity. As a result, a bacteriocin can contain the sequence HA-XXXXXXXXXXXXXX-N-XXXXXXXX-H-XXX-H (SEQ ID NO: 29) or AH-XXXXXXXXXXXXXX-N-XXXXXXXX-H-XXX-H (SEQ ID NO: 30), advantageously HA-XXXXXXXXXXXXXX-N-XXXXXXXX-H-XXX-H (SEQ ID NO: 29).

Bacteriocins corresponding to DNAses, especially HNH-type DNAses, and rRNAses, together with their cognate Immunity proteins, are advantageously used in the frame of the invention since the Exosite and the catalytic domain are both in the C-terminal part of the bacteriocin but distinct so that the catalytic site can be inactivated without affecting the ability of the bacteriocin to bind to the Immunity protein.

Of particular interest are the HNH-type DNAses because they can be easily identified based on the HNH motif:
Among *E. coli* colicins, those having an endonuclease (non-specific DNAse) activity are preferred, i.e. the enzymatic E type colicin ColE2, ColE7, ColE8 or ColE9 (Kd in the range of 10⁻¹⁵ M). According to a preferred embodiment, a polypeptide consisting of or comprising the C-terminal part of colicins E2, E7, E8 or E9, advantageously mutated so as to be deprived of cytotoxic activity, is used. According to a preferred embodiment, the polypeptides used in the frame of the invention have or comprise a sequence selected in the following group:
- A sequence corresponding to amino acids 254 to 386 of SEQ ID NO: 1 (ColE2);
- A sequence corresponding to amino acids 254 to 386 of SEQ ID NO: 2 (ColE7);
- A sequence corresponding to amino acids 251 to 383 of SEQ ID NO: 3 (ColE8);
- A sequence corresponding to amino acids 251 to 383 of SEQ ID NO: 4 (ColE9).

Non-limiting further examples of bacteriocins which can be used in the invention are:
- those isolated from *Pseudomonas aeruginosa,* advantageously the bacteriocins named pyocins such as the HNH-type DNAses SI, S2, and AP41, more advantageously the bacteriocin named ColAP41 in the present application;
- those isolated from *Pseudomonas syringae,* advantageously the bacteriocin named ColSyr in the present application from *Pseudomonas syringae* B728A;
- those isolated from *Pectobacterium carotovorum,* advantageously the bacteriocin named ColErW in the present application;
- those isolated from *Pseudomonas sp Leaf83,* advantageously the bacteriocin named ColLeaf in the present application; and/or
- those isolated from *Photorhabdus khanii,* advantageously the bacteriocin named ColKhan in the present application.

Alternatively, the bacteriocins used in the frame of the invention can have or comprise a sequence selected in the following group:
- A sequence corresponding to amino acids 255 to 390 of SEQ ID NO: 9 (ColAP41);
- A sequence corresponding to amino acids 255 to 388 of SEQ ID NO: 10 (ColSyr);
- A sequence corresponding to amino acids 255 to 383 of SEQ ID NO: 11 (ColErw);
- A sequence corresponding to amino acids 261 to 394 of SEQ ID NO: 12 (ColLeaf);
- A sequence corresponding to amino acids 261 to 393 of SEQ ID NO: 13 (ColKhan).

Said specific sequences correspond to fragments (C-terminal part) of natural bacteriocins, of size ranging from 129 to 136 amino acids, containing the cytotoxic domain but further mutated to be deprived of cytotoxic activity.

Alternatively, *E. coli* colicins having ribonuclease (RNAse, especially rRNAse) activity can be used, in particular Col E3 (Kd in the range of 10⁻¹² M), E4, E5 or E6, or fragments thereof capable of binding their cognate Immunity protein with high affinity, more advantageously with a dissociation constant (Kd) as disclosed above.

Similarly, *P. aeruginosa* pyocins having ribonuclease activity may be used, in particular SD1, SD2 or SD3 having tRNAse activity.

Klebicins produced by *Klebsiella pneumonia* are different types of bacteriocins which can also be used.

Of particular interest in the frame of the invention are fragments of a natural bacteriocin (with an average size of around 650 aa) having the following characteristics:
- having a preferred size of 100 to 150 amino acids, preferably of 125 to 140 amino acids, more preferably of 130 to 135 amino acids ;
- corresponding to its C-terminal part and/or containing the Immunity Protein binding site and/or the cytotoxic domain;
- being deprived of cytotoxic activity because of one or more mutations, whereas said mutation does not affect its binding to its cognate Immunity polypeptide.

According to a specific embodiment, the at least two bacteriocins, fragments or mutants thereof are chosen so that they share an identity over their Immunity Protein Exosite or binding site equal to or less than 70%, 65%, 60%, 55%, 50%, 45% or even 40%.

According to another embodiment of the invention, the support is functionalized with the cognate Immunity proteins (Im) of bacteriocins, and possibly fragments or mutants thereof. Fragments or mutants of interest are those having kept the ability to bind the corresponding bacteriocin (or mutant or fragment thereof).

According to a general definition, the Immunity proteins to be used in the present invention are the cognate binding partners of the bacteriocins as disclosed above. In practice, they originate from the same bacteria and the corresponding genes are advantageously located within 500 nucleotides in the genome.

According to particular embodiments, the at least two Immunity polypeptides to be used in the frame of the present invention have or comprise a sequence selected in the following group:
- A sequence corresponding to amino acids 329 to 413 of SEQ ID NO: 5 or residues 17 to 101 of SEQ ID NO: 19 (Im2);
- A sequence corresponding to amino acids 329 to 414 of SEQ ID NO: 6 or residues 17 to 102 of SEQ ID NO: 20 (Im7);
- A sequence corresponding to amino acids 329 to 412 of SEQ ID NO: 7 or residues 17 to 100 of SEQ ID NO: 21 (Im8);
- A sequence corresponding to amino acids 329 to 407 of SEQ ID NO: 8 or residues 17 to 95 of SEQ ID NO: 22 (Im9);
- A sequence corresponding to amino acids 333 to 423 of SEQ ID NO: 14 or residues 17 to 107 of SEQ ID NO: 23 (ImAP41);
- A sequence corresponding to amino acids 333 to 420 of SEQ ID NO: 15 or residues 17 to 104 of SEQ ID NO: 24 (ImSyr);
- A sequence corresponding to amino acids 333 to 423 of SEQ ID NO: 16 or residues 17 to 107 of SEQ ID NO: 25 (ImErw);
- A sequence corresponding to amino acids 347 to 430 of SEQ ID NO: 17 or residues 45 to 128 of SEQ ID NO: 26 (ImLeaf); and
- A sequence corresponding to amino acids 347 to 429 of SEQ ID NO: 18 or residues 45 to 127 of SEQ ID NO: 27 (ImKahn).

Said specific sequences correspond to fragments of size ranging from 79 to 91 amino acids, originating from natural Immunity proteins having a mean size of 101 amino acids.

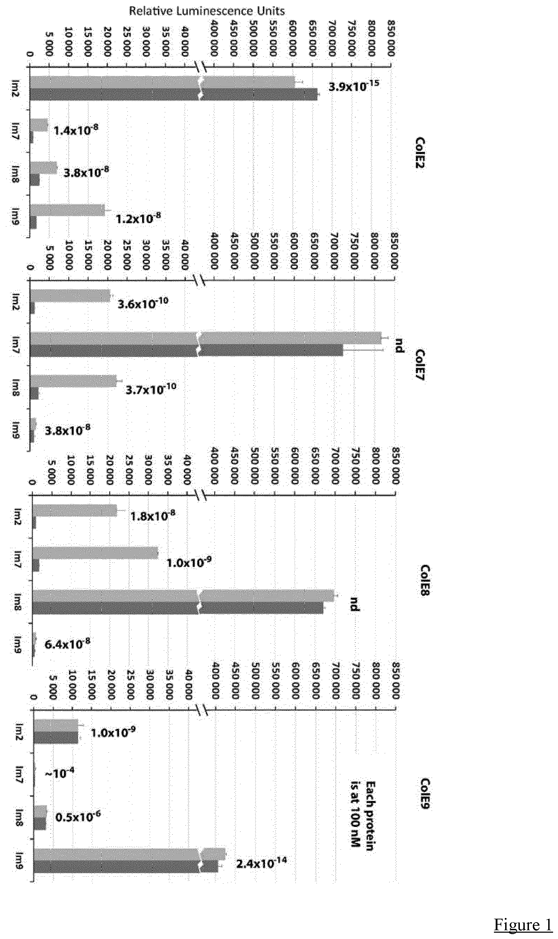

According to a specific embodiment, the Immunity proteins, a mutant or a fragment thereof, contain the sequence involved in the binding to the corresponding bacteriocin, advantageously a DNAse Binding Region. Such a region can be identified by sequence alignment as shown in Figure ID of Joshi et al. (J Mol Biol., 2015, 427(17), 2852-66) and has a proposed consensus sequence as shown in Figure 1C of Sharp et al. (PLOS Computational Biology, 2017, https://doi.org/10.1371/journal.pcbi.1005652).

In relation to *E. coli* Immunity protein 2 (noted Im2), the cognate binding partner of ColE2, this region (DNAse Binding Region) corresponds to residues 29 to 58 of Im2. This sequence further corresponds to residues 356 to 385 of SEQ ID NO: 5 or residues 44 to 73 of SEQ ID NO: 19.

According to a specific embodiment, the at least two Immunity proteins, fragments or mutants thereof, are chosen so that they share an identity over their DNAse Binding Region equal to or less than 70%, 65%, 60%, 55% or even 50%.

According to a preferred embodiment, the different (at least two) couples of bacteriocin/Immunity protein to be used are selected based on both criteria, i.e.:
- The bacteriocins, fragments or mutants thereof, share an identity over their Immunity Protein Exosite equal to or less than 70%, 65%, 60%, 55%, 50%, 45% or even 40%; and
- The Immunity proteins, fragments or mutants thereof, share an identity over their DNAse Binding Region equal to or less than 70%, 65%, 60%, 55% or even 50%.

Because of its use in multiplex binding experiments, the support of the invention comprises at least 2 different polypeptides. In other words, it can comprise, 2, 3, 4, 5, 6, 7, 8, 9, 10 or even more polypeptides, among which at least 2, and preferably all of them, are different. In the frame of the invention, "different" specially refers to their ability to have a high affinity with a different cognate binding partner.

According to a specific embodiment, all the polypeptides on the support are bacteriocins and/or their cognate Immunity polypeptides (Im).

Alternatively, said support comprises at least two polypeptides which are bacteriocins or their cognate Immunity proteins but also further polypeptide(s) having high affinity to its/their cognate binding partner as defined above.

More generally, other nuclease-inhibitor complexes can be used as e.g. the barnase-barstar complex (Kd in the range of 10⁻¹⁴ M) or the ribonuclease inhibitor (RI) binding angiogenin.

Another system is the Dockerin/Cohesin complex involved in the cellulosome.

According to a further embodiment, one such polypeptide is a binding partner of biotin, advantageously avidin, streptavidin, neutrAvidin (the deglycosylated version of avidin), or an antibiotin antibody. As known by the skilled person, homo-tetramers of said proteins have a high affinity for biotin, with a dissociation constant (K_{d}) on the order of 10⁻¹⁴ or 10⁻¹⁵ M.

As illustrated in the present application, the use of complexes between bacteriocins, advantageously the inactivated cytotoxic domain of DNAse bacteriocins, and their immunity polypeptides are of particular interest for among reasons:
- the availability of a large panel of members of the same family having high affinity with their cognate binding partner but low cross-reactivity with the non-cognate binding partners;
- the small size of said polypeptides, advantageous in terms of production, coupling and coating of the support;
- their monomeric state which allows to control their coupling with other molecules;

Advantageously, at least one polypeptide, the at least 2 polypeptides or even all the polypeptides on the support have a sequence selected in the group consisting of: SEQ ID NO: 1 to SEQ ID NO: 27.

Merely to illustrate all the possibilities offered by the present invention and in relation to the ColE2/E7/E8/E9 couples, a support according to the invention can comprise:
- SEQ ID NO: 1 (ColE2) and SEQ ID NO: 2 (ColE7);
- SEQ ID NO: 1 (ColE2) and SEQ ID NO: 3 (ColE8);
- SEQ ID NO: 1 (ColE2) and SEQ ID NO: 4 (ColE9);
- SEQ ID NO: 2 (ColE7) and SEQ ID NO: 3 (ColE8);
- SEQ ID NO: 2 (ColE7) and SEQ ID NO: 4 (ColE9);
- SEQ ID NO: 3 (ColE8) and SEQ ID NO: 4 (ColE9);
- SEQ ID NO: 5 (Im2) and SEQ ID NO: 6 (Im7);
- SEQ ID NO: 5 (Im2) and SEQ ID NO: 7 (Im8);
- SEQ ID NO: 5 (Im2) and SEQ ID NO: 8 (Im9);
- SEQ ID NO: 6 (Im7) and SEQ ID NO: 7 (Im8);
- SEQ ID NO: 6 (Im7) and SEQ ID NO: 8 (Im9);
- SEQ ID NO: 7 (Im8) and SEQ ID NO: 8 (Im9).

According to other specific embodiments, a support according to the invention can comprise:
- SEQ ID NO: 1 (ColE2) and SEQ ID NO: 2 (ColE7) and SEQ ID NO: 3 (ColE8) and SEQ ID NO: 4 (ColE9); or
- SEQ ID NO: 5 (Im2) and SEQ ID NO: 6 (Im7) and SEQ ID NO: 7 (Im8) and SEQ ID NO: 8 (Im9).

When used in the context of a lateral flow assay, the following order can be adopted:
- SEQ ID NO: 2 (ColE7) then SEQ ID NO: 1 (ColE2) then SEQ ID NO: 3 (ColE8) then SEQ ID NO: 4 (ColE9), or SEQ ID NO: 6 (Im7) then SEQ ID NO: 5 (Im2) then SEQ ID NO: 7 (Im8) then SEQ ID NO: 8 (Im9); or
- SEQ ID NO: 3 (ColE8) then SEQ ID NO: 1 (ColE2) then SEQ ID NO: 4 (ColE9) then SEQ ID NO: 2 (ColE7) or SEQ ID NO: 7 (Im8) then SEQ ID NO: 5 (Im2) then SEQ ID NO: 8 (Im9) then SEQ ID NO: 6 (Im7).

A specific embodiment of the invention is based on the use of the couples ErW and AP41 as defined above, advantageously combined with other bacteriocin/immunity protein couple(s), more advantageously those disclosed above.

For any useful purpose, especially purification and labeling (e.g. by fluorescence or bioluminescence), said polypeptides can be linked or fused (recombinantly, by chemical synthesis or through chemical conjugation methods) to another entity, also named a tag. Examples of such tags are: AviTag, Calmodulin-tag, polyglutamate tag, E-tag, FLAG-tag, HA-tag, His-tag, MYC-tag, NE-tag, S-tag, SBP-tag, Softag 1, Softag 3, Spot-tag, Strep-tag, TC tag, Ty tag, V5 tag, VSV-tag, Xpress tag, Isopeptag, SpyTag, SnoopTag, SnoopTagJr, DogTag, Biotin Carboxyl Carrier Protein (BCCP), Glutathione-S-transferase, HaloTag, Maltose binding protein (MBP), Nus, Thioredoxin, Fc, Green fluorescent protein (GFP), Luciferase (e.g. RLuc8 for Renilla Luciferase or FLuc for Firefly Luciferase), mCherry, horseradish peroxidase (HRP), alkaline phosphatase (AP), glucose oxidase. A radioactive isotope, a DNA reporter, a fluorogenic reporter (e.g. phycoerythrin), or an electro-chemiluminescent tag can also be used.

In a remarkable manner, the stoichiometry of the multiplex binding experiment can be controlled based on the control of the distribution of the different polypeptides on the support according to the invention, especially by controlling the ratio between said polypeptides.

According to another aspect, the present invention relates to a reaction mixture to be used together with the support as disclosed above. In other words, said reaction mixture comprises the cognate binding partners of the at least two polypeptides immobilized on the support.

Even if mixtures can be used, it is preferred that when the polypeptides on the support correspond to bacteriocins (or fragments or mutants thereof), then the reaction mixture contains the cognate Immunity proteins (or fragments or mutants thereof). Conversely, when the polypeptides on the support correspond to Immunity proteins (or fragments or mutants thereof), then the reaction mixture contains the cognate bacteriocins (or fragments or mutants thereof).

It is to be noted that such a reaction mixture can be ready to use or can be prepared extemporally by mixing the different binding partners just before the multiplex binding experiment. Such a reaction mixture can be in a liquid form or lyophilized.

According to a specific embodiment, at least one of the at least 2 binding partners is a polypeptide. Preferably, all the binding partners are polypeptides.

According to another embodiment, at least one of the at least 2 binding partners comprises or is fused to at least another entity which can serve as a tag for purification and/or labeling purposes, as listed above.

According to a specific embodiment, at least one of the at least 2 binding partners, advantageously the at least 2 binding partners, further comprises or is fused to a molecule of interest in relation to binding experiments. For clarity purposes and in the rest of the description, the molecule of interest is named an analyte-capture entity. It is to be noted that at least the 2 binding partners of the polypeptides on the support can comprise the same analyte-capture entity but advantageously different analyte-capture entities.

After incubation, the support and the reaction mixture which forms a multiplex capture reagent can have a series of applications.

The multiplex capture reagent according to the invention allows immobilizing said analyte-capture entities on the support, while controlling their location, orientation and stoichiometry.

The multiplex capture reagent can be used for detecting, studying or even isolating, and purifying the binding partner of said analyte-capture entities. For clarity purpose and in the rest of the description, the binding partner of the analyte-capture entity is named an analyte.

Therefore and as previously mentioned, it can be used for detection or isolation of analytes contained in a sample or for studying the binding of said analytes with the corresponding analyte-capture entity.

In the frame of the invention, the analyte and/or the analyte-capture entity can be any type of molecules in terms of nature and function, in particular chemical compounds, polypeptides (e.g. viral capsid proteins, glycoproteins, immunoglobulins), metabolites, viruses, biomarkers, lipids, nucleic acids (RNA, miRNA, DNA, aptamers, exosomes, ...), imprinted polymers, functionalized nanocages or nanoparticles.

According to a specific embodiment, the analyte(s)/analyte-capture entity(ies) can be e.g. a receptor/ligand couple or an antibody/antigen couple. According to more specific embodiments, the analyte-capture entity is an antigen and the analyte an antibody or the analyte-capture entity is an antibody and the analyte an antigen.

According to a preferred embodiment, the analyte(s) is in solution. According to another embodiment, the analyte(s) is obtained from a sample or biological sample. The terms "sample" or "biological sample" as used herein include any biological specimen obtained from a subject. Samples may include, without limitation, whole blood, plasma, serum, red blood cells, white blood cells *(e.g.*, peripheral blood mononuclear cells), saliva, urine, stool *(i.e.,* faeces), tears, mucus, sweat, sebum, nipple aspirate, ductal lavage, tumour exudates, synovial fluid, cerebrospinal fluid, lymph, fine needle aspirate, amniotic fluid, any other bodily fluid, cell lysates, cellular secretion products, inflammation fluid, vaginal secretions, pleural fluid, spinal fluid, gastric fluid, sweat, semen, fluid from ulcers and/or other surface eruptions, blisters, abscesses, and/or extracts of tissues, such as biopsies of normal, malignant, and/or suspect tissues.

Especially for diagnosis purposes, the analyte-capture entity is preferably a polypeptide, which can be an antigen or an antibody, depending on the techniques used.

In certain embodiments, the analyte may be an antibody and the analyte-capture entity may be an antigen recognized by the antibody analyte. In further embodiments the analyte may be a human antibody; in certain embodiments the analyte antibody may belong to the immunoglobulin (Ig) group A, D, E, G or M. The antibody may be a non-human antibody in other embodiments. In addition, the antibody may be a chimeric antibody.

In certain aspects, the pathogen may be detected by binding of an antibody to a pathogen antigen, e.g. a surface antigen or a secreted antigen. The pathogen antigen may be a protein, glycoprotein, polysaccharide, lipopolysaccharide or lipid. The surface antigen may belong to, but is not limited to, a component of the pathogen's coat, capsule, cell wall, flagella, fimbriae, toxin, pili, cytoplasmic membrane, outer membrane, peptidoglycan layer, periplasmic space, S-layer, capsid, protein coat, or envelope. Said antigen can be present on the pathogen surface or secreted by the pathogen.

In still other embodiments, the analyte may be a pathogen, especially a bacterium, a virus or a parasite. It may be selected from the list of, but is not restricted to *Bordetella (e.g. Bordetella pertussis*), *Borrelia, Brucella, Campylobacter, Chlamydia, Chlamydophila, Clostridium, Corynebacterium, Enterococcus, Escherichia* (*e.g. Escherichia coli*), *Francisella, Haemophilus, Helicobacter, Legionella, Leptospira, Listeria, Mycobacterium* (*e.g. Mycobacterium leprae* or *Mycobacterium ulcerans*), *Mycoplasma, Neisseria, Pseudomonas, Rickettsia, Salmonella, Shigella (e.g. Shigella dysenteriae), Staphylococcus, Streptococcus (e.g. Streptococcus pneumoniae), Treponema, Vibrio (e.g. Vibrio cholerae), Yersinia, Plasmodium falciparum, Plasmodium vivax, Plasmodium ovale, Plasmodium malariae, Plasmodium knowlesi, Schistosomiasis mansoni, Schistosomiasis japonicum, Schistosomiasis haematobium, Trypanosoma cruzi,* arboviruses such as *Dengue virus, Zika virus, Yellow fever virus* and *Chikungunya virus, Human Immunodeficiency Virus* (e.g. HIV1 or HIV2), *Hepatitis virus* (e.g. HCV or HBV), *Human T cell leukemia*/ *lymphoma virus* (HTLV), *Influenza virus, Norovirus,* or *Ebola virus.*

It is to be noted that a given multiplex diagnostic test can be based on the combined detection of both antigens and antibodies. Moreover, the analytes can originate from the same pathogen or from different pathogens.

Multiplex diagnostic tests, e.g. in the form of rapid diagnostic tests, are useful in medicine and veterinary, especially in the field of cancer, infectious diseases, metabolic diseases, cardiac diseases, or for performing companion tests. Other fields of interest concern e.g. process control (quality control) or environmental control.

In relation to infectious diseases and merely for illustrative purposes, multiplex diagnostic tests can be dedicated to the specific detection of:
- sexually transmitted diseases;
- viral infections such as HIV1, HIV2, HCV, HBV, HTLV;
- infections due to arboviruses: Dengue, Zika, Yellow fever, Chikungunya;
- pathogens responsible for fever/sepsis: malaria versus bacterial infections versus viral infections versus leptospirosis versus neglected tropical diseases.

Analytes which can be detected according to the invention are for example:
- the p24 component of Human Immunodeficiency Virus (HIV) capsid;
- the Influenza virus nucleoprotein (NP);
- the Norovirus capsid protein VP1; and/or
- the Ebola virus glycoprotein.

In certain embodiments, the support and the reaction mixture according to the invention are used in multiplex diagnostic tests, advantageously in enzymatic immunoassays (EIA), bead-based immunoassays (e.g. Luminex^{®}), or vertical flow (VF) or lateral flow (LF) assays including assays performed using the Dual Path Platform (DPP^{®}) as disclosed in e.g. US 7,189,522.

Said immunoassays require a detection entity, advantageously a labeled detection entity. According to a specific embodiment, the detection entity is an antibody or an antigen able to bind to the analyte (i.e. an antigen or an antibody), without disturbing its binding to the analyte-capture entity. More generally, the detection entity is defined as the analyte-capture entity above, taking into account that it further comprises a labeled moiety.

The presence and/or quantity of the analytes bound on the support is evaluated through the signal evaluation, i.e. the measurement of the labeling (by fluorescence, bioluminescence, electrochemiluminescence, colorimetry, ...) of the detection entity during the so-called detection or revelation step.

The detection entity can be a labeled antibody, advantageously a monoclonal antibody, more advantageously chosen in the following group: an antigen-binding fragment (Fab), a single chain variable fragment (scFv), a diabody and a nanobody. The different options for labeling said antibodies are common knowledge in the art. Said antibody can be fused (recombinantly, by chemical synthesis or through chemical conjugation methods) to a tag as disclosed above.

In certain embodiments and without being limitative, the detection entity can comprise a bioluminescent, fluorescent, electrochemiluminescent, or colorimetric reporter which may be selected from the list of, but is not restricted to Green fluorescent protein (GFP), Luciferase, mCherry, horseradish peroxidase (HRP), alkaline phosphatase (AP), glucose oxidase. A radioactive isotope, a DNA reporter, a fluorogenic reporter (e.g. phycoerythrin), or an electro-chemiluminescent tag can also be used.

According to a specific embodiment, the detection entity may comprise a luciferase reporter. The luciferase reporter may comprise a Renilla, Gaussia, Photinus, or Cypridina luciferase. In other embodiments the luciferase may comprise a luciferase from one of the following organisms: Photinus pyralis, Luciola cruciata, Luciola italica, Luciola lateralis, Luciola mingrelica, Photuris pennsylvanica, Pyrophorus plagiophthalamus, Phrixothrix hirtus, Renilla reniformis, Gaussia princeps, Metridia longa or Oplophorus gracilorostris. In other embodiments the luciferase may be North American firefly luciferase, Japanese firefly (Genji-botaru) luciferase, Italian firefly Luciferase, Japanese firefly (Heike) luciferase, East European firefly luciferase, Pennsylvania firefly luciferase, Click beetle luciferase, Railroad worm luciferase, Renilla luciferase, Rluc8 (mutant of Renilla luciferase), Green Renilla luciferase, Gaussia luciferase, Gaussia-Dura luciferase, Cypridina luciferase, Cypridina (Vargula) luciferase, Metridia luciferase, Oplophorus luciferase (OLuc) or a bacterial luciferase. Substrates used in certain embodiments may include Luciferin, Coelenterazine, Vargulin or any compounds based on these substrates. Synthetic luciferases, e.g. NanoLuc^{®} having furimazine as a substrate, can also be used.

According to a specific embodiment and in relation to e.g. antigenic EIA tests or bead-based immunoassays such as Luminex^{®}, the analyte to be detected, possibly from a sample, is an antigen.

In that case and according to a preferred embodiment, the analyte-capture entity is an antibody (Ab), advantageously a monoclonal antibody, more advantageously chosen in the following group: an antigen-binding fragment (Fab), a single chain variable fragment (scFv), a diabody and a nanobody. Nanobodies, also named single domain antibodies (sdAb) or *V_{H}H* fragments, consist of one heavy chain variable domain, are very stable and relatively small peptide chains of about 110 amino acids which preserve the antigen-binding capacity. Alternatively, synthetic binders also named non-immunoglobulin scaffolds such as Affibodies, Adnectin, Affilin, Anticalin, alphabody or DARPin can be used.

In that context, the detection entity is usually a labeled antibody.

On the contrary, in serological EIA tests, the analytes are usually antibodies possibly contained in a sample, e.g. IgM and/or IgG, and their detection requires a detection entity, i.e. a labeled antibody (indirect detection) or a labeled antigen (sandwich detection), able to further bind said antibodies.

Also in relation to serological EIA tests, the analyte-capture entity can be an antibody or an antigen.

Basically and especially in relation to EIA tests, the diagnostic procedure includes the analyte capture and the addition of the detection entity before revelation. The revelation is usually preceded by a washing step.

According to a first embodiment, the support functionalized with the polypeptides (e.g. ColE proteins or fragments thereof) is coincubated with the binding partners thereof (e.g. the cognate Im proteins) fused to the analyte-capture entities (e.g. an antibody or an antigen) and the analytes or the sample containing said analytes (e.g. an antigen or an antibody). The detection entity (e.g. a labeled antibody or antigen) can be added simultaneously (1 step) or after a wash (2 steps).

According to another embodiment, the support and the reaction mixture (i.e. the cognate binding partners of the polypeptides immobilized on the support, fused to the analyte-capture entities) are previously incubated and then provided as a prefunctionalized support, to which the analytes or the sample containing said analytes (e.g. an antigen or an antibody) are added. The detection entity (e.g. a labeled antibody or antigen) can be added together with the analytes (1 step) or after a wash (2 steps).

The support and the reaction mixture according to the invention can also be used in lateral flow assays (LFA) or vertical flow assays (VFA) which principle is well known to the skilled person.

In that context, the support corresponds to a membrane or strip, which is functionalized with the at least two polypeptides, each polypeptide forming a test line.

Moreover, the reaction mixture of the invention containing the cognate binding partners of the polypeptides immobilized in the test lines fused to an analyte-capture entity, advantageously analyte-capture polypeptides, more advantageously antibodies, corresponds to the so-called capture antibodies.

Typically, the sample containing the analytes of interest, advantageously antigens, is deposit in the sample pad together with the capture antibodies and/or the detection antibodies. Alternatively, the capture antibodies and/or the detection antibodies can be deposit in the conjugate pad.

A control line containing a molecule able to bind the detection antibodies is advantageously present at the end of the strip to ensure that the reaction mixture together with the detection antibodies has properly migrated.

Therefore and according to another aspect, the invention concerns a method for detecting at least 2 analytes, possibly contained in a sample, comprising:
(a) contacting a support as disclosed above, a reaction mixture as disclosed above, the analytes or the sample containing said analytes, and the labeled detection entities as disclosed above; and
(b) testing the labeling, thereby detecting the presence of the analytes.

Basically, such a method encompasses 2 steps, i.e. the reaction step during which the binding of the analytes occurs and then a revelation step which allows to conclude about the analyte binding. A washing step is usually performed in between.

In practice, the first step can be performed in one step or sequentially. When performed sequentially and according to a preferred embodiment, the detection entities are added together with the analytes or the sample containing said analytes, or afterwards.

According to a specific embodiment, the support, the reaction mixture and the analytes or the sample(s) containing said analytes are all contacted simultaneously. According to another specific embodiment, the support and the reaction mixture are first contacted and then the analytes or the sample(s) containing said analytes are added. Alternatively, the reaction mixture and the analytes or the sample(s) containing said analytes are first contacted and then added to the support. Before the addition(s), a washing step can be performed.

According to a further aspect, the present invention concerns a kit for practicing the methods of the invention. At the minimum, such a kit contains a support and a reaction mixture as disclosed above.

The support and the reaction mixture can be provided separately or the support can be prefunctionalized, i.e. the polypeptides on the support are already bound to their cognate partners fused to the analyte-capture entities. Said components may be suitably labeled as known from the skilled person.

Said kit can also comprise controls, standards and/or calibrators. It can also comprise means for collecting the sample from the subject.

According to a further embodiment, a kit according to the invention further contains suitable detection entities, advantageously labeled detection entities e.g. labeled antibodies and/or antigens as defined above. Means for detecting and measuring the labels can also been provided.

According to another embodiment, said kit further comprises instructions for use.

### EXAMPLES

The invention is further described in detail by reference to the following experimental examples. These examples are provided for purposes of illustration only, and are not intended to be limiting unless otherwise specified.

Without further description, it is believed that one of ordinary skill in the art can, using the preceding description and the following illustrative examples, make and utilize the compounds of the present invention and practice the claimed methods. The following working examples therefore, specifically point out the preferred embodiments of the present invention, and are not to be construed as limiting in any way the remainder of the disclosure.

### BRIEF DESCRIPTION OF THE FIGURES

**Figure 1****: Results of the cross-reactivity tests in EIA, Enzyme Immuno-Assay (EIA) experiments with Colicins and Immunity proteins, 4 plex.**
   Colicins (E2, E7, E8 and E9) were coated on Maxisorb 96-well plates as indicated above each graph. Each well (light grey bars) was then incubated with a solution containing an Immunity protein fused to RLuc8 alone as indicated below each bar pair. In a separate well (dark grey bars), the same Immunity protein fused to RLuc8 (as indicated below each bar pair) was incubated with the same Colicin (as indicated above each graph) but in presence of the 3 non-cognate Immunity proteins devoid of Luciferase activity. All proteins were used at 100 nM. Binding affinity constants are indicated above each bar pair, as previously determined by kinetics (mole/L) (Li W. et al., 2004, J Mol Biol., 337(3):743-59.).

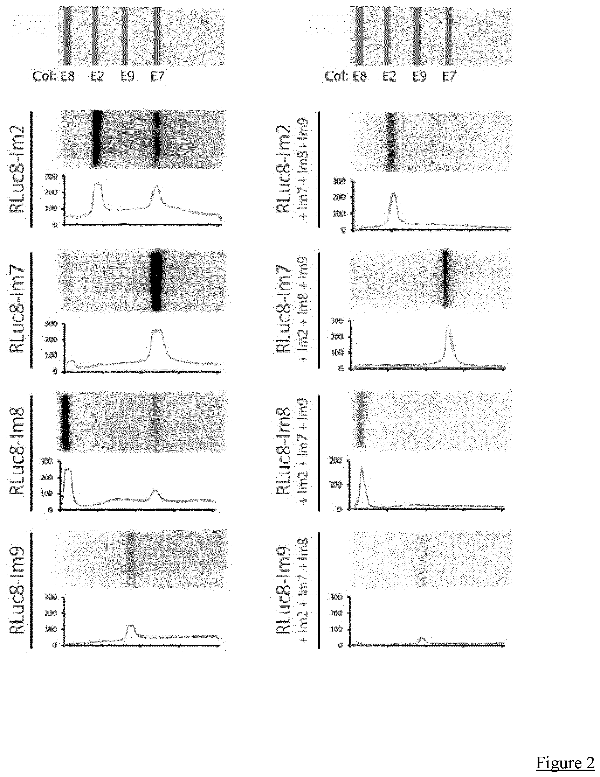

**Figure 2****: Results of the corresponding LFA test: Bioluminescence images (top) and corresponding profile plots (bottom).**
   Colicins E2, E7, E8 and E9 (10 µM) were sprayed on nitrocellulose membranes as shown on the top of each column. Each membrane was then incubated with a solution containing an Immunity protein fused to RLuc8 alone (100 nM) as indicated on the left of each graph (left column). On a separate membrane, the same Immunity protein (fused to RLuc8, 100 nM) was incubated with the same Colicins (10 µM) but in presence of the 3 non-cognate Immunity proteins (devoid of Luciferase activity, at 100 nM) (right column). Intensity plots are represented below each membrane.

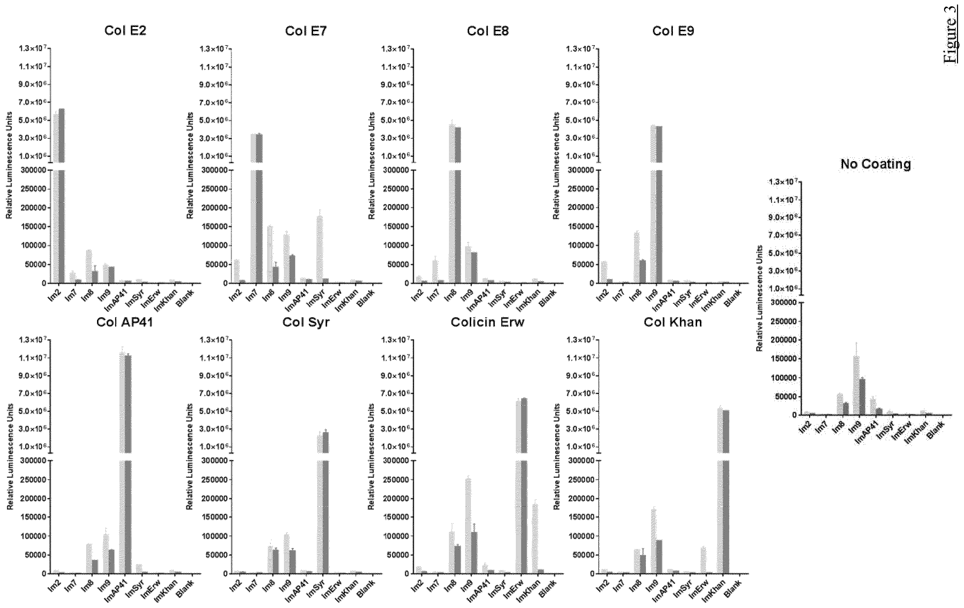

**Figure 3****: Results of the cross-reactivity tests in EIA, Enzyme Immuno-Assay (EIA) experiments with Colicins and Immunity proteins, 8 plex n°1.**
   Colicins ColE2, ColE7, ColE8, ColE9, ColAP41, ColSyr, ColErw, ColKhan were coated on 96-well plates as indicated above each graph. The "no coating" graph stands for "no Colicin was coated and only the coating buffer was used". Each well (light grey bars) was then incubated with a solution containing an Immunity protein fused to RLuc8 alone as indicated below each bar pair ("blank" condition stands for "no Immunity protein"). In a separate well (dark grey bars), the same Immunity protein fused to RLuc8 (as indicated below each bar pair) was incubated with the same Colicin (as indicated above each graph) but in presence of the 7 non-cognate Immunity proteins devoid of Luciferase activity. All proteins were used at 100 nM.

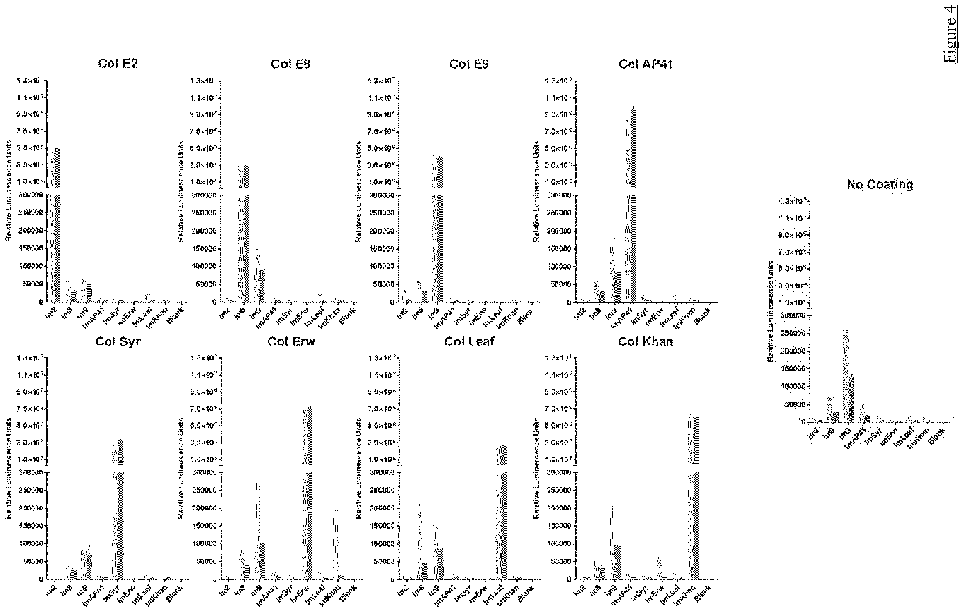

**Figure 4****: Results of the cross-reactivity tests in EIA, Enzyme Immuno-Assav (EIA) experiments with Colicins and Immunity proteins. 8 plex n°2.**
   Colicins ColE2, ColE8, ColE9, ColAP41, ColSyr, ColErw, ColLeaf, ColKhan were coated on 96-well plates as indicated above each graph. The "no coating" graph stands for no Colicin was coated and only the coating buffer was used. Each well (light grey bars) was then incubated with a solution containing an Immunity protein fused to RLuc8 alone as indicated below each bar pair ("blank" condition stands for "no Immunity protein"). In a separate well (dark grey bars), the same Immunity protein fused to RLuc8 (as indicated below each bar pair) was incubated with the same Colicin (as indicated above each graph) but in presence of the 7 non-cognate Immunity proteins devoid of Luciferase activity. All proteins were used at 100 nM.

### MATERIAL AND METHODS

### Plasmids and sequences

### Plasmids

pBA01 is a plasmid to express proteins in *E. coli.* The most important functional sequences comprised in the plasmid are:
- *LacI* - Lac repressor encoding site.
- T7 promoter - T7 polymerase binding domain
- *cer* - for plasmid stability
- *kanR* - sequence coding for Kanamycin resistance protein
- *colE1* - plasmid origin of replication
- *bom* - basis of mobility
- ROP protein coding sequence, which regulates the number of plasmid copies.

Between the T7 promoter and its T7 Terminator sequences, there is a multiple cloning site allowing to use a variety of restriction enzymes, such as BamHI, and XhoI used to clone different inserts into the plasmid.

### DNA sequences

The nucleotide sequences encoding some Colicins (Col) and the corresponding Immunity proteins (Im) have already been reported. As an example, the sequences for *E. coli* ColE2, E7, E8 and E9 are disclosed in WO2017/100584.

In order to prove the feasibility of the present invention using a large number of toxin/antitoxin couples (even not characterized or even not isolated yet), an *in silico* automated screening approach was applied in order to recover novel candidate bacteriocin sequences.

Profiles were built to capture the conserved residues of both bacteriocins and Immunity proteins, based on a multiple sequence alignment of seven bacteriocin sequences:
- ColE2 (VCW48573);
- ColE7 (WP_021530049);
- ColE8 (WP_012766032);
- ColE9 (WP_012644886);
- ColAP41 *Pseudomonas aeruginosa* AP41 (WP_134294768);
- ColErW *Pectobacterium carotovorum* (WP_039472082);
- ColSyr *Pseudomonas syringae* B728A (WP_057415187);
and a separate multiple sequence alignment of seven Immunity proteins:
- Im2 (AAA23069);
- Im7 (WP_001560791);
- Im8 (WP_000421100);
- Im9 (WP_012644887);
- ImAP41 *Pseudomonas aeruginosa* AP41 (WP_017002173);
- ImErw *Pectobacterium carotovorum* (WP_103165271);
- ImSyr *Pseudomonas syringae* B728A (WP_003403237).

Alignments were performed with Muscle and used to build two separate HMMER3 profiles. Both HMMER3 profiles were used to search for homologous bacteriocins and bacteriocin Immunity proteins against translated open reading frames of 72210 bacterial genomes from the NCBI RefSeq database (accessed on 29/03/2019). Candidate couples were kept if all the following criteria were met:
- both the bacteriocin and the bacteriocin Immunity protein were found within 500 nucleotides in the genome;
- the bacteriocin had a length of 300 to 1000 amino acids long, and contained the "HH-14X-N-8X-H-3X-H" motif (SEQ ID NO: 28).

Based on such a screening, 3935 putative "bacteriocin - bacteriocin Immunity protein" couples have been identified.

Among them, nine bacteriocin / Immunity protein listed in Table 1 below were selected for *in vitro* validation in multiplex (8 plex) experiments.

**Table 1: List of the NCBI protein accession numbers of the 9 "bacteriocin -bacteriocin Immunity protein" couples used in multiplex experiments.**

| Couple | Accession number bacteriocin | Accession number Immunity protein |
|---|---|---|
| Escherichia_coli_E2 (ColE2 or Im2) | VCW48573 | AAA23069 |
| Escherichia_coli_E7 (ColE7 or Im7) | WP_021530049 | WP_001560791 |
| Escherichia_coli_E8 (ColE8 or Im8) | WP_012766032 | WP_000421100 |
| Escherichia_coli_E9 (ColE9 or Im9) | WP_012644886 | WP_012644887 |
| Pseudomonas_aeruginosa_AP41 (ColAP41 or ImAP41) | WP_134294768 | WP_017002173 |
| Pectobacterium_carotovorum (ColErW or ImErw) | WP_039472082 | WP_103165271 |
| Pseudomonas_syringae_B728A (ColSyr or ImSyr) | WP_057415187 | WP_003403237 |
| Pseudomonas_sp_Leaf83 (ColLeaf or ImLeaf) | WP_055983760 | WP_055983763 |
| Photorhabdus_khanii (ColKhan or ImKhan) | WP_036849747 | WP_036849748 |

As a further illustration, additional couples, selected merely because they all originate from distinct bacteria species, are shown in Table 2 below:

**Table 2: List of the NCBI genome accession numbers and relative coordinates in the genomes of 133 putative "bacteriocin -bacteriocin Immunity protein" couples.**

| Species | Accession_genome | Position_bacteriocin | Position_Immunity |
|---|---|---|---|
| Aliivibrio_logei | NZ_MAJU01000029 | 97489-99528 | 99519-99782 |
| Citrobacter_europaeus | NZ_PQSZ01000001 | 440066-438300 | 438300-438010 |
| Citrobacter_freundii | NZ_CP024679 | 844619-842847 | 842869-842576 |
| Citrobacter_koseri | NZ_CP026697 | 3966242-3967987 | 3967987-3968277 |
| Citrobacter_portucalensis | NZ_PJEP01000009 | 183525-181753 | 181775-181482 |
| Enterobacter_asburiae | NZ_JWBX01000050 | 3771-6071 | 6034-6330 |
| Enterobacter_bugandensis | NZ_LT992502 | 599024-597366 | 597403-597116 |
| Enterobacter_cancerogenus | NZ_CP025225 | 4093243-4094901 | 4094864-4095151 |
| Enterobacter_cloacae | NZ_FJZR01000001 | 163227-161569 | 161606-161319 |
| Enterobacter_hormaechei | NZ_RHUO01000014 | 120989-122647 | 122610-122897 |
| Enterobacter_kobei | NZ_NEES01000038 | 98261-99919 | 99885-100169 |
| Enterobacter_roggenkampii | NZ_CP033802 | 973-3273 | 3236-3532 |
| Enterovibrio_norvegicus | NZ_MCYQ01000031 | 15865-13430 | 13426-13172 |
| Erwinia_tasmaniensis | NC_010693 | 3117-5048 | 5011-5307 |
| Escherichia_coli | NZ_KZ269654 | 130769-132553 | 132538-132840 |
| Escherichia_marmotae | NZ_QON001000171 | 3-1700 | 1666-1965 |
| Halomonas_anticariensis | NZ_KE332388 | 1023731-1022658 | 1022695-1022333 |
| Klebsiella_aerogenes | NZ_FKAH01000009 | 30758-32416 | 32379-32666 |
| Klebsiella_oxytoca | NZ_CP026716 | 59426-58119 | 58153-57860 |
| Klebsiella_pneumoniae | NZ_MSLK01000030 | 37999-40404 | 40370-40684 |
| Klebsiella_quasipneumoniae | NZ_CP012300 | 2868460-2866370 | 2866370-2866110 |
| Klebsiella_variicola | NZ_JVDR01000265 | 2680-257 | 291-1 |
| Kluyvera_intermedia | NZ_LR134138 | 932802-931042 | 931085-930756 |
| Kosakonia_oryziphila | NZ_FMBC01000002 | 58343-60001 | 59985-60260 |
| Lelliottia_amnigena | NZ_CP015774 | 585864-584212 | 584233-583967 |
| Morganella_morganii | NZ_CP028957 | 5884-4799 | 4799-4536 |
| Obesumbacterium_proteus | NZ_CP014608 | 124224-126662 | 126666-126917 |
| Pectobacterium_carotovorum | NZ_FQWI01000003 | 68421-66571 | 66599-66276 |
| Pectobacterium_parmentieri | NZ_CP015749 | 2249728-2247821 | 2247860-2247558 |
| Pectobacterium_polaris | NZ_CP017481 | 1587590-1585659 | 1585692-1585396 |
| Pectobacterium_wasabiae | NZ_CP015750 | 5952-4033 | 4072-3770 |
| Photorhabdus_bodei | NZ_NSCM01000010 | 57799-56114 | 56174-55860 |
| Photorhabdus_khanii | NZ_AYSJ01000015 | 196941-198614 | 198614-198883 |
| Photorhabdus_laumondii | NZ_CP024901 | 2256107-2254422 | 2254482-2254168 |
| Photorhabdus_luminescens | NZ_JQOC01000002 | 350148-349249 | 349309-348995 |
| Pluralibacter_gergoviae | NZ_LDZL01000001 | 384414-382663 | 382700-382404 |
| Pragia_fontium | NZ_LR134531 | 3278098-3279660 | 3279660-3279923 |
| Proteus_mirabilis | NZ_CP015347 | 1204401-1206560 | 1206545-1206820 |
| Providencia_heimbachae | NZ_LS483422 | 2015035-2016924 | 2016924-2017184 |
| Providencia_rettgeri | NZ_CP029736 | 4227492-4225660 | 4225660-4225397 |
| Pseudocitrobacter_faecalis | NZ_QNRL01000002 | 363033-364886 | 364886-365161 |
| Pseudomonas_aeruginosa | NZ_JTXI01000011 | 49766-52135 | 52129-52410 |
| Pseudomonas_alkylphenolica | NZ_QJRG01000044 | 135848-133548 | 133605-133288 |
| Pseudomonas_amygdali | NZ_LJQN01000063 | 16580-14601 | 14613-14332 |
| Pseudomonas_arsenicoxydans | NZ_LT629705 | 4889248-4886537 | 4886586-4886272 |
| Pseudomonas_asplenii | NZ_LT629777 | 6235463-6236842 | 6236842-6237093 |
| Pseudomonas_avellanae | NZ_CP026562 | 5759816-5761771 | 5761771-5762037 |
| Pseudomonas_azotoformans | NZ_MZZJ01000003 | 411928-414207 | 414167-414463 |
| Pseudomonas_brassicacearum | NZ_MOBD01000001 | 1975927-1978125 | 1978071-1978370 |
| Pseudomonas_cannabina | NZ_RBPH01000272 | 83147-81114 | 81160-80852 |
| Pseudomonas_caricapapayae | NZ_RBVC01000083 | 67923-69953 | 69872-70219 |
| Pseudomonas_cedrina | NZ_PCQE01000020 | 55716-53440 | 53506-53186 |
| Pseudomonas_cerasi | NZ_LT963397 | 105958-103886 | 103929-103609 |
| Pseudomonas_chlororaphis | NZ_LR134334 | 6808804-6807254 | 6807250-6806999 |
| Pseudomonas_cichorii | NZ_QPDT01000003 | 84964-86898 | 86880-87170 |
| Pseudomonas_citronellolis | NZ_LKKN01000022 | 92207-93529 | 93533-93784 |
| Pseudomonas_congelans | NZ_LJQB01000083 | 15910-13985 | 13997-13716 |
| Pseudomonas_coronafaciens | NZ_RBUX01000220 | 5942-4053 | 4096-3776 |
| Pseudomonas_cremoricolorata | NZ_AUEA01000015 | 48681-46741 | 46790-46425 |
| Pseudomonas_denitrificans | NZ_JWBF01000085 | 9256-6887 | 6893-6612 |
| Pseudomonas_entomophila | NZ_CP034337 | 4480018-4478258 | 4478282-4477989 |
| Pseudomonas_extremaustralis | NZ_FUYI01000055 | 12481-11180 | 11229-10918 |
| Pseudomonas_floridensis | NZ_MUI001000016 | 39978-39022 | 39199-38747 |
| Pseudomonas_fluorescens | NZ_MOBS01000003 | 146610-148553 | 148510-148803 |
| Pseudomonas_fragi | NZ_NQKO01000004 | 51155-52069 | 52060-52332 |
| Pseudomonas_frederiksbergensis | NZ_MOBQ01000007 | 169294-166709 | 166715-166428 |
| Pseudomonas_fulva | NZ_QJRV01000012 | 127779-125068 | 125092-124808 |
| Pseudomonas_furukawaii | NZ_AP014862 | 1305011-1306675 | 1306679-1306948 |
| Pseudomonas_guariconensis | NZ_PJCQ01000007 | 144669-142675 | 142732-142415 |
| Pseudomonas_indica | NZ_FNFD01000011 | 166565-167962 | 167950-168552 |
| Pseudomonas_japonica | NZ_FZOL01000004 | 145806-143581 | 143662-143306 |
| Pseudomonas_jessenii | NZ_QJRT01000027 | 36739-38259 | 38214-38537 |
| Pseudomonas_knackmussii | NZ_HG322950 | 1180118-1181608 | 1181562-1181891 |
| Pseudomonas_koreensis | NZ_CP027480 | 252327-250873 | 250900-250625 |
| Pseudomonas_kribbensis | NZ_CP029608 | 1985670-1987589 | 1987534-1987830 |
| Pseudomonas_lundensis | NZ_NQKJ01000079 | 3273-4679 | 4679-4930 |
| Pseudomonas_mandelii | NZ_KB906325 | 1131640-1129004 | 1129053-1128739 |
| Pseudomonas_marginalis | NZ_LKGY01000053 | 35114-36436 | 36427-36699 |
| Pseudomonas_monteilii | NC_023075 | 5293278-5295494 | 5295440-5295766 |
| Pseudomonas_moraviensis | NZ_LT629788 | 2948147-2946240 | 2946291-2945986 |
| Pseudomonas_mosselii | NZ_JHYW01000003 | 368183-370423 | 370420-370695 |
| Pseudomonas_mucidolens | NZ_LT629802 | 4738936-4737593 | 4737620-4737345 |
| Pseudomonas_orientalis | NZ_SGFE01000009 | 133864-131522 | 131562-131266 |
| Pseudomonas_parafulva | NZ_CP019952 | 3668857-3666917 | 3666966-3666658 |
| Pseudomonas_plecoglossicida | NZ_PJCM01000002 | 311156-313486 | 313345-313746 |
| Pseudomonas_poae | NZ_MOAY01000019 | 593641-591341 | 591381-591085 |
| Pseudomonas_prosekii | NZ_LT629762 | 4143268-4144623 | 4144586-4144885 |
| Pseudomonas_psychrophila | NZ_LT629795 | 1488178-1490163 | 1490126-1490422 |
| Pseudomonas_putida | NZ_LDJF01000008 | 58965-56653 | 56707-56393 |
| Pseudomonas_reinekei | NZ_LT629709 | 3037984-3036791 | 3036797-3036534 |
| Pseudomonas_savastanoi | NZ_RBU001000308 | 87559-89538 | 89526-89807 |
| Pseudomonas_silesiensis | NZ_CP014870 | 3388561-3385919 | 3385968-3385654 |
| Pseudomonas_synxantha | NZ_MSDH01000008 | 112199-109920 | 109960-109664 |
| Pseudomonas_syringae | NZ_CP024646 | 6040664-6038262 | 6038302-6038003 |
| Pseudomonas_thivervalensis | NZ_LT629691 | 6114207-6116864 | 6116840-6117136 |
| Pseudomonas_umsongensis | NZ_KK211098 | 159766-162393 | 162381-162668 |
| Pseudomonas_viridiflava | NZ_RBTP01000069 | 75581-77533 | 77512-77808 |
| Rahnella_aquatilis | NZ_JUHL01000013 | 83031-81625 | 81621-81370 |
| Salmonella_bongori | NZ_CP006692 | 155727-157544 | 157529-157837 |
| Salmonella_enterica | NZ_LS483428 | 3814826-3813015 | 3813018-3812719 |
| Serratia_liquefaciens | NZ_MQMW01000001 | 1085598-1083985 | 1084028-1083729 |
| Serratia_marcescens | NZ_CP018930 | 651822-653930 | 653930-654193 |
| Serratia_plymuthica | NZ_LR134151 | 516118-513902 | 513936-513643 |
| Serratia_rubidaea | NZ_LR134493 | 3408154-3409866 | 3409820-3410125 |
| Shigella_boydii | NZ_MSJS02000064 | 6230-4707 | 4707-4444 |
| Shigella_sonnei | NZ_UDZS01000182 | 2475-4238 | 4238-4501 |
| Vibrio_alginolyticus | NZ_CP013487 | 153989-152676 | 152685-152422 |
| Vibrio_anguillarum | NZ_CP023310 | 3110346-3112865 | 3112846-3113115 |
| Vibrio_bivalvicida | NZ_LLEI02000053 | 2-2383 | 2367-2663 |
| Vibrio_campbellii | NZ_CP025953 | 2028610-2031222 | 2031213-2031476 |
| Vibrio_cholerae | NZ_NMTN01000030 | 9295-6791 | 6807-6508 |
| Vibrio_gazogenes | NZ_CP018835 | 451698-450124 | 450124-449867 |
| Vibrio_harveyi | NZ_CP025537 | 1236450-1233943 | 1233946-1233686 |
| Vibrio_hyugaensis | NZ_BBLD01000064 | 24167-21537 | 21546-21283 |
| Vibrio_jasicida | NZ_PKNL01000090 | 30389-27768 | 27780-27511 |
| Vibrio_ordalii | NZ_AJYV02000046 | 57185-59701 | 59691-59942 |
| Vibrio_parahaemolyticus | NZ_LFUM01000020 | 85568-82938 | 82990-82652 |
| Vibrio_rhizosphaerae | NZ_JONG01000020 | 14180-15781 | 15785-16039 |
| Vibrio_tasmaniensis | NZ_AJZM02000361 | 2591-5098 | 5095-5355 |
| Vibrio_xiamenensis | NZ_FNDD01000016 | 52209-49693 | 49736-49440 |
| Xenorhabdus_griffiniae | NZ_LDNM01000005 | 17351-15990 | 16005-15727 |
| Xenorhabdus_hominickii | NZ_NJAI01000009 | 40650-38698 | 38684-38430 |
| Yersinia_aldovae | NZ_CQAX01000017 | 76108-73244 | 73287-72988 |
| Yersinia_aleksiciae | NZ_CGBL01000013 | 135186-132484 | 132487-132233 |
| Yersinia_bercovieri | NZ_CQBU01000024 | 21553-24288 | 24267-24545 |
| Yersinia_enterocolitica | NZ_CTJG01000026 | 47217-44497 | 44497-44237 |
| Yersinia_frederiksenii | NZ_CQEC01000014 | 125730-123019 | 123040-122762 |
| Yersinia_intermedia | NZ_NHOH01000020 | 134816-132165 | 132166-131909 |
| Yersinia_kristensenii | NZ_CQDL01000042 | 17278-14537 | 14559-14281 |
| Yersinia_mollaretii | NZ_CQDS01000012 | 8531-11071 | 11070-11327 |
| Yersinia_pekkanenii | NZ_CWJL01000010 | 133463-131838 | 131881-131579 |
| Yersinia_pseudotuberculosis | NZ_CIFL01000026 | 21042-22937 | 22936-23193 |
| Yersinia_similis | NZ_CQBK01000011 | 121244-118638 | 118639-118382 |
| Yersinia_wautersii | NZ_CVMG01000008 | 21823-24435 | 24434-24691 |

### Protein sequences

The sequences corresponding to the Colicins and Immunity proteins of Table 1 encoded by the plasmids are listed below. All fusion proteins harbor a 6His tag at their COOH-terminus. All colicins fusion proteins contained a NH2-terminal Flag tag (underlined) optionally following by a fluorescent protein (in italic) fused to the Colicin NH2-terminus (in bold). ColE2 and ColE7 are in frame with EmGFP while all other Colicins (ColE8, ColE9, ColAP41, ColSyr, ColErw, ColLeaf, ColKhan) are in frame with mCherry. All immunity proteins (in bold) are in frame at their NH2-terminus with a polypeptide comprising a Myc tag or Avitag (underlined). Half of Im proteins are fused to RLuc8 (underlined and italic).
- SEQ ID NO: 1 corresponds to the so-called Flag-EmGFP-ColE2-6His: 
MGGDYKDDDDKGGSVSKGEELFTGVVPILVELDGDVNGHKFSVSGEGEGDATY
GKLTLKFICTTGKLPVPWPTLVTTLTYGVQCFARYPDHMKQHDFFKSAMPEGYVQ
ERTIFFKDDGNYKTRAEVKFEGDTLVNRIELKGIDFKEDGNILGHKLEYNYNSHKV
YITADKQKNGIKVNFKTRHNIEDGSVQLADHYQQNTPIGDGPVLLPDNHYLSTQSA
LSKDPNEKRDHMVLLEFVTAAGITLGMDELYKLESKRNKPGKATGKGKPVGD
KWLDDAGKDSGAPIPDRIADKLRDKEFKNFDDFRKKFWEEVSKDPDLSK
QFKGSNKTNIQKGKAPFARKKDQVGGRERFELHADKPISQDGGVYDMN
NIRVTTPKRHIDIHRGKLEGGGSHHHHHH
- SEQ ID NO: 2 corresponds to the so-called Flag-EmGFP-ColE7-6His: 
MGGDYKDDDDKGGSVSKGEELFTGVVPILVELDGDVNGHKFSVSGEGEGDATYG
KLTLKFICTTGKLPVPWPTLVTTLTYGVQCFARYPDHMKQHDFFKSAMPEGYVQER
TIFFKDDGNYKTRAEVKFEGDTLVNRIELKGIDFKEDGNILGHKLEYNYNSHKVYITA
DKQKNGIKVNFKTRHNIEDGSVQLADHYQQNTPIGDGPVLLPDNHYLSTQSALSKDP
NEKRDHMVLLEFVTAAGITLGMDELYKLESKRNKPGKATGKGKPVNNKWLNN
AGKDLGSPVPDRIANKLRDKEFKSFDDFRKKFWEEVSKDPELSKQFSRNNN
DRMKVGKAPKTRTQDVSGKRTSFELHAEKPISQNGGVYDMDNISVVTPKR
HIDIHRGKLEGGGSHHHHHH
- SEQ ID NO: 3 corresponds to the so-called Flag-mCherry-ColE8-6His: 
MGGDYKDDDDKGGSVSKGEEDNMAIIKEFMRFKVHMEGSVNGHEFEIEGEGEGR
PYEGTQTAKLKVTKGGPLPFAWDILSPQFMYGSKAYVKHPADIPDYLKLSFPEGFKW
ERVMNFEDGGVVTVTQDSSLQDGEFIYKVKLRGTNFPSDGPVMQKKTMGWEASSE
RMYPEDGALKGEIKQRLKLKDGGHYDAEVKTTYKAKKPVQLPGAYNVNIKLDITSHN
EDYTIVEQYERAEGRHSTGGMDELYKLESKRNKPGKATGKGKPVGDKWLDDA
GKDSGAPIPDRIADKLRDKEFKNFDDFRRKFWEEVSKDPELSKQFNPGNKK
RLSQGLAPRARNKDTVGGRRSFELHADKPISQDGGVYDMDNLRITTPKRHI
DIHRGQLEGGGSHHHHHH
- SEQ ID NO: 4 corresponds to the so-called Flag-mCherry-ColE9-6His: 
MGGDYKDDDDKGGSVSKGEEDNMAIIKEFMRFKVHMEGSVNGHEFEIEGEGEGR
PYEGTQTAKLKVTKGGPLPFAWDILSPQFMYGSKAYVKHPADIPDYLKLSFPEGFKW
 
ERVMNFEDGGVVTVTQDSSLQDGEFIYKVKLRGTNFPSDGPVMQKKTMGWEASSE
RMYPEDGALKGEIKQRLKLKDGGHYDAEVKTTYKAKKPVQLPGAYNVNIKLDITSHN
EDYTIVEQYERAEGRHSTGGMDELYKLESKRNKPGKATGKGKPVGDKWLDDA
GKDSGAPIPDRIADKLRDKEFKSFDDFRKAVWEEVSKDPELSKNLNPSNKSS
VSKGYSPFTPKNQQVGGRKVYELHADKPISQGGEVYDMDNIRVTTPKRHID
IHRGKLEGGGSHHHHHH
- SEQ ID NO: 5 corresponds to the so-called Myc-RLuc8-Im2-6His: 
MGGEQKLISEEDLGGSASKVYDPEQRKRMITGPQWWARCKQMNVLDSFINYYDSE
KHAENAVIFLHGNATSSYLWRHVVPHIEPVARCIIPDLIGMGKSGKSGNGSYRLLDHY
KYLTAWFELLNLPKKIIFVGHDWGAALAFHYAYEHQDRIKAIVHMESVVDVIESWDE
WPDIEEDIALIKSEEGEKMVLENNFFVETVLPSKIMRKLEPEEFAAYLEPFKEKGEVR
RPTLSWPREIPLVKGGKPDVVQIVRNYNAYLRASDDLPKLFIESDPGFFSNAIVEGAK
KFPNTEFVKVKGLHFLQEDAPDEMGKYIKSFVERVLKNEQKLELKHSISDYTEAE
FLEFVKKICRAEGATEEDDNKLVREFERLTEHPDGSDLIYYPRDDREDSPEG
IVKEIKEWRAANGKSGFKQGLEGGGSHHHHHH
- SEQ ID NO: 6 corresponds to the so-called Myc-RLuc8-Im7-6His: 
MGGEQKLISEEDLGGSASKVYDPEQRKRMITGPQWWARCKQMNVLDSFINYYDSE
KHAENAVIFLHGNATSSYLWRHVVPHIEPVARCIIPDLIGMGKSGKSGNGSYRLLDHY
KYLTAWFELLNLPKKIIFVGHDWGAALAFHYAYEHQDRIKAIVHMESVVDVIESWDE
WPDIEEDIALIKSEEGEKMVLENNFFVETVLPSKIMRKLEPEEFAAYLEPFKEKGEVR
RPTLSWPREIPLVKGGKPDVVQIVRNYNAYLRASDDLPKLFIESDPGFFSNAIVEGAK
KFPNTEFVKVKGLHFLQEDAPDEMGKYIKSFVERVLKNEQKLELKNSISDYTEAE
FVQLLKEIEKENVAATDDVLDVLLEHFVKITEHPDGTDLIYYPSDNRDDSPE
GIVKEIKEWRAANGKPGFKQGLEGGGSHHHHHH
- SEQ ID NO: 7 corresponds to the so-called Myc-RLuc8-Im8-6His: 
MGGEQKLISEEDLGGSASKVYDPEQRKRMITGPQWWARCKQMNVLDSFINYYDSE
KHAENAVIFLHGNATSSYLWRHVVPHIEPVARCIIPDLIGMGKSGKSGNGSYRLLDHY
KYLTAWFELLNLPKKIIFVGHDWGAALAFHYAYEHQDRIKAIVHMESVVDVIESWDE
WPDIEEDIALIKSEEGEKMVLENNFFVETVLPSKIMRKLEPEEFAAYLEPFKEKGEVR
RPTLSWPREIPLVKGGKPDVVQIVRNYNAYLRASDDLPKLFIESDPGFFSNAIVEGAK
KFPNTEFVKVKGLHFLQEDAPDEMGKYIKSFVERVLKNEQKLELKNSISDYTETEF
KKIIEDIINCEGDEKKQDDNLEHFISVTEHPSGSDLIYYPEGNNDGSPEAVIKE
IKEWRAANGKSGFKQGLEGGGSHHHHHH
- SEQ ID NO: 8 corresponds to the so-called Myc-RLuc8-Im9-6His: 
MGGEQKLISEEDLGGSASKVYDPEQRKRMITGPQWWARCKQMNVLDSFINYYDSE
KHAENAVIFLHGNATSSYLWRHVVPHIEPVARCIIPDLIGMGKSGKSGNGSYRLLDHY
KYLTAWFELLNLPKKIIFVGHDWGAALAFHYAYEHQDRIKAIVHMESVVDVIESWDE
WPDIEEDIALIKSEEGEKMVLENNFFVETVLPSKIMRKLEPEEFAAYLEPFKEKGEVR
RPTLSWPREIPLVKGGKPDVVQIVRNYNAYLRASDDLPKLFIESDPGFFSNAIVEGAK
KFPNTEFVKVKGLHFLQEDAPDEMGKYIKSFVERVLKNEQKLELKHSISDYTEAE
FLQLVTTICNADTSSEEELVKLVTHFEEMTEHPSGSDLIYYPKEGDDDSPSGI
VNTVKQWRAANGKLEGGGSHHHHHH
- SEQ ID NO: 9 corresponds to the so-called Flag-mCherry-ColAP41-Cys-6His: 
MGGDYKDDDDKGGSVSKGEEDNMAIIKEFMRFKVHMEGSVNGHEFEIEGEGEGR
PYEGTQTAKLKVTKGGPLPFAWDILSPQFMYGSKAYVKHPADIPDYLKLSFPEGFKW
ERVMNFEDGGVVTVTQDSSLQDGEFIYKVKLRGTNFPSDGPVMQKKTMGWEASSE
RMYPEDGALKGEIKQRLKLKDGGHYDAEVKTTYKAKKPVQLPGAYNVNIKLDITSHN
EDYTIVEQYERAEGRHSTGGMDELYKLPGGSDEPGVATGNGQPVTGNWLAGAS
QGDGVPIPSQIADQLRGKEFKSWRDFREQFWMAVSKDPSALENLSPSNRYF
VSQGLAPYAVPEEHLGSKEKFEIHAVVPLESGGALYNIDNLVIVTPKRHSEI
HKELKLKRKEKGGSGGSCLEGGGSHHHHHH
- SEQ ID NO: 10 corresponds to the so-called Flag-mCherry-ColSyr-Cys-6His 
MGGDYKDDDDKGGSVSKGEEDNMAIIKEFMRFKVHMEGSVNGHEFEIEGEGEGR
PYEGTQTAKLKVTKGGPLPFAWDILSPQFMYGSKAYVKHPADIPDYLKLSFPEGFKW
ERVMNFEDGGVVTVTQDSSLQDGEFIYKVKLRGTNFPSDGPVMQKKTMGWEASSE
RMYPEDGALKGEIKQRLKLKDGGHYDAEVKTTYKAKKPVQLPGAYNVNIKLDITSHN
EDYTIVEQYERAEGRHSTGGMDELYKLPGGSRSIPGVASGYGEAVNGVWLGDK
TRAEGASIPAHIADQLRGRRFGNFDSLRKATWIAVANDPELVKQFTQHNLE
IMRDGGAPYPRLVDQAGGRTKFEIHAKKHIANGGAVYDIDNLVIMTPRQHI
DHHRSHENDLGGSGGSCLEGGGSHHHHHH
- SEQ ID NO: 11 corresponds to the so-called Flag-mCherry-ColErw-Cys-6His: 
MGGDYKDDDDKGGSVSKGEEDNMAIIKEFMRFKVHMEGSVNGHEFEIEGEGEGR
PYEGTQTAKLKVTKGGPLPFAWDILSPQFMYGSKAYVKHPADIPDYLKLSFPEGFKW
ERVMNFEDGGVVTVTQDSSLQDGEFIYKVKLRGTNFPSDGPVMQKKTMGWEASSE
RMYPEDGALKGEIKQRLKLKDGGHYDAEVKTTYKAKKPVQLPGAYNVNIKLDITSHN
EDYTIVEQYERAEGRHSTGGMDELYKLPGGSRDKPGTVTGKGEVLSSEGKWLE
SASSGLGAPVPAQVADKLRGQKFERFDDFREAFWLAVAECPELMVQFNRS
 
NQTIIRAGTSPFAIPEEQVGKRKRFEIHAVKNIQHRGEVYNIDNLRVNTPKN
HIGLHGGSGGSCLEGGGSHHHHHH
- SEQ ID NO: 12 corresponds to the so-called Flag-mCherry-ColLeaf-Cys-6His: 
MGGDYKDDDDKGGSVSKGEEDNMAIIKEFMRFKVHMEGSVNGHEFEIEGEGEGR
PYEGTQTAKLKVTKGGPLPFAWDILSPQFMYGSKAYVKHPADIPDYLKLSFPEGFKW
ERVMNFEDGGVVTVTQDSSLQDGEFIYKVKLRGTNFPSDGPVMQKKTMGWEASSE
RMYPEDGALKGEIKQRLKLKDGGHYDAEVKTTYKAKKPVQLPGAYNVNIKLDITSHN
EDYTIVEQYERAEGRHSTGGMDELYKLGGGGSGGGGSSLRHEPGVVTGQGQDV
TGIWLENAGRELGAPIPSQIADQLRGKQFSSFDSFRKRFWKTVGTDATLSN
QFISANRKRMLAGKAAKLREKDRVGGRTTYELHAVEKISEGGEVYNVDNL
RVVTAKRHIEIHKTEGKCLEGGGSHHHHHH
- SEQ ID NO: 13 corresponds to the so-called Flag-mCherry-ColKhan-Cys-6His: 
MGGDYKDDDDKGGSVSKGEEDNMAIIKEFMRFKVHMEGSVNGHEFEIEGEGEGR
PYEGTQTAKLKVTKGGPLPFAWDILSPQFMYGSKAYVKHPADIPDYLKLSFPEGFKW
ERVMNFEDGGVVTVTQDSSLQDGEFIYKVKLRGTNFPSDGPVMQKKTMGWEASSE
RMYPEDGALKGEIKQRLKLKDGGHYDAEVKTTYKAKKPVQLPGAYNVNIKLDITSHN
EDYTIVEQYERAEGRHSTGGMDELYKLGGGGSGGGGSNTPRNQPGTVTGQGQK
VEGNWLSRAGQDMGAPIPSQIADKLRGRTFNNFDDFRKAFWKEVGNDPEL
AKDLSDVNKKRIKELGYAPFAIPIEQVGGKKKFDIHAVKPIKDGGAVYDLD
NLRVVTPKKHIELHSNCLEGGGSHHHHHH
- SEQ ID NO: 14 corresponds to the so-called Myc-RLuc8-ImAP41-Avitag-6His: 
MGGEQKLISEEDLGGSASKVYDPEQRKRMITGPQWWARCKQMNVLDSFINYYDSE
KHAENAVIFLHGNATSSYLWRHVVPHIEPVARCIIPDLIGMGKSGKSGNGSYRLLDHY
KYLTAWFELLNLPKKIIFVGHDWGAALAFHYAYEHQDRIKAIVHMESVVDVIESWDE
WPDIEEDIALIKSEEGEKMVLENNFFVETVLPSKIMRKLEPEEFAAYLEPFKEKGEVR
RPTLSWPREIPLVKGGKPDVVQIVRNYNAYLRASDDLPKLFIESDPGFFSNAIVEGAK
KFPNTEFVKVKGLHFLQEDAPDEMGKYIKSFVERVLKNEQKLPGGSDIKNNLSDY
TESEFLEIIEEFFKNKSGLKGSELEKRMDKLVKHFEEVTSHPRKSGVIFHPK
PGFETPEGIVKEVKEWRAANGLPGFKAGLEGGSGGSGLNDIFEAQKIEWHEL
EGGGSHHHHHH
- SEQ ID NO: 15 corresponds to the so-called Myc-RLuc8-ImSyr-Avitag-6His: 
MGGEQKLISEEDLGGSASKVYDPEQRKRMITGPQWWARCKQMNVLDSFINYYDSE
KHAENAVIFLHGNATSSYLWRHVVPHIEPVARCIIPDLIGMGKSGKSGNGSYRLLDHY
 
KYLTAWFELLNLPKKIIFVGHDWGAALAFHYAYEHQDRIKAIVHMESVVDVIESWDE
WPDIEEDIALIKSEEGEKMVLENNFFVETVLPSKIMRKLEPEEFAAYLEPFKEKGEVR
RPTLSWPREIPLVKGGKPDVVQIVRNYNAYLRASDDLPKLFIESDPGFFSNAIVEGAK
KFPNTEFVKVKGLHFLQEDAPDEMGKYIKSFVERVLKNEQKLPGGSIFKEKIEDYT
EEEFLEFLKGLSSEYSQLHGDEFIKHMDRSVEHFVKITEHPAQTDVIFYPEE
GQEDTPEGILKVIKEWRAKNGKPGFKSGGSGGSGLNDIFEAQKIEWHELEGG
GSHHHHHH
- SEQ ID NO: 16 corresponds to the so-called Myc-RLuc8-ImErw-Avitag-6His: 
MGGEQKLISEEDLGGSASKVYDPEQRKRMITGPQWWARCKQMNVLDSFINYYDSE
KHAENAVIFLHGNATSSYLWRHVVPHIEPVARCIIPDLIGMGKSGKSGNGSYRLLDHY
KYLTAWFELLNLPKKIIFVGHDWGAALAFHYAYEHQDRIKAIVHMESVVDVIESWDE
WPDIEEDIALIKSEEGEKMVLENNFFVETVLPSKIMRKLEPEEFAAYLEPFKEKGEVR
RPTLSWPREIPLVKGGKPDVVQIVRNYNAYLRASDDLPKLFIESDPGFFSNAIVEGAK
KFPNTEFVKVKGLHFLQEDAPDEMGKYIKSFVERVLKNEQKLPGGSNLKEKLEDY
TEAEFISYLKEFFDNPMGLRGKELETHLDSLVEHFDKIVFHPEGNGLIFYPP
DERDDSPEGVLNEIKRWRKSQGLPLFKDSKGGSGGSGLNDIFEAQKIEWHEL
EGGGSHHHHHH
- SEQ ID NO: 17 corresponds to the so-called Avitag-RLuc8-ImLeaf-6His 
MGLNDIFEAQKIEWHEGGSGGSGGSASKVYDPEQRKRMITGPQWWARCKQMNVL
DSFINYYDSEKHAENAVIFLHGNATSSYLWRHVVPHIEPVARCIIPDLIGMGKSGKSGN
GSYRLLDHYKYLTAWFELLNLPKKIIFVGHDWGAALAFHYAYEHQDRIKAIVHMESVV
DVIESWDEWPDIEEDIALIKSEEGEKMVLENNFFVETVLPSKIMRKLEPEEFAAYLEP
FKEKGEVRRPTLSWPREIPLVKGGKPDVVQIVRNYNAYLRASDDLPKLFIESDPGFFS
NAIVEGAKKFPNTEFVKVKGLHFLQEDAPDEMGKYIKSFVERVLKNEQGGGSTAGG
GGSKRQFADYTEAEFIAFMEDIFRENEAETDDRLDVLLDQFREITGHPDGTD
LIYYCESDAECTPERITQKVKSWRAANGLPGFKSTLEGGGSHHHHHH
- SEQ ID NO: 18 corresponds to the so-called Avitag-RLuc8-ImKhan-6His: 
MGLNDIFEAQKIEWHEGGSGGSGGSASKVYDPEQRKRMITGPQWWARCKQMNVL
DSFINYYDSEKHAENAVIFLHGNATSSYLWRHVVPHIEPVARCIIPDLIGMGKSGKSGN
GSYRLLDHYKYLTAWFELLNLPKKIIFVGHDWGAALAFHYAYEHQDRIKAIVHMESVV
DVIESWDEWPDIEEDIALIKSEEGEKMVLENNFFVETVLPSKIMRKLEPEEFAAYLEP
FKEKGEVRRPTLSWPREIPLVKGGKPDVVQIVRNYNAYLRASDDLPKLFIESDPGFFS
NAIVEGAKKFPNTEFVKVKGLHFLQEDAPDEMGKYIKSFVERVLKNEQGGGSTAGG
 
GGSELKNKLEDYTEAEFLSLLNKIWAVDVSEEEHDNLIDHFEKLSEHPNGN
GLIFYPENGVEDSPEGVLKVIKEWRAKNGKPGFKKLEGGGSHHHHHH
- SEQ ID NO: 19 corresponds to the so-called Myc-Im2-6His: 
MGGEQKLISEEDLGGSELKHSISDYTEAEFLEFVKKICRAEGATEEDDNKLV
REFERLTEHPDGSDLIYYPRDDREDSPEGIVKEIKEWRAANGKSGFKQGLE
GGGSHHHHHH
- SEQ ID NO: 20 corresponds to the so-called Myc-Im7-6His: 
MGGEQKLISEEDLGGSELKNSISDYTEAEFVQLLKEIEKENVAATDDVLDVLL
EHFVKITEHPDGTDLIYYPSDNRDDSPEGIVKEIKEWRAANGKPGFKQGLEG
GGSHHHHHH
- SEQ ID NO: 21 corresponds to the so-called Myc-Im8-6His: 
MGGEQKLISEEDLGGSELKNSISDYTETEFKKIIEDIINCEGDEKKQDDNLEHF
ISVTEHPSGSDLIYYPEGNNDGSPEAVIKEIKEWRAANGKSGFKQGLEGGGS
HHHHHH
- SEQ ID NO: 22 corresponds to the so-called Myc-Im9-6His: 
MGGEQKLISEEDLGGSELKHSISDYTEAEFLQLVTTICNADTSSEEELVKLVT
HFEEMTEHPSGSDLIYYPKEGDDDSPSGIVNTVKQWRAANGKLEGGGSHHH
HHH
- SEQ ID NO: 23 corresponds to the so-called Myc-ImAP41-Avitag-6His: 
MGGEQKLISEEDLGGSDIKNNLSDYTESEFLEIIEEFFKNKSGLKGSELEKRM
DKLVKHFEEVTSHPRKSGVIFHPKPGFETPEGIVKEVKEWRAANGLPGFKA
GLEGGSGGSGLNDIFEAQKIEWHELEGGGSHHHHHH
- SEQ ID NO: 24 corresponds to the so-called Myc-ImSyr-Avitag-6His: 
MGGEQKLISEEDLGGSIFKEKIEDYTEEEFLEFLKGLSSEYSQLHGDEFIKHM
DRSVEHFVKITEHPAQTDVIFYPEEGQEDTPEGILKVIKEWRAKNGKPGFKS
GGSGGSGLNDIFEAQKIEWHELEGGGSHHHHHH
- SEQ ID NO: 25 corresponds to the so-called Myc-ImErw-Avitag-6His: 
MGGEQKLISEEDLGGSNLKEKLEDYTEAEFISYLKEFFDNPMGLRGKELETH
LDSLVEHFDKIVFHPEGNGLIFYPPDERDDSPEGVLNEIKRWRKSQGLPLFK
DSKGGSGGSGLNDIFEAQKIEWHELEGGGSHHHHHH
- SEQ ID NO: 26 corresponds to the so-called Avitag-ImLeaf-6His: 
MGLNDIFEAQKIEWHEGGSGGSGGSGGGGSKLGGGGSTAGGGGSKRQFADYT
EAEFIAFMEDIFRENEAETDDRLDVLLDQFREITGHPDGTDLIYYCESDAEC
TPERITQKVKSWRAANGLPGFKSTLEGGGSHHHHHH
- SEQ ID NO: 27 corresponds to the so-called Avitag-ImKhan-6His:

MGLNDIFEAQKIEWHEGGSGGSGGSGGGGSKLGGGGSTAGGGGSELKNKLED
YTEAEFLSLLNKIWAVDVSEEEHDNLIDHFEKLSEHPNGNGLIFYPENGVED
SPEGVLKVIKEWRAKNGKPGFKKLEGGGSHHHHHH

### Protein production and purification

Each plasmid was sequenced and the corresponding sequence was verified. For protein production, each plasmid was transformed into E. coli bacteria (BL21(DE3) strain, NEB) following manufacturer protocol and bacteria were selected on Kanamycin plate. The day after, all the bacteria from one plate were resuspended in 500 ml of LB medium plus kanamycin (50 µg/ml) and incubated at 37°C under shaking at 220 rpm. When the culture OD600 was close to 0.6, protein expression was induced with 0.1 mM of Isopropyl 3-D-1-thiogalactopyranoside (IPTG) and incubated overnight at 20°C, shaking at 220 rpm. Bacteria were then centrifuged for 30 min at 4°C at 4 000 relative centrifugal force (rcf) and washed in 50 mL of phosphate-buffered saline (PBS). The pellet was then resuspended in 5 mL of lysis buffer (50 mM Tris pH 7.5, 150 mM NaCl, 10 mM imidazole, containing 1x complete EDTA-free protease inhibitor, Roche) and 10 µg/mL of Deoxyribonuclease I from bovine pancreas (Sigma) and 1 mg/mL of lysozyme (Sigma) were added and incubated on ice for 30 min. Bacteria were lysed using glass particles. Alternatively, bacteria were resuspended in 10 ml of lysis buffer and sonicated for 1 minute and this process was repeated 4 times, with 1 minute off between each period of sonication. An other alternative is the disruption of cells at high pressure (2,5Kbar) with a OS system (Constant Systems Limited). The lysate was then centrifuged for 20 min at 14000 rcf at 4°C. Supernatant containing soluble proteins was then poured onto a Ni2+-NTA resin (QIAGEN) column at room temperature (RT). Resin was washed with 20 bed volumes of 50 mM Tris pH 7.5, 150 mM NaCl, 10 mM imidazole. Elution was performed with 50 mM Tris pH 7.5, 150 mM NaCl, 250 mM imidazole poured onto the column and collected in separate eppendorf tubes. The elution tubes containing the protein of interest were dialysed using Spectrum dialysis membrane Spectra/Por 6 (Thermo) in 4 L, 50 mM Tris H 7.5, 150 mM NaCl overnight at 4°C. Alternatively, eluted proteins were inserted in a gel filtration (GF Hiload 16/600 Superdex 75 µp from GE Healthcare) with the same buffer (50 mM Tris H 7.5, 150 mM NaCl).

### Enzyme Immuno-Assay (EIA) experiments with Colicins and Immunity proteins, 4 plex

100 µL of the chosen Colicin protein (SEQ ID NO: 1 to 4) diluted in carbonate buffer (final concentration 100 nM) were individually incubated at 4°C overnight on Nunc Maxisorp white 96-well plate (ThermoFischer). Each well was washed three times with 200 µL of PBS, 0.05% Tween 20 (PBST). Well saturation was achieved by incubation for 1 hour at RT with 2% (w/v) Bovine Serum Albumin (BSA) in PBST, shaking at 180 rpm. Each well was washed three times with 200 µL of PBST. 50 µL of 100 nM Immunity protein fused to RLuc8 (SEQ ID NO: 5 to 8) with or without Immunity protein (SEQ ID NO: 19 to 22) solutions (dilution in PBST 0.2% BSA (w/v)) were introduced into wells and incubated for 20 min at RT, shaking at 180 rpm. Each well was washed three times with 200 µL of PBST. 40 µL of RLuc8 buffer consisting of 25 mM HEPES pH 7,1 mM EDTA, 0.5% (v/v) Tergitol, 50 mM KI and 5 mM Thiosulfate were added into each well and subsequently 10 µL of the same buffer containing 5 µM of Coelenterazine H (Sigma) was added. After shaking at 180 rpm for 10 s, bioluminescence signal was measured with Tecan Infinite 200 Pro, using an integration time of 1000 ms in luminescence mode.

### Enzyme Immuno-Assay (EIA) experiments with Colicins and Immunity proteins, 8 plex n°1

100 µL of the chosen Colicin protein (SEQ ID NO: 1 to 4 , 9 to 11 and 13) diluted in carbonate buffer (final concentration 100 nM) were individually incubated at room temperature (RT) overnight on Costar High Binding white 96-well plate (Corning). Each well was washed three times with 250 µL of PBS, 0.05% Tween 20 (PBST). Well saturation was achieved by incubation for 2 hours at RT with 1% (w/v) Casein in PBST, shaking at 280 rpm. Each well was washed three times with 250 µL of PBST. 100 µL of 100 nM Immunity protein fused to RLuc8 (SEQ ID NO: 5 to 8, 14 to 16 and 18) with or without Immunity protein (SEQ ID NO: 19 to 25 and 27) solutions (dilution in PBST 0.1% Casein (w/v)) were introduced into wells and incubated for 15 min at RT, shaking at 280 rpm. Each well was washed three times with 250 µL of PBST.

100 µL of RLuc8 revelation buffer consisting of 25 mM HEPES pH 7,1 mM EDTA, 0.5% (v/v) Tergitol, 50 mM KI, 5 mM Thiosulfate and 1 µM of Coelenterazine H were added into each. After shaking manually, bioluminescence signal was measured with the EnSight HH3400 (Perkin Elmer) reader using an integration time of 0.2 s in single luminescence mode.

### Enzyme Immuno-Assay (EIA) experiments with Colicins and Immunity proteins, 8 plex n°2

100 µL of the chosen Colicin protein (SEQ ID NO: 1, 3, 4, 9 to 13) diluted in carbonate buffer (final concentration 100 nM) were individually incubated at room temperature (RT) overnight on Costar High Binding white 96-well plate (Corning). Each well was washed three times with 250 µL of PBS, 0.05% Tween 20 (PBST). Well saturation was achieved by incubation for 2 hours at RT with 1% (w/v) Casein in PBST, shaking at 280 rpm. Each well was washed three times with 250 µL of PBST. 100 µL of 100 nM Immunity protein fused to RLuc8 (SEQ ID NO: 5, 7, 8, 14 to 18) with or without Immunity protein (SEQ ID NO: 19 and 21 to 27) solutions (dilution in PBST 0.1% Casein (w/v)) were introduced into wells and incubated for 15 min at RT, shaking at 280 rpm. Each well was washed three times with 250 µL of PBST.

100 µL of RLuc8 revelation buffer consisting of 25 mM HEPES pH 7,1 mM EDTA, 0.5% (v/v) Tergitol, 50 mM KI, 5 mM Thiosulfate and 1 µM of Coelenterazine H were added into each. After shaking manually, bioluminescence signal was measured with the EnSight HH3400 (Perkin Elmer) reader using an integration time of 0.2 s in single luminescence mode.

### Multiplexed LFA-liked experiment with Colicins and Immunity proteins

Nitrocellulose membranes were sprayed with different mCherry- or EmGFP-fused Colicin proteins (SEQ ID NO: 1 to 4) at 10 µM. 10 µL of 100 nM RLuc8-fused Immunity protein (SEQ ID NO: 5 to 8) solution (alone or in the presence of the 3 other Immunity proteins as indicated in figure 2) diluted in PBST with 0.2% BSA (w/v) was deposited on the conjugated pad, followed by a deposition of 100 µL of PBS with 0.2% BSA (w/v) and Tween 20 0.05% (v/v) on the sample pad. Following a migration of 10 minutes at room temperature, membrane was separated from pads and 40 µL of RLuc8 buffer (consisting of 25 mM HEPES pH7, 1 mM EDTA, 0.5% (v/v) Tergitol, 50 mM KI and 5 mM Thiosulfate with 5 µM of Coelenterazine H) was deposited on the membrane. Pictures were acquired with a FusionFX Vilber Lourmat bioluminescent imager. 10 acquisitions with exposure time of 10 seconds were recorded and intensity of each photo was accumulated with the previous ones. Last picture before reaching saturation was chosen for the analysis.

### RESULTS

### A/ Proof of concept based on the ColE2/E7/E8/E9 couples

In an attempt to establish a multiplex analysis, advantage was taken of the very high affinity of DNase Colicins (ColE) to their cognate Immunity protein (Im) (Kd ∼ 10^{-14/-15} M). Because Im proteins are able to bind to non-cognate Colicin proteins with an affinity ranging from 10⁻⁶ to 10⁻¹⁰ M, the capability of these proteins to be used in multiplex assays, without significant cross-reactivity, was first assessed. Experiments were designed to first evaluate the cross-reactivity and second their potential use in a test mimicking a multiplex assay. The interactions of both cognate and non-cognate pairs of proteins were thus tested, each couple separately, to quantify and compare the interactions. Experiments were performed with the Colicin proteins (E2, E7, E8 and E9) that were incubated individually with every Im protein fused to RLuc8 as a reporter and Luciferase activity was evaluated. These experiments were performed using a EIA or LFA format.

### 1) Immunoassay (EIA) tests

For EIA experiments, the Colicins (E2, E7, E8 and E9) were individually coated on a well of Maxisorb 96-well plate and one Im protein (Im2 or Im7 or Im8 or Im9) fused to RLuc8 was added into each well. Following incubation at room temperature for 15 minutes and washing steps, the Luciferase activity was monitored following substrate addition. Results are presented in Figure 1, light grey bars. In these experimental conditions, a strong interaction between cognate binding partners (RLU above 400 000) was observed, while the interaction of non-cognate Im protein fused to RLuc8 was very low but still reproducible (between 2 000 and 35 000 RLU) depending the pair of interacting proteins. They show that non-cognate complexes are formed when an Im protein fused to RLuc8 is incubated alone with a non-cognate ColE, the highest non-cognate interactions being reported for the couples Im9-ColE2, Im2-ColE7, Im8-ColE7, Im2-ColE8, Im7-ColE8 and Im2-ColE9. It is interesting to note that this cross-reactivity was not quantitatively correlated with the affinity constant (Kd) of each pair. For example, binding of Im7-RLuc8 or Im8-RLuc8 or Im9-RLuc8 to ColE2 occurs independently of their respective affinity to ColE2 (Figure 1). Im2 and Im9 binding to ColE2 results in 5 000 RLU and 20 000 RLU respectively while their Kd for ColE2 is almost the same (1.4x10⁻⁸ M vs 1.2x10⁻⁸ M) (Figure 1, first panel, grey bars). This is also true for the interaction of ColE7 with Im2-RLuc8 and Im9-RLuc8: Im2-RLuc8 does interact with ColE7 while Im9-RLuc8 interaction is barely detected, despite having very close Kd for ColE7 (1.8x10⁻⁸ and 6.4x10⁻⁸ respectively) (Figure 1, third panel, grey bars).

Then, each ColE was incubated with a mix containing an Im-Rluc8 fusion protein plus the 3 other Im proteins, devoid of Luciferase activity (Im protein without fusion) (Figure 1, dark grey bars). This setup is close to a multiplex assay, where all the binding partners will be incubated together in the same buffer. Unexpectedly, when using this experimental design, any cross-reactivity between non cognate partners was completely abolished when ColE2, ColE7 and ColE8 were coated on the plate (Figure 1, left three panels, dark grey bars). In contrast, ColE9 was still cross-reacting with both Im2 and to a lesser extent, with ColE8 (Figure 1, dark grey bars, right panel). Of note, there is no effect on the cognate binding reactions, which validate the concept of using these binding partners in future multiplex assays, especially ColE2, ColE7 and ColE9.

### 2) LFA tests

The cross-reactivity of Im proteins towards non cognate biding partners was next evaluated in Lateral Flow Assay, where the 4 ColE proteins (1µl of each ColE at 10 µM) were sprayed as line on nitrocellulose membranes, in the following order: ColE8, ColE2, ColE9, ColE7. The sample pad is on the left and the adsorbent pad on the right. Each Im protein fused to Rluc8 alone was first applied on the sample pad (7 µl of 10 nM Im-RLuc8 diluted in 100 µl of PBS 0.2% BSA, 0.05% Tween-20) and then the Luciferase activity was monitored on the membrane (Figure 2, left panel). In this format, a strong cross-reactivity was observed between Im2-RLuc8 and ColE7, whereas Im7-RLuc8 and Im8-RLuc8 cross-reacted moderately with ColE8 and ColE7 respectively (Figure 2, left panel). Interestingly, the observed cross-reactivity are in agreement with Kd values for these non cognate binding (Im2 and ColE2 3.6x10⁻¹⁰ M, Im8 and ColE7 3.7x10⁻¹⁰ M, Im7 and ColE8 1.0x10⁻⁹ M). A weaker interaction of Im9-RLuc8 with ColE9 was observed, which was not expected (Figure 2, left panel). The experiments were repeated with the addition of 4 Im proteins in the same solution, one only being fused to RLuc8 (Figure 2, right panel). This setting completely abolished the cross-reactivity of non cognate partners, as already observed in EIA. It is also to be noted that the overall band intensity is also decreased (Figure 2, compare left and right panels).

### 3) Conclusions

In both EIA and LFA formats, a cross-reactive binding between non-cognate pairs was observed when one Im protein is incubated with non cognate ColE, which appears stronger in experimental settings used in LFA. When incubation is carried out with all the 4 Im proteins together in the solution, with only one fused to RLuc8, this cross-reactivity was completely abrogated, in both EIA and LFA, demonstrating the capability of this system to be used in multiplex assays.

### B/ Proof of concept with further Col/Im couples at a larger scale (8 plex)

In an attempt to further exemplify the multiplex capability according to the invention, advantage was taken of the very high affinity of five newly identified DNase Colicins to their cognate Immunity proteins (Im). Experiments were designed to evaluate the cross-reactivity and their potential use in a test mimicking a multiplex assay based on 8 couples. The interactions of both cognate and non-cognate pairs of proteins were tested. Experiments were performed with the Colicin proteins ColE2, ColE7, ColE8, ColE9, ColAP41, ColSyr, ColErw, ColKhan for the 8plex n°1 and ColE2, ColE8, ColE9, ColAP41, ColSyr, ColErw, ColLeaf, ColKhan for the 8plex n°2 that were incubated individually with Im2, Im7, Im8, Im9, ImAP41, ImSyr, ImErw, ImKhan proteins (for the 8 plex n°1) or Im2, Im8, Im9, ImAP41, ImSyr, ImErw, ImLeaf, ImKhan proteins (for the 8 plex n°2) fused or not to RLuc8 as a reporter. Luciferase activity was evaluated. These experiments were performed using an EIA format.

### Enzyme Immuno-Assay (EIA) experiments with Colicins and Immunity proteins, 8 plex n°1

Colicins (ColE2, ColE7, ColE8, ColE9, ColAP41, ColSyr, ColErw, ColKhan) were individually coated on a well of Maxisorb 96-well plate and one Im protein (Im2, Im7, Im8, Im9, ImAP41, ImSyr, ImErw, ImKhan) fused to RLuc8 was added into each well. Following incubation at room temperature for 15 minutes and washing steps, the Luciferase activity was monitored following substrate addition. Results are presented in Figure 3, light grey bars. In these experimental conditions, a strong interaction between cognate binding partners (RLU above 2x10⁶ RLU) was observed, while the interaction of non-cognate Im protein fused to RLuc8 was low and reproducible (between 800 and 255 000 RLU) depending the pair of interacting proteins. They show that non-cognate complexes are formed when an Im protein fused to RLuc8 is incubated alone with a non-cognate ColE, the highest non-cognate interactions (above 10⁵ RLU) being reported for the couples Im8-ColE7, Im8-ColE9, Im8-ColErw, Im9-ColE7, Im9-ColAP41, Im9-ColSyr, Im9-ColKhan, ImSyr-ColE7, ImKhan-ColErw.

Then, each Colicin was incubated with a mix containing an Im-Rluc8 fusion protein plus the 7 other Im proteins, devoid of Luciferase activity (Im protein without fusion) (Figure 3, dark grey bars). This setup is close to a multiplex assay, where all the binding partners will be incubated together in the same buffer. Unexpectedly, when using this experimental design, any cross-reactivity between non cognate partners was completely abolished when Im2-Rluc8 or Im7-Rluc8 or ImAP41-Rluc8 or ImSyr-Rluc8 or ImErw-Rluc8 or ImKhan-Rluc8 plus the 7 other Im proteins, devoid of Luciferase activity (Im protein without fusion) were deposited within the well (Figure 3, dark grey bars). Regarding Im8-Rluc8 or Im9-Rluc8, plus the 7 other Im proteins, devoid of Luciferase activity (Im protein without fusion), cross-reaction was significantly decreased (Figure 3, dark grey bars) compared to the signal measured when Im8-Rluc8 or Im9-Rluc8 were solely added into each well (Figure 3, light grey bars).

Of note, there is no effect on the cognate binding reactions, which validate the concept of using these binding partners in future multiplex assays.

The "no coating" graph (Figure 3) illustrates the "sticky" behavior of Im9 and Im8 toward the 96-well plate material. In fact, without any Colicin coated on the surface of the well, a non-specific signal is generated in the case of Im9 and Im8 to a lower extent. Interestingly, the non-specific signal is significantly decreased when Im9-Rluc8 or Im8-Rluc8, plus the 7 other Im proteins (devoid of Luciferase) are used.

A non-binding hypothesis is that non-specific binding of Im9 and Im8 to the surface of 96-well plate is occurring. Additional saturating coating agent (proteins, polymers ...) could be used to prevent this non-specific binding.

### Enzyme Immuno-Assay (EIA) experiments with Colicins and Immunity proteins, 8 plex n°2

Colicins (ColE2, ColE8, ColE9, ColAP41, ColSyr, ColErw, ColLeaf, ColKhan) were individually coated on a well of Maxisorb 96-well plate and one Im protein (Im2, Im8, Im9, ImAP41, ImSyr, ImErw, ImLeaf, ImKhan) fused to RLuc8 was added into each well. Following incubation at room temperature for 15 minutes and washing steps, the Luciferase activity was monitored following substrate addition. Results are presented in Figure 4, light grey bars. In these experimental conditions, a strong interaction between cognate binding partners (RLU above 2.4 x10⁶ RLU) was observed, while the interaction of non-cognate Im protein fused to RLuc8 was low and reproducible (between 1300 and 275000 RLU) depending the pair of interacting proteins. They show that non-cognate complexes are formed when an Im protein fused to RLuc8 is incubated alone with a non-cognate ColE, the highest non-cognate interactions (above 10⁵ RLU) being reported for the couples Im8-ColLeaf, Im9-ColE8, Im9-ColAP41, Im9-ColErw, Im9-ColLeaf, Im9-ColKhan, ImKhan-ColErw.

Then, each Colicin was incubated with a mix containing an Im-Rluc8 fusion protein plus the 7 other Im proteins, devoid of Luciferase activity (Im protein without fusion) (Figure 4, dark grey bars). This setup is close to a multiplex assay, where all the binding partners will be incubated together in the same buffer. Unexpectedly, when using this experimental design, any cross-reactivity between non cognate partners was completely abolished when Im2-Rluc8 or ImAP41-Rluc8 or ImSyr-Rluc8 or ImErw-Rluc8 or ImLeaf-Rluc8 or ImKhan-Rluc8 plus the 7 other Im proteins, devoid of Luciferase activity (Im protein without fusion) were deposited within the well (Figure 4, dark grey bars). Regarding Im8-Rluc8 or Im9-Rluc8, plus the 7 other Im proteins, devoid of Luciferase activity (Im protein without fusion), cross-reaction was significantly decreased (Figure 4, dark grey bars) compared to the signal measured when Im8-Rluc8 or Im9-Rluc8 were solely added into each well (Figure 4, light grey bars).

Of note, there is no effect on the cognate binding reactions, which validate the concept of using these binding partners in future multiplex assays.

The "no coating" graph (Figure 4) illustrates the "sticky" behavior of Im9 and Im8 toward the 96-well plate material. In fact, without any Colicin coated on the surface of the well, a non-specific signal is generated in the case of Im9 and Im8 to a lower extent. Interestingly, the non-specific signal is significantly decreased when Im9-Rluc8 or Im8-Rluc8, plus the 7 other Im proteins (devoid of Luciferase) are used.

Once again, a non-binding hypothesis is hypothesis is that non-specific binding of Im9 and Im8 to the surface of 96-well plate is occurring. Additional saturating coating agent (proteins, polymers ...) could be used to prevent this non-specific binding.

### 3) Conclusions

A cross-reactive binding between non-cognate pairs was observed when one Im protein is incubated with non cognate Colicin. When incubation is carried out with all the 8 Im proteins together in the solution, with only one fused to RLuc8, this cross-reactivity was abrogated, demonstrating the capability of this system to be used in large multiplex assays.

## Claims

1. A support for a multiplex binding experiment functionalized with at least two different polypeptides having high affinity to their cognate binding partner, at least one polypeptide being a bacteriocin or its cognate Immunity protein (Im).

2. The support of claim 1, wherein two polypeptides are a bacteriocin or its cognate Immunity protein (Im).

3. The support of claim 1 or 2, wherein the at least one polypeptide is a bacteriocin, a mutant or a fragments thereof.

4. The support of claim 3, wherein the at least one polypeptide contains the cytotoxic domain of a bacteriocin, possibly mutated to be deprived of cytotoxic activity.

5. The support of claim 3 or 4, wherein the at least one polypeptide contains the sequence SEQID NO: 28, SEQ ID NO: 29 or SEQ ID NO: 30.

6. The support of claim 5, wherein the at least one polypeptide has or contains a sequence chosen in the following group: residues 254 to 386 of SEQ ID NO:1, residues 254 to 386 of SEQ ID NO: 2, residues 251 to 383 of SEQ ID NO: 3, residues 251 to 383 of SEQ ID NO: 4, residues 255 to 390 of SEQ ID NO: 9, residues 255 to 388 of SEQ ID NO: 10, residues 255 to 383 of SEQ ID NO: 11, residues 261 to 394 of SEQ ID NO: 12 and residues 261 to 393 of SEQ ID NO: 13.

7. The support of claim 1 or 2, wherein the at least one polypeptide is an Immunity protein, a mutant or a fragment thereof.

8. The support of claim 7, wherein the at least one polypeptide has or contains a sequence chosen in the following group: residues 329 to 413 of SEQ ID NO: 5, residues 329 to 414 of SEQ ID NO: 6, residues 329 to 412 of SEQ ID NO: 7, residues 329 to 407 of SEQ ID NO: 8, residues 333 to 423 of SEQ ID NO: 14, residues 333 to 420 of SEQ ID NO: 15, residues 333 to 423 of SEQ ID NO: 16, residues 347 to 430 of SEQ ID NO: 17, and residues 347 to 429 of SEQ ID NO: 18.

9. The support of claim 1 or 2, wherein the at least one polypeptide has a sequence selected in the group consisting of: SEQ ID NO: 1 to SEQ ID NO: 27.

10. The support of any of claims 1 to 9, wherein another polypeptide is a binding partner of biotin, advantageously avidin, streptavidin, neutrAvidin or an antibiotin antibody.

11. A reaction mixture comprising the cognate binding partners of the polypeptides as defined in claims 1 to 10.

12. A reaction mixture according to claim 11, wherein the cognate binding partners further comprise an analyte-capture entity, advantageously a polypeptide.

13. A kit comprising a support according to any of claims 1 to 10 and a reaction mixture according to any of claims 11 to 12, advantageously further comprising labeled detection entities.

14. Use of a support according to any of claims 1 to 10 and a reaction mixture according to any of claims 11 to 12, or of a kit according to claim 13 to perform multiplex binding experiments, advantageously immunoassays, more advantageously chosen in the group consisting of: enzymatic immunoassays, lateral flow assays and vertical flow assays.

15. A method for detecting at least 2 analytes, possibly contained in a sample, comprising:
(a) contacting a support according to any of claims 1 to 10, a reaction mixture according to any of claims 11 to 12, the analytes or the sample containing said analytes, and the labeled detection entities of claim 13; and
(b) testing the labeling, thereby detecting the presence of the analytes.


SEQUENCE LISTING

<110> BIOASTER

<120> METHODS AND REAGENTS FOR MULTIPLEX BINDING EXPERIMENTS

<130> B344-B-53149 EP DIV

<140> PCT/EP2019/081692
<141> 2019-11-18

<150> EP18306517.6
<151> 2018-11-19

<160> 30

<170> BiSSAP 1.3.6

<210> 1
<211> 398
<212> PRT
<213> Artificial Sequence

<220>
<223> Flag-EmGFP-ColE2-6His

<400> 1
Met Gly Gly Asp Tyr Lys Asp Asp Asp Asp Lys Gly Gly Ser Val Ser
1               5                   10                  15
Lys Gly Glu Glu Leu Phe Thr Gly Val Val Pro Ile Leu Val Glu Leu
            20                  25                  30
Asp Gly Asp Val Asn Gly His Lys Phe Ser Val Ser Gly Glu Gly Glu
        35                  40                  45
Gly Asp Ala Thr Tyr Gly Lys Leu Thr Leu Lys Phe Ile Cys Thr Thr
    50                  55                  60
Gly Lys Leu Pro Val Pro Trp Pro Thr Leu Val Thr Thr Leu Thr Tyr
65                  70                  75                  80
Gly Val Gln Cys Phe Ala Arg Tyr Pro Asp His Met Lys Gln His Asp
                85                  90                  95
Phe Phe Lys Ser Ala Met Pro Glu Gly Tyr Val Gln Glu Arg Thr Ile
            100                 105                 110
Phe Phe Lys Asp Asp Gly Asn Tyr Lys Thr Arg Ala Glu Val Lys Phe
        115                 120                 125
Glu Gly Asp Thr Leu Val Asn Arg Ile Glu Leu Lys Gly Ile Asp Phe
    130                 135                 140
Lys Glu Asp Gly Asn Ile Leu Gly His Lys Leu Glu Tyr Asn Tyr Asn
145                 150                 155                 160
Ser His Lys Val Tyr Ile Thr Ala Asp Lys Gln Lys Asn Gly Ile Lys
                165                 170                 175
Val Asn Phe Lys Thr Arg His Asn Ile Glu Asp Gly Ser Val Gln Leu
            180                 185                 190
Ala Asp His Tyr Gln Gln Asn Thr Pro Ile Gly Asp Gly Pro Val Leu
        195                 200                 205
Leu Pro Asp Asn His Tyr Leu Ser Thr Gln Ser Ala Leu Ser Lys Asp
    210                 215                 220
Pro Asn Glu Lys Arg Asp His Met Val Leu Leu Glu Phe Val Thr Ala
225                 230                 235                 240
Ala Gly Ile Thr Leu Gly Met Asp Glu Leu Tyr Lys Leu Glu Ser Lys
                245                 250                 255
Arg Asn Lys Pro Gly Lys Ala Thr Gly Lys Gly Lys Pro Val Gly Asp
            260                 265                 270
Lys Trp Leu Asp Asp Ala Gly Lys Asp Ser Gly Ala Pro Ile Pro Asp

275                 280                 285
Arg Ile Ala Asp Lys Leu Arg Asp Lys Glu Phe Lys Asn Phe Asp Asp
    290                 295                 300
Phe Arg Lys Lys Phe Trp Glu Glu Val Ser Lys Asp Pro Asp Leu Ser
305                 310                 315                 320
Lys Gln Phe Lys Gly Ser Asn Lys Thr Asn Ile Gln Lys Gly Lys Ala
                325                 330                 335
Pro Phe Ala Arg Lys Lys Asp Gln Val Gly Gly Arg Glu Arg Phe Glu
            340                 345                 350
Leu His Ala Asp Lys Pro Ile Ser Gln Asp Gly Gly Val Tyr Asp Met
        355                 360                 365
Asn Asn Ile Arg Val Thr Thr Pro Lys Arg His Ile Asp Ile His Arg
    370                 375                 380
Gly Lys Leu Glu Gly Gly Gly Ser His His His His His His
385                 390                 395

<210> 2
<211> 398
<212> PRT
<213> Artificial Sequence

<220>
<223> Flag-EmGFP-ColE7-6His

<400> 2
Met Gly Gly Asp Tyr Lys Asp Asp Asp Asp Lys Gly Gly Ser Val Ser
1               5                   10                  15
Lys Gly Glu Glu Leu Phe Thr Gly Val Val Pro Ile Leu Val Glu Leu
            20                  25                  30
Asp Gly Asp Val Asn Gly His Lys Phe Ser Val Ser Gly Glu Gly Glu
        35                  40                  45
Gly Asp Ala Thr Tyr Gly Lys Leu Thr Leu Lys Phe Ile Cys Thr Thr
    50                  55                  60
Gly Lys Leu Pro Val Pro Trp Pro Thr Leu Val Thr Thr Leu Thr Tyr
65                  70                  75                  80
Gly Val Gln Cys Phe Ala Arg Tyr Pro Asp His Met Lys Gln His Asp
                85                  90                  95
Phe Phe Lys Ser Ala Met Pro Glu Gly Tyr Val Gln Glu Arg Thr Ile
            100                 105                 110
Phe Phe Lys Asp Asp Gly Asn Tyr Lys Thr Arg Ala Glu Val Lys Phe
        115                 120                 125
Glu Gly Asp Thr Leu Val Asn Arg Ile Glu Leu Lys Gly Ile Asp Phe
    130                 135                 140
Lys Glu Asp Gly Asn Ile Leu Gly His Lys Leu Glu Tyr Asn Tyr Asn
145                 150                 155                 160
Ser His Lys Val Tyr Ile Thr Ala Asp Lys Gln Lys Asn Gly Ile Lys
                165                 170                 175
Val Asn Phe Lys Thr Arg His Asn Ile Glu Asp Gly Ser Val Gln Leu
            180                 185                 190
Ala Asp His Tyr Gln Gln Asn Thr Pro Ile Gly Asp Gly Pro Val Leu
        195                 200                 205
Leu Pro Asp Asn His Tyr Leu Ser Thr Gln Ser Ala Leu Ser Lys Asp
    210                 215                 220
Pro Asn Glu Lys Arg Asp His Met Val Leu Leu Glu Phe Val Thr Ala
225                 230                 235                 240
Ala Gly Ile Thr Leu Gly Met Asp Glu Leu Tyr Lys Leu Glu Ser Lys
                245                 250                 255
Arg Asn Lys Pro Gly Lys Ala Thr Gly Lys Gly Lys Pro Val Asn Asn
            260                 265                 270
Lys Trp Leu Asn Asn Ala Gly Lys Asp Leu Gly Ser Pro Val Pro Asp
        275                 280                 285
Arg Ile Ala Asn Lys Leu Arg Asp Lys Glu Phe Lys Ser Phe Asp Asp

290                 295                 300
Phe Arg Lys Lys Phe Trp Glu Glu Val Ser Lys Asp Pro Glu Leu Ser
305                 310                 315                 320
Lys Gln Phe Ser Arg Asn Asn Asn Asp Arg Met Lys Val Gly Lys Ala
                325                 330                 335
Pro Lys Thr Arg Thr Gln Asp Val Ser Gly Lys Arg Thr Ser Phe Glu
            340                 345                 350
Leu His Ala Glu Lys Pro Ile Ser Gln Asn Gly Gly Val Tyr Asp Met
        355                 360                 365
Asp Asn Ile Ser Val Val Thr Pro Lys Arg His Ile Asp Ile His Arg
    370                 375                 380
Gly Lys Leu Glu Gly Gly Gly Ser His His His His His His
385                 390                 395

<210> 3
<211> 395
<212> PRT
<213> Artificial Sequence

<220>
<223> Flag-mCherry-ColE8-6His

<400> 3
Met Gly Gly Asp Tyr Lys Asp Asp Asp Asp Lys Gly Gly Ser Val Ser
1               5                   10                  15
Lys Gly Glu Glu Asp Asn Met Ala Ile Ile Lys Glu Phe Met Arg Phe
            20                  25                  30
Lys Val His Met Glu Gly Ser Val Asn Gly His Glu Phe Glu Ile Glu
        35                  40                  45
Gly Glu Gly Glu Gly Arg Pro Tyr Glu Gly Thr Gln Thr Ala Lys Leu
    50                  55                  60
Lys Val Thr Lys Gly Gly Pro Leu Pro Phe Ala Trp Asp Ile Leu Ser
65                  70                  75                  80
Pro Gln Phe Met Tyr Gly Ser Lys Ala Tyr Val Lys His Pro Ala Asp
                85                  90                  95
Ile Pro Asp Tyr Leu Lys Leu Ser Phe Pro Glu Gly Phe Lys Trp Glu
            100                 105                 110
Arg Val Met Asn Phe Glu Asp Gly Gly Val Val Thr Val Thr Gln Asp
        115                 120                 125
Ser Ser Leu Gln Asp Gly Glu Phe Ile Tyr Lys Val Lys Leu Arg Gly
    130                 135                 140
Thr Asn Phe Pro Ser Asp Gly Pro Val Met Gln Lys Lys Thr Met Gly
145                 150                 155                 160
Trp Glu Ala Ser Ser Glu Arg Met Tyr Pro Glu Asp Gly Ala Leu Lys
                165                 170                 175
Gly Glu Ile Lys Gln Arg Leu Lys Leu Lys Asp Gly Gly His Tyr Asp
            180                 185                 190
Ala Glu Val Lys Thr Thr Tyr Lys Ala Lys Lys Pro Val Gln Leu Pro
        195                 200                 205
Gly Ala Tyr Asn Val Asn Ile Lys Leu Asp Ile Thr Ser His Asn Glu
    210                 215                 220
Asp Tyr Thr Ile Val Glu Gln Tyr Glu Arg Ala Glu Gly Arg His Ser
225                 230                 235                 240
Thr Gly Gly Met Asp Glu Leu Tyr Lys Leu Glu Ser Lys Arg Asn Lys
                245                 250                 255
Pro Gly Lys Ala Thr Gly Lys Gly Lys Pro Val Gly Asp Lys Trp Leu
            260                 265                 270
Asp Asp Ala Gly Lys Asp Ser Gly Ala Pro Ile Pro Asp Arg Ile Ala
        275                 280                 285
Asp Lys Leu Arg Asp Lys Glu Phe Lys Asn Phe Asp Asp Phe Arg Arg
    290                 295                 300
Lys Phe Trp Glu Glu Val Ser Lys Asp Pro Glu Leu Ser Lys Gln Phe

305                 310                 315                 320
Asn Pro Gly Asn Lys Lys Arg Leu Ser Gln Gly Leu Ala Pro Arg Ala
                325                 330                 335
Arg Asn Lys Asp Thr Val Gly Gly Arg Arg Ser Phe Glu Leu His Ala
            340                 345                 350
Asp Lys Pro Ile Ser Gln Asp Gly Gly Val Tyr Asp Met Asp Asn Leu
        355                 360                 365
Arg Ile Thr Thr Pro Lys Arg His Ile Asp Ile His Arg Gly Gln Leu
    370                 375                 380
Glu Gly Gly Gly Ser His His His His His His
385                 390                 395

<210> 4
<211> 395
<212> PRT
<213> Artificial Sequence

<220>
<223> Flag-mCherry-ColE9-6His

<400> 4
Met Gly Gly Asp Tyr Lys Asp Asp Asp Asp Lys Gly Gly Ser Val Ser
1               5                   10                  15
Lys Gly Glu Glu Asp Asn Met Ala Ile Ile Lys Glu Phe Met Arg Phe
            20                  25                  30
Lys Val His Met Glu Gly Ser Val Asn Gly His Glu Phe Glu Ile Glu
        35                  40                  45
Gly Glu Gly Glu Gly Arg Pro Tyr Glu Gly Thr Gln Thr Ala Lys Leu
    50                  55                  60
Lys Val Thr Lys Gly Gly Pro Leu Pro Phe Ala Trp Asp Ile Leu Ser
65                  70                  75                  80
Pro Gln Phe Met Tyr Gly Ser Lys Ala Tyr Val Lys His Pro Ala Asp
                85                  90                  95
Ile Pro Asp Tyr Leu Lys Leu Ser Phe Pro Glu Gly Phe Lys Trp Glu
            100                 105                 110
Arg Val Met Asn Phe Glu Asp Gly Gly Val Val Thr Val Thr Gln Asp
        115                 120                 125
Ser Ser Leu Gln Asp Gly Glu Phe Ile Tyr Lys Val Lys Leu Arg Gly
    130                 135                 140
Thr Asn Phe Pro Ser Asp Gly Pro Val Met Gln Lys Lys Thr Met Gly
145                 150                 155                 160
Trp Glu Ala Ser Ser Glu Arg Met Tyr Pro Glu Asp Gly Ala Leu Lys
                165                 170                 175
Gly Glu Ile Lys Gln Arg Leu Lys Leu Lys Asp Gly Gly His Tyr Asp
            180                 185                 190
Ala Glu Val Lys Thr Thr Tyr Lys Ala Lys Lys Pro Val Gln Leu Pro
        195                 200                 205
Gly Ala Tyr Asn Val Asn Ile Lys Leu Asp Ile Thr Ser His Asn Glu
    210                 215                 220
Asp Tyr Thr Ile Val Glu Gln Tyr Glu Arg Ala Glu Gly Arg His Ser
225                 230                 235                 240
Thr Gly Gly Met Asp Glu Leu Tyr Lys Leu Glu Ser Lys Arg Asn Lys
                245                 250                 255
Pro Gly Lys Ala Thr Gly Lys Gly Lys Pro Val Gly Asp Lys Trp Leu
            260                 265                 270
Asp Asp Ala Gly Lys Asp Ser Gly Ala Pro Ile Pro Asp Arg Ile Ala
        275                 280                 285
Asp Lys Leu Arg Asp Lys Glu Phe Lys Ser Phe Asp Asp Phe Arg Lys
    290                 295                 300
Ala Val Trp Glu Glu Val Ser Lys Asp Pro Glu Leu Ser Lys Asn Leu
305                 310                 315                 320
Asn Pro Ser Asn Lys Ser Ser Val Ser Lys Gly Tyr Ser Pro Phe Thr

                325                 330                 335
Pro Lys Asn Gln Gln Val Gly Gly Arg Lys Val Tyr Glu Leu His Ala
            340                 345                 350
Asp Lys Pro Ile Ser Gln Gly Gly Glu Val Tyr Asp Met Asp Asn Ile
        355                 360                 365
Arg Val Thr Thr Pro Lys Arg His Ile Asp Ile His Arg Gly Lys Leu
    370                 375                 380
Glu Gly Gly Gly Ser His His His His His His
385                 390                 395

<210> 5
<211> 425
<212> PRT
<213> Artificial Sequence

<220>
<223> Myc-RLuc8-Im2-6His

<400> 5
Met Gly Gly Glu Gln Lys Leu Ile Ser Glu Glu Asp Leu Gly Gly Ser
1               5                   10                  15
Ala Ser Lys Val Tyr Asp Pro Glu Gln Arg Lys Arg Met Ile Thr Gly
            20                  25                  30
Pro Gln Trp Trp Ala Arg Cys Lys Gln Met Asn Val Leu Asp Ser Phe
        35                  40                  45
Ile Asn Tyr Tyr Asp Ser Glu Lys His Ala Glu Asn Ala Val Ile Phe
    50                  55                  60
Leu His Gly Asn Ala Thr Ser Ser Tyr Leu Trp Arg His Val Val Pro
65                  70                  75                  80
His Ile Glu Pro Val Ala Arg Cys Ile Ile Pro Asp Leu Ile Gly Met
                85                  90                  95
Gly Lys Ser Gly Lys Ser Gly Asn Gly Ser Tyr Arg Leu Leu Asp His
            100                 105                 110
Tyr Lys Tyr Leu Thr Ala Trp Phe Glu Leu Leu Asn Leu Pro Lys Lys
        115                 120                 125
Ile Ile Phe Val Gly His Asp Trp Gly Ala Ala Leu Ala Phe His Tyr
    130                 135                 140
Ala Tyr Glu His Gln Asp Arg Ile Lys Ala Ile Val His Met Glu Ser
145                 150                 155                 160
Val Val Asp Val Ile Glu Ser Trp Asp Glu Trp Pro Asp Ile Glu Glu
                165                 170                 175
Asp Ile Ala Leu Ile Lys Ser Glu Glu Gly Glu Lys Met Val Leu Glu
            180                 185                 190
Asn Asn Phe Phe Val Glu Thr Val Leu Pro Ser Lys Ile Met Arg Lys
        195                 200                 205
Leu Glu Pro Glu Glu Phe Ala Ala Tyr Leu Glu Pro Phe Lys Glu Lys
    210                 215                 220
Gly Glu Val Arg Arg Pro Thr Leu Ser Trp Pro Arg Glu Ile Pro Leu
225                 230                 235                 240
Val Lys Gly Gly Lys Pro Asp Val Val Gln Ile Val Arg Asn Tyr Asn
                245                 250                 255
Ala Tyr Leu Arg Ala Ser Asp Asp Leu Pro Lys Leu Phe Ile Glu Ser
            260                 265                 270
Asp Pro Gly Phe Phe Ser Asn Ala Ile Val Glu Gly Ala Lys Lys Phe
        275                 280                 285
Pro Asn Thr Glu Phe Val Lys Val Lys Gly Leu His Phe Leu Gln Glu
    290                 295                 300
Asp Ala Pro Asp Glu Met Gly Lys Tyr Ile Lys Ser Phe Val Glu Arg
305                 310                 315                 320
Val Leu Lys Asn Glu Gln Lys Leu Glu Leu Lys His Ser Ile Ser Asp
                325                 330                 335
Tyr Thr Glu Ala Glu Phe Leu Glu Phe Val Lys Lys Ile Cys Arg Ala

340                 345                 350
Glu Gly Ala Thr Glu Glu Asp Asp Asn Lys Leu Val Arg Glu Phe Glu
        355                 360                 365
Arg Leu Thr Glu His Pro Asp Gly Ser Asp Leu Ile Tyr Tyr Pro Arg
    370                 375                 380
Asp Asp Arg Glu Asp Ser Pro Glu Gly Ile Val Lys Glu Ile Lys Glu
385                 390                 395                 400
Trp Arg Ala Ala Asn Gly Lys Ser Gly Phe Lys Gln Gly Leu Glu Gly
                405                 410                 415
Gly Gly Ser His His His His His His
            420                 425

<210> 6
<211> 426
<212> PRT
<213> Artificial Sequence

<220>
<223> Myc-RLuc8-Im7-6His

<400> 6
Met Gly Gly Glu Gln Lys Leu Ile Ser Glu Glu Asp Leu Gly Gly Ser
1               5                   10                  15
Ala Ser Lys Val Tyr Asp Pro Glu Gln Arg Lys Arg Met Ile Thr Gly
            20                  25                  30
Pro Gln Trp Trp Ala Arg Cys Lys Gln Met Asn Val Leu Asp Ser Phe
        35                  40                  45
Ile Asn Tyr Tyr Asp Ser Glu Lys His Ala Glu Asn Ala Val Ile Phe
    50                  55                  60
Leu His Gly Asn Ala Thr Ser Ser Tyr Leu Trp Arg His Val Val Pro
65                  70                  75                  80
His Ile Glu Pro Val Ala Arg Cys Ile Ile Pro Asp Leu Ile Gly Met
                85                  90                  95
Gly Lys Ser Gly Lys Ser Gly Asn Gly Ser Tyr Arg Leu Leu Asp His
            100                 105                 110
Tyr Lys Tyr Leu Thr Ala Trp Phe Glu Leu Leu Asn Leu Pro Lys Lys
        115                 120                 125
Ile Ile Phe Val Gly His Asp Trp Gly Ala Ala Leu Ala Phe His Tyr
    130                 135                 140
Ala Tyr Glu His Gln Asp Arg Ile Lys Ala Ile Val His Met Glu Ser
145                 150                 155                 160
Val Val Asp Val Ile Glu Ser Trp Asp Glu Trp Pro Asp Ile Glu Glu
                165                 170                 175
Asp Ile Ala Leu Ile Lys Ser Glu Glu Gly Glu Lys Met Val Leu Glu
            180                 185                 190
Asn Asn Phe Phe Val Glu Thr Val Leu Pro Ser Lys Ile Met Arg Lys
        195                 200                 205
Leu Glu Pro Glu Glu Phe Ala Ala Tyr Leu Glu Pro Phe Lys Glu Lys
    210                 215                 220
Gly Glu Val Arg Arg Pro Thr Leu Ser Trp Pro Arg Glu Ile Pro Leu
225                 230                 235                 240
Val Lys Gly Gly Lys Pro Asp Val Val Gln Ile Val Arg Asn Tyr Asn
                245                 250                 255
Ala Tyr Leu Arg Ala Ser Asp Asp Leu Pro Lys Leu Phe Ile Glu Ser
            260                 265                 270
Asp Pro Gly Phe Phe Ser Asn Ala Ile Val Glu Gly Ala Lys Lys Phe
        275                 280                 285
Pro Asn Thr Glu Phe Val Lys Val Lys Gly Leu His Phe Leu Gln Glu
    290                 295                 300
Asp Ala Pro Asp Glu Met Gly Lys Tyr Ile Lys Ser Phe Val Glu Arg
305                 310                 315                 320
Val Leu Lys Asn Glu Gln Lys Leu Glu Leu Lys Asn Ser Ile Ser Asp

                    325                     330                     335
Tyr Thr Glu Ala Glu Phe Val Gln Leu Leu Lys Glu Ile Glu Lys Glu
                340                     345                     350
Asn Val Ala Ala Thr Asp Asp Val Leu Asp Val Leu Leu Glu His Phe
            355                     360                     365
Val Lys Ile Thr Glu His Pro Asp Gly Thr Asp Leu Ile Tyr Tyr Pro
    370                     375                     380
Ser Asp Asn Arg Asp Asp Ser Pro Glu Gly Ile Val Lys Glu Ile Lys
385                     390                     395                     400
Glu Trp Arg Ala Ala Asn Gly Lys Pro Gly Phe Lys Gln Gly Leu Glu
                405                     410                     415
Gly Gly Gly Ser His His His His His His
            420                     425

<210> 7
<211> 424
<212> PRT
<213> Artificial Sequence

<220>
<223> Myc-RLuc8-Im8-6His

<400> 7
Met Gly Gly Glu Gln Lys Leu Ile Ser Glu Glu Asp Leu Gly Gly Ser
1               5                   10                  15
Ala Ser Lys Val Tyr Asp Pro Glu Gln Arg Lys Arg Met Ile Thr Gly
            20                  25                  30
Pro Gln Trp Trp Ala Arg Cys Lys Gln Met Asn Val Leu Asp Ser Phe
        35                  40                  45
Ile Asn Tyr Tyr Asp Ser Glu Lys His Ala Glu Asn Ala Val Ile Phe
    50                  55                  60
Leu His Gly Asn Ala Thr Ser Ser Tyr Leu Trp Arg His Val Val Pro
65                  70                  75                  80
His Ile Glu Pro Val Ala Arg Cys Ile Ile Pro Asp Leu Ile Gly Met
                85                  90                  95
Gly Lys Ser Gly Lys Ser Gly Asn Gly Ser Tyr Arg Leu Leu Asp His
            100                 105                 110
Tyr Lys Tyr Leu Thr Ala Trp Phe Glu Leu Leu Asn Leu Pro Lys Lys
        115                 120                 125
Ile Ile Phe Val Gly His Asp Trp Gly Ala Ala Leu Ala Phe His Tyr
    130                 135                 140
Ala Tyr Glu His Gln Asp Arg Ile Lys Ala Ile Val His Met Glu Ser
145                 150                 155                 160
Val Val Asp Val Ile Glu Ser Trp Asp Glu Trp Pro Asp Ile Glu Glu
                165                 170                 175
Asp Ile Ala Leu Ile Lys Ser Glu Glu Gly Glu Lys Met Val Leu Glu
            180                 185                 190
Asn Asn Phe Phe Val Glu Thr Val Leu Pro Ser Lys Ile Met Arg Lys
        195                 200                 205
Leu Glu Pro Glu Glu Phe Ala Ala Tyr Leu Glu Pro Phe Lys Glu Lys
    210                 215                 220
Gly Glu Val Arg Arg Pro Thr Leu Ser Trp Pro Arg Glu Ile Pro Leu
225                 230                 235                 240
Val Lys Gly Gly Lys Pro Asp Val Val Gln Ile Val Arg Asn Tyr Asn
                245                 250                 255
Ala Tyr Leu Arg Ala Ser Asp Asp Leu Pro Lys Leu Phe Ile Glu Ser
            260                 265                 270
Asp Pro Gly Phe Phe Ser Asn Ala Ile Val Glu Gly Ala Lys Lys Phe
        275                 280                 285
Pro Asn Thr Glu Phe Val Lys Val Lys Gly Leu His Phe Leu Gln Glu
    290                 295                 300
Asp Ala Pro Asp Glu Met Gly Lys Tyr Ile Lys Ser Phe Val Glu Arg

305                 310                 315                 320
Val Leu Lys Asn Glu Gln Lys Leu Glu Leu Lys Asn Ser Ile Ser Asp
                325                 330                 335
Tyr Thr Glu Thr Glu Phe Lys Lys Ile Ile Glu Asp Ile Ile Asn Cys
            340                 345                 350
Glu Gly Asp Glu Lys Lys Gln Asp Asp Asn Leu Glu His Phe Ile Ser
        355                 360                 365
Val Thr Glu His Pro Ser Gly Ser Asp Leu Ile Tyr Tyr Pro Glu Gly
    370                 375                 380
Asn Asn Asp Gly Ser Pro Glu Ala Val Ile Lys Glu Ile Lys Glu Trp
385                 390                 395                 400
Arg Ala Ala Asn Gly Lys Ser Gly Phe Lys Gln Gly Leu Glu Gly Gly
                405                 410                 415
Gly Ser His His His His His His
            420

<210> 8
<211> 419
<212> PRT
<213> Artificial Sequence

<220>
<223> Myc-RLuc8-Im9-6His

<400> 8
Met Gly Gly Glu Gln Lys Leu Ile Ser Glu Glu Asp Leu Gly Gly Ser
1               5                   10                  15
Ala Ser Lys Val Tyr Asp Pro Glu Gln Arg Lys Arg Met Ile Thr Gly
            20                  25                  30
Pro Gln Trp Trp Ala Arg Cys Lys Gln Met Asn Val Leu Asp Ser Phe
        35                  40                  45
Ile Asn Tyr Tyr Asp Ser Glu Lys His Ala Glu Asn Ala Val Ile Phe
    50                  55                  60
Leu His Gly Asn Ala Thr Ser Ser Tyr Leu Trp Arg His Val Val Pro
65                  70                  75                  80
His Ile Glu Pro Val Ala Arg Cys Ile Ile Pro Asp Leu Ile Gly Met
                85                  90                  95
Gly Lys Ser Gly Lys Ser Gly Asn Gly Ser Tyr Arg Leu Leu Asp His
            100                 105                 110
Tyr Lys Tyr Leu Thr Ala Trp Phe Glu Leu Leu Asn Leu Pro Lys Lys
        115                 120                 125
Ile Ile Phe Val Gly His Asp Trp Gly Ala Ala Leu Ala Phe His Tyr
    130                 135                 140
Ala Tyr Glu His Gln Asp Arg Ile Lys Ala Ile Val His Met Glu Ser
145                 150                 155                 160
Val Val Asp Val Ile Glu Ser Trp Asp Glu Trp Pro Asp Ile Glu Glu
                165                 170                 175
Asp Ile Ala Leu Ile Lys Ser Glu Glu Gly Glu Lys Met Val Leu Glu
            180                 185                 190
Asn Asn Phe Phe Val Glu Thr Val Leu Pro Ser Lys Ile Met Arg Lys
        195                 200                 205
Leu Glu Pro Glu Glu Phe Ala Ala Tyr Leu Glu Pro Phe Lys Glu Lys
    210                 215                 220
Gly Glu Val Arg Arg Pro Thr Leu Ser Trp Pro Arg Glu Ile Pro Leu
225                 230                 235                 240
Val Lys Gly Gly Lys Pro Asp Val Val Gln Ile Val Arg Asn Tyr Asn
                245                 250                 255
Ala Tyr Leu Arg Ala Ser Asp Asp Leu Pro Lys Leu Phe Ile Glu Ser
            260                 265                 270
Asp Pro Gly Phe Phe Ser Asn Ala Ile Val Glu Gly Ala Lys Lys Phe
        275                 280                 285
Pro Asn Thr Glu Phe Val Lys Val Lys Gly Leu His Phe Leu Gln Glu

290                 295                 300
Asp Ala Pro Asp Glu Met Gly Lys Tyr Ile Lys Ser Phe Val Glu Arg
305                 310                 315                 320
Val Leu Lys Asn Glu Gln Lys Leu Glu Leu Lys His Ser Ile Ser Asp
                325                 330                 335
Tyr Thr Glu Ala Glu Phe Leu Gln Leu Val Thr Thr Ile Cys Asn Ala
            340                 345                 350
Asp Thr Ser Ser Glu Glu Glu Leu Val Lys Leu Val Thr His Phe Glu
        355                 360                 365
Glu Met Thr Glu His Pro Ser Gly Ser Asp Leu Ile Tyr Tyr Pro Lys
    370                 375                 380
Glu Gly Asp Asp Asp Ser Pro Ser Gly Ile Val Asn Thr Val Lys Gln
385                 390                 395                 400
Trp Arg Ala Ala Asn Gly Lys Leu Glu Gly Gly Gly Ser His His His
                405                 410                 415
His His His

<210> 9
<211> 409
<212> PRT
<213> Artificial Sequence

<220>
<223> Flag-mCherry-ColAP41-Cys-6His

<400> 9
Met Gly Gly Asp Tyr Lys Asp Asp Asp Asp Lys Gly Gly Ser Val Ser
1               5                   10                  15
Lys Gly Glu Glu Asp Asn Met Ala Ile Ile Lys Glu Phe Met Arg Phe
            20                  25                  30
Lys Val His Met Glu Gly Ser Val Asn Gly His Glu Phe Glu Ile Glu
        35                  40                  45
Gly Glu Gly Glu Gly Arg Pro Tyr Glu Gly Thr Gln Thr Ala Lys Leu
    50                  55                  60
Lys Val Thr Lys Gly Gly Pro Leu Pro Phe Ala Trp Asp Ile Leu Ser
65                  70                  75                  80
Pro Gln Phe Met Tyr Gly Ser Lys Ala Tyr Val Lys His Pro Ala Asp
                85                  90                  95
Ile Pro Asp Tyr Leu Lys Leu Ser Phe Pro Glu Gly Phe Lys Trp Glu
            100                 105                 110
Arg Val Met Asn Phe Glu Asp Gly Gly Val Val Thr Val Thr Gln Asp
        115                 120                 125
Ser Ser Leu Gln Asp Gly Glu Phe Ile Tyr Lys Val Lys Leu Arg Gly
    130                 135                 140
Thr Asn Phe Pro Ser Asp Gly Pro Val Met Gln Lys Lys Thr Met Gly
145                 150                 155                 160
Trp Glu Ala Ser Ser Glu Arg Met Tyr Pro Glu Asp Gly Ala Leu Lys
                165                 170                 175
Gly Glu Ile Lys Gln Arg Leu Lys Leu Lys Asp Gly Gly His Tyr Asp
            180                 185                 190
Ala Glu Val Lys Thr Thr Tyr Lys Ala Lys Lys Pro Val Gln Leu Pro
        195                 200                 205
Gly Ala Tyr Asn Val Asn Ile Lys Leu Asp Ile Thr Ser His Asn Glu
    210                 215                 220
Asp Tyr Thr Ile Val Glu Gln Tyr Glu Arg Ala Glu Gly Arg His Ser
225                 230                 235                 240
Thr Gly Gly Met Asp Glu Leu Tyr Lys Leu Pro Gly Gly Ser Asp Glu
                245                 250                 255
Pro Gly Val Ala Thr Gly Asn Gly Gln Pro Val Thr Gly Asn Trp Leu
            260                 265                 270
Ala Gly Ala Ser Gln Gly Asp Gly Val Pro Ile Pro Ser Gln Ile Ala

275                 280                 285
Asp Gln Leu Arg Gly Lys Glu Phe Lys Ser Trp Arg Asp Phe Arg Glu
    290                 295                 300
Gln Phe Trp Met Ala Val Ser Lys Asp Pro Ser Ala Leu Glu Asn Leu
305                 310                 315                 320
Ser Pro Ser Asn Arg Tyr Phe Val Ser Gln Gly Leu Ala Pro Tyr Ala
                325                 330                 335
Val Pro Glu Glu His Leu Gly Ser Lys Glu Lys Phe Glu Ile His Ala
            340                 345                 350
Val Val Pro Leu Glu Ser Gly Gly Ala Leu Tyr Asn Ile Asp Asn Leu
        355                 360                 365
Val Ile Val Thr Pro Lys Arg His Ser Glu Ile His Lys Glu Leu Lys
    370                 375                 380
Leu Lys Arg Lys Glu Lys Gly Gly Ser Gly Gly Ser Cys Leu Glu Gly
385                 390                 395                 400
Gly Gly Ser His His His His His His
                405

<210> 10
<211> 407
<212> PRT
<213> Artificial Sequence

<220>
<223> Flag-mCherry-ColSyr-Cys-6His

<400> 10
Met Gly Gly Asp Tyr Lys Asp Asp Asp Asp Lys Gly Gly Ser Val Ser
1               5                   10                  15
Lys Gly Glu Glu Asp Asn Met Ala Ile Ile Lys Glu Phe Met Arg Phe
            20                  25                  30
Lys Val His Met Glu Gly Ser Val Asn Gly His Glu Phe Glu Ile Glu
        35                  40                  45
Gly Glu Gly Glu Gly Arg Pro Tyr Glu Gly Thr Gln Thr Ala Lys Leu
    50                  55                  60
Lys Val Thr Lys Gly Gly Pro Leu Pro Phe Ala Trp Asp Ile Leu Ser
65                  70                  75                  80
Pro Gln Phe Met Tyr Gly Ser Lys Ala Tyr Val Lys His Pro Ala Asp
                85                  90                  95
Ile Pro Asp Tyr Leu Lys Leu Ser Phe Pro Glu Gly Phe Lys Trp Glu
            100                 105                 110
Arg Val Met Asn Phe Glu Asp Gly Gly Val Val Thr Val Thr Gln Asp
        115                 120                 125
Ser Ser Leu Gln Asp Gly Glu Phe Ile Tyr Lys Val Lys Leu Arg Gly
    130                 135                 140
Thr Asn Phe Pro Ser Asp Gly Pro Val Met Gln Lys Lys Thr Met Gly
145                 150                 155                 160
Trp Glu Ala Ser Ser Glu Arg Met Tyr Pro Glu Asp Gly Ala Leu Lys
                165                 170                 175
Gly Glu Ile Lys Gln Arg Leu Lys Leu Lys Asp Gly Gly His Tyr Asp
            180                 185                 190
Ala Glu Val Lys Thr Thr Tyr Lys Ala Lys Lys Pro Val Gln Leu Pro
        195                 200                 205
Gly Ala Tyr Asn Val Asn Ile Lys Leu Asp Ile Thr Ser His Asn Glu
    210                 215                 220
Asp Tyr Thr Ile Val Glu Gln Tyr Glu Arg Ala Glu Gly Arg His Ser
225                 230                 235                 240
Thr Gly Gly Met Asp Glu Leu Tyr Lys Leu Pro Gly Gly Ser Arg Ser
                245                 250                 255
Ile Pro Gly Val Ala Ser Gly Tyr Gly Glu Ala Val Asn Gly Val Trp
            260                 265                 270
Leu Gly Asp Lys Thr Arg Ala Glu Gly Ala Ser Ile Pro Ala His Ile

275                 280                 285
Ala Asp Gln Leu Arg Gly Arg Arg Phe Gly Asn Phe Asp Ser Leu Arg
    290                 295                 300
Lys Ala Thr Trp Ile Ala Val Ala Asn Asp Pro Glu Leu Val Lys Gln
305                 310                 315                 320
Phe Thr Gln His Asn Leu Glu Ile Met Arg Asp Gly Gly Ala Pro Tyr
                325                 330                 335
Pro Arg Leu Val Asp Gln Ala Gly Gly Arg Thr Lys Phe Glu Ile His
            340                 345                 350
Ala Lys Lys His Ile Ala Asn Gly Gly Ala Val Tyr Asp Ile Asp Asn
        355                 360                 365
Leu Val Ile Met Thr Pro Arg Gln His Ile Asp His His Arg Ser His
    370                 375                 380
Glu Asn Asp Leu Gly Gly Ser Gly Gly Ser Cys Leu Glu Gly Gly Gly
385                 390                 395                 400
Ser His His His His His His
                405

<210> 11
<211> 402
<212> PRT
<213> Artificial Sequence

<220>
<223> Flag-mCherry-ColErw-Cys-6His

<400> 11
Met Gly Gly Asp Tyr Lys Asp Asp Asp Asp Lys Gly Gly Ser Val Ser
1               5                   10                  15
Lys Gly Glu Glu Asp Asn Met Ala Ile Ile Lys Glu Phe Met Arg Phe
            20                  25                  30
Lys Val His Met Glu Gly Ser Val Asn Gly His Glu Phe Glu Ile Glu
        35                  40                  45
Gly Glu Gly Glu Gly Arg Pro Tyr Glu Gly Thr Gln Thr Ala Lys Leu
    50                  55                  60
Lys Val Thr Lys Gly Gly Pro Leu Pro Phe Ala Trp Asp Ile Leu Ser
65                  70                  75                  80
Pro Gln Phe Met Tyr Gly Ser Lys Ala Tyr Val Lys His Pro Ala Asp
                85                  90                  95
Ile Pro Asp Tyr Leu Lys Leu Ser Phe Pro Glu Gly Phe Lys Trp Glu
            100                 105                 110
Arg Val Met Asn Phe Glu Asp Gly Gly Val Val Thr Val Thr Gln Asp
        115                 120                 125
Ser Ser Leu Gln Asp Gly Glu Phe Ile Tyr Lys Val Lys Leu Arg Gly
    130                 135                 140
Thr Asn Phe Pro Ser Asp Gly Pro Val Met Gln Lys Lys Thr Met Gly
145                 150                 155                 160
Trp Glu Ala Ser Ser Glu Arg Met Tyr Pro Glu Asp Gly Ala Leu Lys
                165                 170                 175
Gly Glu Ile Lys Gln Arg Leu Lys Leu Lys Asp Gly Gly His Tyr Asp
            180                 185                 190
Ala Glu Val Lys Thr Thr Tyr Lys Ala Lys Lys Pro Val Gln Leu Pro
        195                 200                 205
Gly Ala Tyr Asn Val Asn Ile Lys Leu Asp Ile Thr Ser His Asn Glu
    210                 215                 220
Asp Tyr Thr Ile Val Glu Gln Tyr Glu Arg Ala Glu Gly Arg His Ser
225                 230                 235                 240
Thr Gly Gly Met Asp Glu Leu Tyr Lys Leu Pro Gly Gly Ser Arg Asp
                245                 250                 255
Lys Pro Gly Thr Val Thr Gly Lys Gly Glu Val Leu Ser Ser Glu Gly
            260                 265                 270
Lys Trp Leu Glu Ser Ala Ser Ser Gly Leu Gly Ala Pro Val Pro Ala

275                 280                 285
Gln Val Ala Asp Lys Leu Arg Gly Gln Lys Phe Glu Arg Phe Asp Asp
    290                 295                 300
Phe Arg Glu Ala Phe Trp Leu Ala Val Ala Glu Cys Pro Glu Leu Met
305                 310                 315                 320
Val Gln Phe Asn Arg Ser Asn Gln Thr Ile Ile Arg Ala Gly Thr Ser
                325                 330                 335
Pro Phe Ala Ile Pro Glu Glu Gln Val Gly Lys Arg Lys Arg Phe Glu
            340                 345                 350
Ile His Ala Val Lys Asn Ile Gln His Arg Gly Glu Val Tyr Asn Ile
        355                 360                 365
Asp Asn Leu Arg Val Asn Thr Pro Lys Asn His Ile Gly Leu His Gly
    370                 375                 380
Gly Ser Gly Gly Ser Cys Leu Glu Gly Gly Gly Ser His His His His
385                 390                 395                 400
His His

<210> 12
<211> 407
<212> PRT
<213> Artificial Sequence

<220>
<223> Flag-mCherry-ColLeaf-Cys-6His

<400> 12
Met Gly Gly Asp Tyr Lys Asp Asp Asp Asp Lys Gly Gly Ser Val Ser
1               5                   10                  15
Lys Gly Glu Glu Asp Asn Met Ala Ile Ile Lys Glu Phe Met Arg Phe
            20                  25                  30
Lys Val His Met Glu Gly Ser Val Asn Gly His Glu Phe Glu Ile Glu
        35                  40                  45
Gly Glu Gly Glu Gly Arg Pro Tyr Glu Gly Thr Gln Thr Ala Lys Leu
    50                  55                  60
Lys Val Thr Lys Gly Gly Pro Leu Pro Phe Ala Trp Asp Ile Leu Ser
65                  70                  75                  80
Pro Gln Phe Met Tyr Gly Ser Lys Ala Tyr Val Lys His Pro Ala Asp
                85                  90                  95
Ile Pro Asp Tyr Leu Lys Leu Ser Phe Pro Glu Gly Phe Lys Trp Glu
            100                 105                 110
Arg Val Met Asn Phe Glu Asp Gly Gly Val Val Thr Val Thr Gln Asp
        115                 120                 125
Ser Ser Leu Gln Asp Gly Glu Phe Ile Tyr Lys Val Lys Leu Arg Gly
    130                 135                 140
Thr Asn Phe Pro Ser Asp Gly Pro Val Met Gln Lys Lys Thr Met Gly
145                 150                 155                 160
Trp Glu Ala Ser Ser Glu Arg Met Tyr Pro Glu Asp Gly Ala Leu Lys
                165                 170                 175
Gly Glu Ile Lys Gln Arg Leu Lys Leu Lys Asp Gly Gly His Tyr Asp
            180                 185                 190
Ala Glu Val Lys Thr Thr Tyr Lys Ala Lys Lys Pro Val Gln Leu Pro
        195                 200                 205
Gly Ala Tyr Asn Val Asn Ile Lys Leu Asp Ile Thr Ser His Asn Glu
    210                 215                 220
Asp Tyr Thr Ile Val Glu Gln Tyr Glu Arg Ala Glu Gly Arg His Ser
225                 230                 235                 240
Thr Gly Gly Met Asp Glu Leu Tyr Lys Leu Gly Gly Gly Gly Ser Gly
                245                 250                 255
Gly Gly Gly Ser Ser Leu Arg His Glu Pro Gly Val Val Thr Gly Gln
            260                 265                 270
Gly Gln Asp Val Thr Gly Ile Trp Leu Glu Asn Ala Gly Arg Glu Leu

275                 280                 285
Gly Ala Pro Ile Pro Ser Gln Ile Ala Asp Gln Leu Arg Gly Lys Gln
    290                 295                 300
Phe Ser Ser Phe Asp Ser Phe Arg Lys Arg Phe Trp Lys Thr Val Gly
305                 310                 315                 320
Thr Asp Ala Thr Leu Ser Asn Gln Phe Ile Ser Ala Asn Arg Lys Arg
                325                 330                 335
Met Leu Ala Gly Lys Ala Ala Lys Leu Arg Glu Lys Asp Arg Val Gly
            340                 345                 350
Gly Arg Thr Thr Tyr Glu Leu His Ala Val Glu Lys Ile Ser Glu Gly
        355                 360                 365
Gly Glu Val Tyr Asn Val Asp Asn Leu Arg Val Val Thr Ala Lys Arg
    370                 375                 380
His Ile Glu Ile His Lys Thr Glu Gly Lys Cys Leu Glu Gly Gly Gly
385                 390                 395                 400
Ser His His His His His His
                405

<210> 13
<211> 406
<212> PRT
<213> Artificial Sequence

<220>
<223> Flag-mCherry-ColKhan-Cys-6His

<400> 13
Met Gly Gly Asp Tyr Lys Asp Asp Asp Asp Lys Gly Gly Ser Val Ser
1               5                   10                  15
Lys Gly Glu Glu Asp Asn Met Ala Ile Ile Lys Glu Phe Met Arg Phe
            20                  25                  30
Lys Val His Met Glu Gly Ser Val Asn Gly His Glu Phe Glu Ile Glu
        35                  40                  45
Gly Glu Gly Glu Gly Arg Pro Tyr Glu Gly Thr Gln Thr Ala Lys Leu
    50                  55                  60
Lys Val Thr Lys Gly Gly Pro Leu Pro Phe Ala Trp Asp Ile Leu Ser
65                  70                  75                  80
Pro Gln Phe Met Tyr Gly Ser Lys Ala Tyr Val Lys His Pro Ala Asp
                85                  90                  95
Ile Pro Asp Tyr Leu Lys Leu Ser Phe Pro Glu Gly Phe Lys Trp Glu
            100                 105                 110
Arg Val Met Asn Phe Glu Asp Gly Gly Val Val Thr Val Thr Gln Asp
        115                 120                 125
Ser Ser Leu Gln Asp Gly Glu Phe Ile Tyr Lys Val Lys Leu Arg Gly
    130                 135                 140
Thr Asn Phe Pro Ser Asp Gly Pro Val Met Gln Lys Lys Thr Met Gly
145                 150                 155                 160
Trp Glu Ala Ser Ser Glu Arg Met Tyr Pro Glu Asp Gly Ala Leu Lys
                165                 170                 175
Gly Glu Ile Lys Gln Arg Leu Lys Leu Lys Asp Gly Gly His Tyr Asp
            180                 185                 190
Ala Glu Val Lys Thr Thr Tyr Lys Ala Lys Lys Pro Val Gln Leu Pro
        195                 200                 205
Gly Ala Tyr Asn Val Asn Ile Lys Leu Asp Ile Thr Ser His Asn Glu
    210                 215                 220
Asp Tyr Thr Ile Val Glu Gln Tyr Glu Arg Ala Glu Gly Arg His Ser
225                 230                 235                 240
Thr Gly Gly Met Asp Glu Leu Tyr Lys Leu Gly Gly Gly Gly Ser Gly
                245                 250                 255
Gly Gly Gly Ser Asn Thr Pro Arg Asn Gln Pro Gly Thr Val Thr Gly
            260                 265                 270
Gln Gly Gln Lys Val Glu Gly Asn Trp Leu Ser Arg Ala Gly Gln Asp

275                 280                 285
Met Gly Ala Pro Ile Pro Ser Gln Ile Ala Asp Lys Leu Arg Gly Arg
    290                 295                 300
Thr Phe Asn Asn Phe Asp Asp Phe Arg Lys Ala Phe Trp Lys Glu Val
305                 310                 315                 320
Gly Asn Asp Pro Glu Leu Ala Lys Asp Leu Ser Asp Val Asn Lys Lys
                325                 330                 335
Arg Ile Lys Glu Leu Gly Tyr Ala Pro Phe Ala Ile Pro Ile Glu Gln
            340                 345                 350
Val Gly Gly Lys Lys Lys Phe Asp Ile His Ala Val Lys Pro Ile Lys
        355                 360                 365
Asp Gly Gly Ala Val Tyr Asp Leu Asp Asn Leu Arg Val Val Thr Pro
    370                 375                 380
Lys Lys His Ile Glu Leu His Ser Asn Cys Leu Glu Gly Gly Gly Ser
385                 390                 395                 400
His His His His His His
                405

<210> 14
<211> 456
<212> PRT
<213> Artificial Sequence

<220>
<223> Myc-RLuc8-ImAP41-Avitag-6His:

<400> 14
Met Gly Gly Glu Gln Lys Leu Ile Ser Glu Glu Asp Leu Gly Gly Ser
1               5                   10                  15
Ala Ser Lys Val Tyr Asp Pro Glu Gln Arg Lys Arg Met Ile Thr Gly
            20                  25                  30
Pro Gln Trp Trp Ala Arg Cys Lys Gln Met Asn Val Leu Asp Ser Phe
        35                  40                  45
Ile Asn Tyr Tyr Asp Ser Glu Lys His Ala Glu Asn Ala Val Ile Phe
    50                  55                  60
Leu His Gly Asn Ala Thr Ser Ser Tyr Leu Trp Arg His Val Val Pro
65                  70                  75                  80
His Ile Glu Pro Val Ala Arg Cys Ile Ile Pro Asp Leu Ile Gly Met
                85                  90                  95
Gly Lys Ser Gly Lys Ser Gly Asn Gly Ser Tyr Arg Leu Leu Asp His
            100                 105                 110
Tyr Lys Tyr Leu Thr Ala Trp Phe Glu Leu Leu Asn Leu Pro Lys Lys
        115                 120                 125
Ile Ile Phe Val Gly His Asp Trp Gly Ala Ala Leu Ala Phe His Tyr
    130                 135                 140
Ala Tyr Glu His Gln Asp Arg Ile Lys Ala Ile Val His Met Glu Ser
145                 150                 155                 160
Val Val Asp Val Ile Glu Ser Trp Asp Glu Trp Pro Asp Ile Glu Glu
                165                 170                 175
Asp Ile Ala Leu Ile Lys Ser Glu Glu Gly Glu Lys Met Val Leu Glu
            180                 185                 190
Asn Asn Phe Phe Val Glu Thr Val Leu Pro Ser Lys Ile Met Arg Lys
        195                 200                 205
Leu Glu Pro Glu Glu Phe Ala Ala Tyr Leu Glu Pro Phe Lys Glu Lys
    210                 215                 220
Gly Glu Val Arg Arg Pro Thr Leu Ser Trp Pro Arg Glu Ile Pro Leu
225                 230                 235                 240
Val Lys Gly Gly Lys Pro Asp Val Val Gln Ile Val Arg Asn Tyr Asn
                245                 250                 255
Ala Tyr Leu Arg Ala Ser Asp Asp Leu Pro Lys Leu Phe Ile Glu Ser
            260                 265                 270
Asp Pro Gly Phe Phe Ser Asn Ala Ile Val Glu Gly Ala Lys Lys Phe

275                 280                 285
Pro Asn Thr Glu Phe Val Lys Val Lys Gly Leu His Phe Leu Gln Glu
    290                 295                 300
Asp Ala Pro Asp Glu Met Gly Lys Tyr Ile Lys Ser Phe Val Glu Arg
305                 310                 315                 320
Val Leu Lys Asn Glu Gln Lys Leu Pro Gly Gly Ser Asp Ile Lys Asn
                325                 330                 335
Asn Leu Ser Asp Tyr Thr Glu Ser Glu Phe Leu Glu Ile Ile Glu Glu
            340                 345                 350
Phe Phe Lys Asn Lys Ser Gly Leu Lys Gly Ser Glu Leu Glu Lys Arg
        355                 360                 365
Met Asp Lys Leu Val Lys His Phe Glu Glu Val Thr Ser His Pro Arg
    370                 375                 380
Lys Ser Gly Val Ile Phe His Pro Lys Pro Gly Phe Glu Thr Pro Glu
385                 390                 395                 400
Gly Ile Val Lys Glu Val Lys Glu Trp Arg Ala Ala Asn Gly Leu Pro
                405                 410                 415
Gly Phe Lys Ala Gly Leu Glu Gly Gly Ser Gly Gly Ser Gly Leu Asn
            420                 425                 430
Asp Ile Phe Glu Ala Gln Lys Ile Glu Trp His Glu Leu Glu Gly Gly
        435                 440                 445
Gly Ser His His His His His His
    450                 455

<210> 15
<211> 454
<212> PRT
<213> Artificial Sequence

<220>
<223> Myc-RLuc8-ImSyr-Avitag-6His

<400> 15
Met Gly Gly Glu Gln Lys Leu Ile Ser Glu Glu Asp Leu Gly Gly Ser
1               5                   10                  15
Ala Ser Lys Val Tyr Asp Pro Glu Gln Arg Lys Arg Met Ile Thr Gly
            20                  25                  30
Pro Gln Trp Trp Ala Arg Cys Lys Gln Met Asn Val Leu Asp Ser Phe
        35                  40                  45
Ile Asn Tyr Tyr Asp Ser Glu Lys His Ala Glu Asn Ala Val Ile Phe
    50                  55                  60
Leu His Gly Asn Ala Thr Ser Ser Tyr Leu Trp Arg His Val Val Pro
65                  70                  75                  80
His Ile Glu Pro Val Ala Arg Cys Ile Ile Pro Asp Leu Ile Gly Met
                85                  90                  95
Gly Lys Ser Gly Lys Ser Gly Asn Gly Ser Tyr Arg Leu Leu Asp His
            100                 105                 110
Tyr Lys Tyr Leu Thr Ala Trp Phe Glu Leu Leu Asn Leu Pro Lys Lys
        115                 120                 125
Ile Ile Phe Val Gly His Asp Trp Gly Ala Ala Leu Ala Phe His Tyr
    130                 135                 140
Ala Tyr Glu His Gln Asp Arg Ile Lys Ala Ile Val His Met Glu Ser
145                 150                 155                 160
Val Val Asp Val Ile Glu Ser Trp Asp Glu Trp Pro Asp Ile Glu Glu
                165                 170                 175
Asp Ile Ala Leu Ile Lys Ser Glu Glu Gly Glu Lys Met Val Leu Glu
            180                 185                 190
Asn Asn Phe Phe Val Glu Thr Val Leu Pro Ser Lys Ile Met Arg Lys
        195                 200                 205
Leu Glu Pro Glu Glu Phe Ala Ala Tyr Leu Glu Pro Phe Lys Glu Lys
    210                 215                 220
Gly Glu Val Arg Arg Pro Thr Leu Ser Trp Pro Arg Glu Ile Pro Leu

225                 230                 235                 240
Val Lys Gly Gly Lys Pro Asp Val Val Gln Ile Val Arg Asn Tyr Asn
                245                 250                 255
Ala Tyr Leu Arg Ala Ser Asp Asp Leu Pro Lys Leu Phe Ile Glu Ser
            260                 265                 270
Asp Pro Gly Phe Phe Ser Asn Ala Ile Val Glu Gly Ala Lys Lys Phe
        275                 280                 285
Pro Asn Thr Glu Phe Val Lys Val Lys Gly Leu His Phe Leu Gln Glu
    290                 295                 300
Asp Ala Pro Asp Glu Met Gly Lys Tyr Ile Lys Ser Phe Val Glu Arg
305                 310                 315                 320
Val Leu Lys Asn Glu Gln Lys Leu Pro Gly Gly Ser Ile Phe Lys Glu
                325                 330                 335
Lys Ile Glu Asp Tyr Thr Glu Glu Glu Phe Leu Glu Phe Leu Lys Gly
            340                 345                 350
Leu Ser Ser Glu Tyr Ser Gln Leu His Gly Asp Glu Phe Ile Lys His
        355                 360                 365
Met Asp Arg Ser Val Glu His Phe Val Lys Ile Thr Glu His Pro Ala
    370                 375                 380
Gln Thr Asp Val Ile Phe Tyr Pro Glu Glu Gly Gln Glu Asp Thr Pro
385                 390                 395                 400
Glu Gly Ile Leu Lys Val Ile Lys Glu Trp Arg Ala Lys Asn Gly Lys
                405                 410                 415
Pro Gly Phe Lys Ser Gly Gly Ser Gly Gly Ser Gly Leu Asn Asp Ile
            420                 425                 430
Phe Glu Ala Gln Lys Ile Glu Trp His Glu Leu Glu Gly Gly Gly Ser
        435                 440                 445
His His His His His His
    450

<210> 16
<211> 456
<212> PRT
<213> Artificial Sequence

<220>
<223> Myc-RLuc8-ImErw-Avitag-6His

<400> 16
Met Gly Gly Glu Gln Lys Leu Ile Ser Glu Glu Asp Leu Gly Gly Ser
1               5                   10                  15
Ala Ser Lys Val Tyr Asp Pro Glu Gln Arg Lys Arg Met Ile Thr Gly
            20                  25                  30
Pro Gln Trp Trp Ala Arg Cys Lys Gln Met Asn Val Leu Asp Ser Phe
        35                  40                  45
Ile Asn Tyr Tyr Asp Ser Glu Lys His Ala Glu Asn Ala Val Ile Phe
    50                  55                  60
Leu His Gly Asn Ala Thr Ser Ser Tyr Leu Trp Arg His Val Val Pro
65                  70                  75                  80
His Ile Glu Pro Val Ala Arg Cys Ile Ile Pro Asp Leu Ile Gly Met
                85                  90                  95
Gly Lys Ser Gly Lys Ser Gly Asn Gly Ser Tyr Arg Leu Leu Asp His
            100                 105                 110
Tyr Lys Tyr Leu Thr Ala Trp Phe Glu Leu Leu Asn Leu Pro Lys Lys
        115                 120                 125
Ile Ile Phe Val Gly His Asp Trp Gly Ala Ala Leu Ala Phe His Tyr
    130                 135                 140
Ala Tyr Glu His Gln Asp Arg Ile Lys Ala Ile Val His Met Glu Ser
145                 150                 155                 160
Val Val Asp Val Ile Glu Ser Trp Asp Glu Trp Pro Asp Ile Glu Glu
                165                 170                 175
Asp Ile Ala Leu Ile Lys Ser Glu Glu Gly Glu Lys Met Val Leu Glu

180                 185                 190
Asn Asn Phe Phe Val Glu Thr Val Leu Pro Ser Lys Ile Met Arg Lys
        195                 200                 205
Leu Glu Pro Glu Glu Phe Ala Ala Tyr Leu Glu Pro Phe Lys Glu Lys
    210                 215                 220
Gly Glu Val Arg Arg Pro Thr Leu Ser Trp Pro Arg Glu Ile Pro Leu
225                 230                 235                 240
Val Lys Gly Gly Lys Pro Asp Val Val Gln Ile Val Arg Asn Tyr Asn
                245                 250                 255
Ala Tyr Leu Arg Ala Ser Asp Asp Leu Pro Lys Leu Phe Ile Glu Ser
            260                 265                 270
Asp Pro Gly Phe Phe Ser Asn Ala Ile Val Glu Gly Ala Lys Lys Phe
        275                 280                 285
Pro Asn Thr Glu Phe Val Lys Val Lys Gly Leu His Phe Leu Gln Glu
    290                 295                 300
Asp Ala Pro Asp Glu Met Gly Lys Tyr Ile Lys Ser Phe Val Glu Arg
305                 310                 315                 320
Val Leu Lys Asn Glu Gln Lys Leu Pro Gly Gly Ser Asn Leu Lys Glu
                325                 330                 335
Lys Leu Glu Asp Tyr Thr Glu Ala Glu Phe Ile Ser Tyr Leu Lys Glu
            340                 345                 350
Phe Phe Asp Asn Pro Met Gly Leu Arg Gly Lys Glu Leu Glu Thr His
        355                 360                 365
Leu Asp Ser Leu Val Glu His Phe Asp Lys Ile Val Phe His Pro Glu
    370                 375                 380
Gly Asn Gly Leu Ile Phe Tyr Pro Pro Asp Glu Arg Asp Asp Ser Pro
385                 390                 395                 400
Glu Gly Val Leu Asn Glu Ile Lys Arg Trp Arg Lys Ser Gln Gly Leu
                405                 410                 415
Pro Leu Phe Lys Asp Ser Lys Gly Gly Ser Gly Gly Ser Gly Leu Asn
            420                 425                 430
Asp Ile Phe Glu Ala Gln Lys Ile Glu Trp His Glu Leu Glu Gly Gly
        435                 440                 445
Gly Ser His His His His His His
    450                 455

<210> 17
<211> 442
<212> PRT
<213> Artificial Sequence

<220>
<223> Avitag-RLuc8-ImLeaf-6His

<400> 17
Met Gly Leu Asn Asp Ile Phe Glu Ala Gln Lys Ile Glu Trp His Glu
1               5                   10                  15
Gly Gly Ser Gly Gly Ser Gly Gly Ser Ala Ser Lys Val Tyr Asp Pro
            20                  25                  30
Glu Gln Arg Lys Arg Met Ile Thr Gly Pro Gln Trp Trp Ala Arg Cys
        35                  40                  45
Lys Gln Met Asn Val Leu Asp Ser Phe Ile Asn Tyr Tyr Asp Ser Glu
    50                  55                  60
Lys His Ala Glu Asn Ala Val Ile Phe Leu His Gly Asn Ala Thr Ser
65                  70                  75                  80
Ser Tyr Leu Trp Arg His Val Val Pro His Ile Glu Pro Val Ala Arg
                85                  90                  95
Cys Ile Ile Pro Asp Leu Ile Gly Met Gly Lys Ser Gly Lys Ser Gly
            100                 105                 110
Asn Gly Ser Tyr Arg Leu Leu Asp His Tyr Lys Tyr Leu Thr Ala Trp
        115                 120                 125
Phe Glu Leu Leu Asn Leu Pro Lys Lys Ile Ile Phe Val Gly His Asp

130                 135                 140
Trp Gly Ala Ala Leu Ala Phe His Tyr Ala Tyr Glu His Gln Asp Arg
145                 150                 155                 160
Ile Lys Ala Ile Val His Met Glu Ser Val Val Asp Val Ile Glu Ser
                165                 170                 175
Trp Asp Glu Trp Pro Asp Ile Glu Glu Asp Ile Ala Leu Ile Lys Ser
            180                 185                 190
Glu Glu Gly Glu Lys Met Val Leu Glu Asn Asn Phe Phe Val Glu Thr
        195                 200                 205
Val Leu Pro Ser Lys Ile Met Arg Lys Leu Glu Pro Glu Glu Phe Ala
    210                 215                 220
Ala Tyr Leu Glu Pro Phe Lys Glu Lys Gly Glu Val Arg Arg Pro Thr
225                 230                 235                 240
Leu Ser Trp Pro Arg Glu Ile Pro Leu Val Lys Gly Gly Lys Pro Asp
                245                 250                 255
Val Val Gln Ile Val Arg Asn Tyr Asn Ala Tyr Leu Arg Ala Ser Asp
            260                 265                 270
Asp Leu Pro Lys Leu Phe Ile Glu Ser Asp Pro Gly Phe Phe Ser Asn
        275                 280                 285
Ala Ile Val Glu Gly Ala Lys Lys Phe Pro Asn Thr Glu Phe Val Lys
    290                 295                 300
Val Lys Gly Leu His Phe Leu Gln Glu Asp Ala Pro Asp Glu Met Gly
305                 310                 315                 320
Lys Tyr Ile Lys Ser Phe Val Glu Arg Val Leu Lys Asn Glu Gln Gly
                325                 330                 335
Gly Gly Ser Thr Ala Gly Gly Gly Gly Ser Lys Arg Gln Phe Ala Asp
            340                 345                 350
Tyr Thr Glu Ala Glu Phe Ile Ala Phe Met Glu Asp Ile Phe Arg Glu
        355                 360                 365
Asn Glu Ala Glu Thr Asp Asp Arg Leu Asp Val Leu Leu Asp Gln Phe
    370                 375                 380
Arg Glu Ile Thr Gly His Pro Asp Gly Thr Asp Leu Ile Tyr Tyr Cys
385                 390                 395                 400
Glu Ser Asp Ala Glu Cys Thr Pro Glu Arg Ile Thr Gln Lys Val Lys
                405                 410                 415
Ser Trp Arg Ala Ala Asn Gly Leu Pro Gly Phe Lys Ser Thr Leu Glu
            420                 425                 430
Gly Gly Gly Ser His His His His His His
        435                 440

<210> 18
<211> 441
<212> PRT
<213> Artificial Sequence

<220>
<223> Avitag-RLuc8-ImKhan-6His

<400> 18
Met Gly Leu Asn Asp Ile Phe Glu Ala Gln Lys Ile Glu Trp His Glu
1               5                   10                  15
Gly Gly Ser Gly Gly Ser Gly Gly Ser Ala Ser Lys Val Tyr Asp Pro
            20                  25                  30
Glu Gln Arg Lys Arg Met Ile Thr Gly Pro Gln Trp Trp Ala Arg Cys
        35                  40                  45
Lys Gln Met Asn Val Leu Asp Ser Phe Ile Asn Tyr Tyr Asp Ser Glu
    50                  55                  60
Lys His Ala Glu Asn Ala Val Ile Phe Leu His Gly Asn Ala Thr Ser
65                  70                  75                  80
Ser Tyr Leu Trp Arg His Val Val Pro His Ile Glu Pro Val Ala Arg
                85                  90                  95
Cys Ile Ile Pro Asp Leu Ile Gly Met Gly Lys Ser Gly Lys Ser Gly

                100                 105                 110
Asn Gly Ser Tyr Arg Leu Leu Asp His Tyr Lys Tyr Leu Thr Ala Trp
        115                 120                 125
Phe Glu Leu Leu Asn Leu Pro Lys Lys Ile Ile Phe Val Gly His Asp
    130                 135                 140
Trp Gly Ala Ala Leu Ala Phe His Tyr Ala Tyr Glu His Gln Asp Arg
145                 150                 155                 160
Ile Lys Ala Ile Val His Met Glu Ser Val Val Asp Val Ile Glu Ser
                165                 170                 175
Trp Asp Glu Trp Pro Asp Ile Glu Glu Asp Ile Ala Leu Ile Lys Ser
            180                 185                 190
Glu Glu Gly Glu Lys Met Val Leu Glu Asn Asn Phe Phe Val Glu Thr
        195                 200                 205
Val Leu Pro Ser Lys Ile Met Arg Lys Leu Glu Pro Glu Glu Phe Ala
    210                 215                 220
Ala Tyr Leu Glu Pro Phe Lys Glu Lys Gly Glu Val Arg Arg Pro Thr
225                 230                 235                 240
Leu Ser Trp Pro Arg Glu Ile Pro Leu Val Lys Gly Gly Lys Pro Asp
                245                 250                 255
Val Val Gln Ile Val Arg Asn Tyr Asn Ala Tyr Leu Arg Ala Ser Asp
            260                 265                 270
Asp Leu Pro Lys Leu Phe Ile Glu Ser Asp Pro Gly Phe Phe Ser Asn
        275                 280                 285
Ala Ile Val Glu Gly Ala Lys Lys Phe Pro Asn Thr Glu Phe Val Lys
    290                 295                 300
Val Lys Gly Leu His Phe Leu Gln Glu Asp Ala Pro Asp Glu Met Gly
305                 310                 315                 320
Lys Tyr Ile Lys Ser Phe Val Glu Arg Val Leu Lys Asn Glu Gln Gly
                325                 330                 335
Gly Gly Ser Thr Ala Gly Gly Gly Gly Ser Glu Leu Lys Asn Lys Leu
            340                 345                 350
Glu Asp Tyr Thr Glu Ala Glu Phe Leu Ser Leu Leu Asn Lys Ile Trp
        355                 360                 365
Ala Val Asp Val Ser Glu Glu Glu His Asp Asn Leu Ile Asp His Phe
    370                 375                 380
Glu Lys Leu Ser Glu His Pro Asn Gly Asn Gly Leu Ile Phe Tyr Pro
385                 390                 395                 400
Glu Asn Gly Val Glu Asp Ser Pro Glu Gly Val Leu Lys Val Ile Lys
                405                 410                 415
Glu Trp Arg Ala Lys Asn Gly Lys Pro Gly Phe Lys Lys Leu Glu Gly
            420                 425                 430
Gly Gly Ser His His His His His His
        435                 440

<210> 19
<211> 113
<212> PRT
<213> Artificial Sequence

<220>
<223> Myc-Im2-6His

<400> 19
Met Gly Gly Glu Gln Lys Leu Ile Ser Glu Glu Asp Leu Gly Gly Ser
1               5                   10                  15
Glu Leu Lys His Ser Ile Ser Asp Tyr Thr Glu Ala Glu Phe Leu Glu
            20                  25                  30
Phe Val Lys Lys Ile Cys Arg Ala Glu Gly Ala Thr Glu Glu Asp Asp
        35                  40                  45
Asn Lys Leu Val Arg Glu Phe Glu Arg Leu Thr Glu His Pro Asp Gly
    50                  55                  60
Ser Asp Leu Ile Tyr Tyr Pro Arg Asp Asp Arg Glu Asp Ser Pro Glu

65                  70                  75                  80
Gly Ile Val Lys Glu Ile Lys Glu Trp Arg Ala Ala Asn Gly Lys Ser
                85                  90                  95
Gly Phe Lys Gln Gly Leu Glu Gly Gly Gly Ser His His His His His
            100                 105                 110
His

<210> 20
<211> 114
<212> PRT
<213> Artificial Sequence

<220>
<223> Myc-Im7-6His

<400> 20
Met Gly Gly Glu Gln Lys Leu Ile Ser Glu Glu Asp Leu Gly Gly Ser
1               5                   10                  15
Glu Leu Lys Asn Ser Ile Ser Asp Tyr Thr Glu Ala Glu Phe Val Gln
            20                  25                  30
Leu Leu Lys Glu Ile Glu Lys Glu Asn Val Ala Ala Thr Asp Asp Val
        35                  40                  45
Leu Asp Val Leu Leu Glu His Phe Val Lys Ile Thr Glu His Pro Asp
    50                  55                  60
Gly Thr Asp Leu Ile Tyr Tyr Pro Ser Asp Asn Arg Asp Asp Ser Pro
65                  70                  75                  80
Glu Gly Ile Val Lys Glu Ile Lys Glu Trp Arg Ala Ala Asn Gly Lys
                85                  90                  95
Pro Gly Phe Lys Gln Gly Leu Glu Gly Gly Gly Ser His His His His
            100                 105                 110
His His

<210> 21
<211> 112
<212> PRT
<213> Artificial Sequence

<220>
<223> Myc-Im8-6His

<400> 21
Met Gly Gly Glu Gln Lys Leu Ile Ser Glu Glu Asp Leu Gly Gly Ser
1               5                   10                  15
Glu Leu Lys Asn Ser Ile Ser Asp Tyr Thr Glu Thr Glu Phe Lys Lys
            20                  25                  30
Ile Ile Glu Asp Ile Ile Asn Cys Glu Gly Asp Glu Lys Lys Gln Asp
        35                  40                  45
Asp Asn Leu Glu His Phe Ile Ser Val Thr Glu His Pro Ser Gly Ser
    50                  55                  60
Asp Leu Ile Tyr Tyr Pro Glu Gly Asn Asn Asp Gly Ser Pro Glu Ala
65                  70                  75                  80
Val Ile Lys Glu Ile Lys Glu Trp Arg Ala Ala Asn Gly Lys Ser Gly
                85                  90                  95
Phe Lys Gln Gly Leu Glu Gly Gly Gly Ser His His His His His His
            100                 105                 110

<210> 22

<211> 107
<212> PRT
<213> Artificial Sequence

<220>
<223> Myc-Im9-6His

<400> 22
Met Gly Gly Glu Gln Lys Leu Ile Ser Glu Glu Asp Leu Gly Gly Ser
1               5                   10                  15
Glu Leu Lys His Ser Ile Ser Asp Tyr Thr Glu Ala Glu Phe Leu Gln
            20                  25                  30
Leu Val Thr Thr Ile Cys Asn Ala Asp Thr Ser Ser Glu Glu Glu Leu
        35                  40                  45
Val Lys Leu Val Thr His Phe Glu Glu Met Thr Glu His Pro Ser Gly
    50                  55                  60
Ser Asp Leu Ile Tyr Tyr Pro Lys Glu Gly Asp Asp Asp Ser Pro Ser
65                  70                  75                  80
Gly Ile Val Asn Thr Val Lys Gln Trp Arg Ala Ala Asn Gly Lys Leu
                85                  90                  95
Glu Gly Gly Gly Ser His His His His His His
            100                 105

<210> 23
<211> 140
<212> PRT
<213> Artificial Sequence

<220>
<223> Myc-ImAP41-Avitag-6His

<400> 23
Met Gly Gly Glu Gln Lys Leu Ile Ser Glu Glu Asp Leu Gly Gly Ser
1               5                   10                  15
Asp Ile Lys Asn Asn Leu Ser Asp Tyr Thr Glu Ser Glu Phe Leu Glu
            20                  25                  30
Ile Ile Glu Glu Phe Phe Lys Asn Lys Ser Gly Leu Lys Gly Ser Glu
        35                  40                  45
Leu Glu Lys Arg Met Asp Lys Leu Val Lys His Phe Glu Glu Val Thr
    50                  55                  60
Ser His Pro Arg Lys Ser Gly Val Ile Phe His Pro Lys Pro Gly Phe
65                  70                  75                  80
Glu Thr Pro Glu Gly Ile Val Lys Glu Val Lys Glu Trp Arg Ala Ala
                85                  90                  95
Asn Gly Leu Pro Gly Phe Lys Ala Gly Leu Glu Gly Gly Ser Gly Gly
            100                 105                 110
Ser Gly Leu Asn Asp Ile Phe Glu Ala Gln Lys Ile Glu Trp His Glu
        115                 120                 125
Leu Glu Gly Gly Gly Ser His His His His His His
    130                 135                 140

<210> 24
<211> 138
<212> PRT
<213> Artificial Sequence

<220>
<223> Myc-ImSyr-Avitag-6His

<400> 24

Met Gly Gly Glu Gln Lys Leu Ile Ser Glu Glu Asp Leu Gly Gly Ser
1               5                   10                  15
Ile Phe Lys Glu Lys Ile Glu Asp Tyr Thr Glu Glu Glu Phe Leu Glu
            20                  25                  30
Phe Leu Lys Gly Leu Ser Ser Glu Tyr Ser Gln Leu His Gly Asp Glu
        35                  40                  45
Phe Ile Lys His Met Asp Arg Ser Val Glu His Phe Val Lys Ile Thr
    50                  55                  60
Glu His Pro Ala Gln Thr Asp Val Ile Phe Tyr Pro Glu Glu Gly Gln
65                  70                  75                  80
Glu Asp Thr Pro Glu Gly Ile Leu Lys Val Ile Lys Glu Trp Arg Ala
                85                  90                  95
Lys Asn Gly Lys Pro Gly Phe Lys Ser Gly Gly Ser Gly Gly Ser Gly
            100                 105                 110
Leu Asn Asp Ile Phe Glu Ala Gln Lys Ile Glu Trp His Glu Leu Glu
        115                 120                 125
Gly Gly Gly Ser His His His His His His
    130                 135

<210> 25
<211> 140
<212> PRT
<213> Artificial Sequence

<220>
<223> Myc-ImErw-Avitag-6His

<400> 25
Met Gly Gly Glu Gln Lys Leu Ile Ser Glu Glu Asp Leu Gly Gly Ser
1               5                   10                  15
Asn Leu Lys Glu Lys Leu Glu Asp Tyr Thr Glu Ala Glu Phe Ile Ser
            20                  25                  30
Tyr Leu Lys Glu Phe Phe Asp Asn Pro Met Gly Leu Arg Gly Lys Glu
        35                  40                  45
Leu Glu Thr His Leu Asp Ser Leu Val Glu His Phe Asp Lys Ile Val
    50                  55                  60
Phe His Pro Glu Gly Asn Gly Leu Ile Phe Tyr Pro Pro Asp Glu Arg
65                  70                  75                  80
Asp Asp Ser Pro Glu Gly Val Leu Asn Glu Ile Lys Arg Trp Arg Lys
                85                  90                  95
Ser Gln Gly Leu Pro Leu Phe Lys Asp Ser Lys Gly Gly Ser Gly Gly
            100                 105                 110
Ser Gly Leu Asn Asp Ile Phe Glu Ala Gln Lys Ile Glu Trp His Glu
        115                 120                 125
Leu Glu Gly Gly Gly Ser His His His His His His
    130                 135                 140

<210> 26
<211> 140
<212> PRT
<213> Artificial Sequence

<220>
<223> Avitag-ImLeaf-6His

<400> 26
Met Gly Leu Asn Asp Ile Phe Glu Ala Gln Lys Ile Glu Trp His Glu
1               5                   10                  15
Gly Gly Ser Gly Gly Ser Gly Gly Ser Gly Gly Gly Gly Ser Lys Leu
            20                  25                  30
Gly Gly Gly Gly Ser Thr Ala Gly Gly Gly Gly Ser Lys Arg Gln Phe

35                  40                  45
Ala Asp Tyr Thr Glu Ala Glu Phe Ile Ala Phe Met Glu Asp Ile Phe
    50                  55                  60
Arg Glu Asn Glu Ala Glu Thr Asp Asp Arg Leu Asp Val Leu Leu Asp
65                  70                  75                  80
Gln Phe Arg Glu Ile Thr Gly His Pro Asp Gly Thr Asp Leu Ile Tyr
                85                  90                  95
Tyr Cys Glu Ser Asp Ala Glu Cys Thr Pro Glu Arg Ile Thr Gln Lys
            100                 105                 110
Val Lys Ser Trp Arg Ala Ala Asn Gly Leu Pro Gly Phe Lys Ser Thr
        115                 120                 125
Leu Glu Gly Gly Gly Ser His His His His His His
    130                 135                 140

<210> 27
<211> 139
<212> PRT
<213> Artificial Sequence

<220>
<223> Avitag-ImKhan-6His:

<400> 27
Met Gly Leu Asn Asp Ile Phe Glu Ala Gln Lys Ile Glu Trp His Glu
1               5                   10                  15
Gly Gly Ser Gly Gly Ser Gly Gly Ser Gly Gly Gly Gly Ser Lys Leu
            20                  25                  30
Gly Gly Gly Gly Ser Thr Ala Gly Gly Gly Gly Ser Glu Leu Lys Asn
        35                  40                  45
Lys Leu Glu Asp Tyr Thr Glu Ala Glu Phe Leu Ser Leu Leu Asn Lys
    50                  55                  60
Ile Trp Ala Val Asp Val Ser Glu Glu Glu His Asp Asn Leu Ile Asp
65                  70                  75                  80
His Phe Glu Lys Leu Ser Glu His Pro Asn Gly Asn Gly Leu Ile Phe
                85                  90                  95
Tyr Pro Glu Asn Gly Val Glu Asp Ser Pro Glu Gly Val Leu Lys Val
            100                 105                 110
Ile Lys Glu Trp Arg Ala Lys Asn Gly Lys Pro Gly Phe Lys Lys Leu
        115                 120                 125
Glu Gly Gly Gly Ser His His His His His His
    130                 135

<210> 28
<211> 30
<212> PRT
<213> Artificial Sequence

<220>
<223> BACTERIOCIN MOTIF

<220>
<221> SITE
<222> (3)...(16)
<223> Any amino acid

<220>
<221> SITE
<222> (18)...(25)
<223> Any amino acid

<220>
<221> SITE

<222> (27)...(29)
<223> Any amino acid

<400> 28
His His Xaa Xaa Xaa Xaa Xaa Xaa Xaa Xaa Xaa Xaa Xaa Xaa Xaa Xaa
1               5                   10                  15
Asn Xaa Xaa Xaa Xaa Xaa Xaa Xaa Xaa His Xaa Xaa Xaa His
            20                  25                  30

<210> 29
<211> 30
<212> PRT
<213> Artificial Sequence

<220>
<223> BACTERIOCIN MOTIF MUTATION 1

<220>
<221> SITE
<222> (3)...(16)
<223> Any amino acid

<220>
<221> SITE
<222> (18)...(25)
<223> Any amino acid

<220>
<221> SITE
<222> (27)...(29)
<223> Any amino acid

<400> 29
His Ala Xaa Xaa Xaa Xaa Xaa Xaa Xaa Xaa Xaa Xaa Xaa Xaa Xaa Xaa
1               5                   10                  15
Asn Xaa Xaa Xaa Xaa Xaa Xaa Xaa Xaa His Xaa Xaa Xaa His
            20                  25                  30

<210> 30
<211> 30
<212> PRT
<213> Artificial Sequence

<220>
<223> BACTERIOCIN MOTIF MUTATION 2

<220>
<221> SITE
<222> (3)...(16)
<223> Any amino acid

<220>
<221> SITE
<222> (18)...(25)
<223> Any amino acid

<220>
<221> SITE
<222> (27)...(29)
<223> Any amino acid

<400> 30

Ala His Xaa Xaa Xaa Xaa Xaa Xaa Xaa Xaa Xaa Xaa Xaa Xaa Xaa Xaa
1               5                   10                  15
Asn Xaa Xaa Xaa Xaa Xaa Xaa Xaa Xaa His Xaa Xaa Xaa His
            20                  25                  30